(19) Europäisches Patentamt
European Patent Office
Office européen des brevets

(11) **EP 3 234 025 B1**

(12) **EUROPÄISCHE PATENTSCHRIFT**

(45) Veröffentlichungstag und Bekanntmachung des Hinweises auf die Patenterteilung:
**24.04.2019   Patentblatt 2019/17**

(21) Anmeldenummer: **15817839.2**

(22) Anmeldetag: **21.12.2015**

(51) Int Cl.:
*C09C 1/00* (2006.01)          *A61Q 1/04* (2006.01)
*A61Q 3/02* (2006.01)          *A61Q 5/00* (2006.01)
*A61Q 19/00* (2006.01)        *A61Q 19/10* (2006.01)
*A61K 8/02* (2006.01)          *A61K 8/20* (2006.01)
*A61K 8/26* (2006.01)          *A61K 8/29* (2006.01)

(86) Internationale Anmeldenummer:
**PCT/EP2015/080870**

(87) Internationale Veröffentlichungsnummer:
**WO 2016/097421 (23.06.2016 Gazette 2016/25)**

(54) **ROTFARBENE EFFEKTPIGMENTE MIT HOHEM CHROMA UND HOHER BRILLANZ, VERFAHREN ZU IHRER HERSTELLUNG UND VERWENDUNG DERSELBEN**

GOLD-COLOURED EFFECT PIGMENTS WITH HIGH CHROMA AND A HIGH BRILLIANCE, METHOD FOR THEIR PREPARATION AND THEIR USE

PIGMENTS À EFFET COULEUR OR D'UNE PLUS GRANDE BRILLANCE ET AYANT UN DEGRÉ CHROMATIQUE ÉLEVÉ, LEUR PROCÉDÉ DE FABRICATION ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.12.2014   EP 14199293**
          **19.12.2014   EP 14199126**
          **19.12.2014   EP 14199130**

(43) Veröffentlichungstag der Anmeldung:
**25.10.2017   Patentblatt 2017/43**

(73) Patentinhaber: **Eckart GmbH**
**91235 Hartenstein (DE)**

(72) Erfinder:
• **GRÜNER, Michael**
**91275 Auerbach (DE)**
• **KAUPP, Günter**
**91284 Neuhaus (DE)**
• **SCHNEIDER, Ralph**
**91207 Lauf (DE)**

(74) Vertreter: **Henglein, Frank Arwed**
**Altana Management Services GmbH**
**Location Hartenstein**
**Güntersthal 4**
**91235 Hartenstein (DE)**

(56) Entgegenhaltungen:
**WO-A1-2014/094993      US-A- 3 711 308**
**US-B1- 8 585 818**

**Beschreibung**

Die vorliegende Erfindung betrifft rotfarbene Effektpigmente umfassend ein nichtmetallisches plättchenförmiges Substrat und eine darauf aufgebrachte Beschichtung, wobei die Beschichtung wenigstens ein Metalloxid, Metallhydroxid und/oder Metalloxidhydrat umfasst, die Metallionen des Metalloxids, Metallhydroxids und/oder Metalloxidhydrats wenigstens zwei unterschiedliche Metallionen, ausgewählt aus der Gruppe der Metalle, bestehend aus Fe, Sn, Ti und Zr umfassen, ein Verfahren zur Herstellung derselben sowie die Verwendung der rotfarbenen Effektpigmente.

Mehrschichtpigmente, welche basierend auf einem nichtmetallischen plättchenförmigen Substrat wenigstens eine Schichtenfolge aus alternierend hoch-, niedrig-, hochbrechender Schicht umfassen, sind beispielsweise aus EP 1 572 812 A1, EP 1 213 330 A1, EP 1 025 168 B2, EP 1 621 585 A2, EP 0 948 572 A1, EP 0 950 693 A1, EP 1 306 412 A1, EP 1 587 881 A2, EP 2 632 988 A1 oder EP 1 474 486 A2 bekannt. In Abhängigkeit von der optischen Schichtdicke der niedrigbrechenden Schicht können die Mehrschichtpigmente ihr optisches Erscheinungsbild in Abhängigkeit vom Betrachtungswinkel ändern, wie z.B. in EP 1 375 601 A1, EP 1 281 732 A1, EP 0 753 545 A2, US 2004/0003758 A1 beschrieben. Allen vorstehend aufgeführten Anmeldungen ist gemein, dass in der Schichtenfolge eine niedrigbrechende Schicht aus einem niedrigbrechenden Metalloxid, wie beispielsweise Siliziumoxid, vorhanden ist.

Mehrschichtpigmente zeichnen sich gegenüber einschichtigen Effektpigmenten mit nur einer einzigen identischen ersten Schicht durch einen höheren Glanz und gegebenenfalls durch ein höheres Chroma aus, gleiches Substrat und gleiche Teilchengröße werden hierbei selbstverständlich vorausgesetzt.

EP 1 422 268 A2 offenbart ein Pigment mit Mehrschichtaufbau, wobei dieses Pigment zwei oder mehr Metalloxidschichten aufweist, wobei das wenigstens eine Metall(ion) der Metalloxidschicht aus der Gruppe, die aus Cer, Zinn, Titan, Eisen, Zink und Zirkonium, besteht, ausgewählt wird. Ziel dieser Anmeldung sind hochchromatische und hochbrillante Pigmente, welche in ihrer Beschichtung möglichst wenig und möglichst kleine Poren aufweisen. Ein geringes Porenvolumen soll gemäß EP 1 422 268 A2 eine optisch hochwertige Beschichtung gewährleisten.

US 2015/0344677 A1 betrifft Effektpigmente basierend auf beschichteten plättchenförmigen Substraten. Die Beschichtung umfasst eine erste und eine zweite hochbrechende Schicht sowie eine dritte Komponente, welche in eine oder beide der hochbrechenden Schichten teilweise oder zu 100% eindiffundieren soll. Bei der dritten Komponente kann es sich um $SiO_2$ oder ein anderes Metalloxid handeln. Ziel dieser Anmeldung ist es, bei Effektpigmenten mit einem Dso-Wert von 15 $\mu$m oder weniger eine $SiO_2$-Belegung ohne Agglomeration zu erhalten.

US 8,585,818 B1 beschreibt Effektpigmente auf Basis von Perlitplättchen mit einer Schichtenfolge von zunächst $SnO_2$, gefolgt von $TiO_2$ und anschließend $Fe_2O_3$. Die Schichtdicke der $SnO_2$-Schicht beträgt maximal 10 nm und es werden keine rotfarbene Effektpigmente erhalten.

WO 2014/094993 A1 offenbart goldfarbene Effektpigmente, die u.a. einen Schichtaufbau Substrat-$TiO_2$/$Fe_2O_3$ - $SnO_2$ - $TiO_2$/$Fe_2O_3$ aufweisen. Auch hier werden keine rotfarbenen Effektpigmente erhalten.

US 3 711 308 A offenbart Effektpigmente, bei denen Glimmer mit $TiO_2$, anschließend einer Mischung aus $TiO_2$ und $Fe_2O_3$ und anschließend wieder $TiO_2$ beschichtet wird. Es werden nur goldfarbene und keine rotfarbene Effektpigmente offenbart.

Aufgabe der vorliegenden Erfindung war es, ein rotes hochchromatisches Pigment mit hohem Glanz und hoher Deckkraft zur Verfügung zu stellen, welches eine rote Interferenzfarbe, eine hohe mechanische Stabilität sowie eine hohe Chemikalienstabilität aufweist und gleichzeitig mit geringem Materialeinsatz auf einfachste Art und Weise herstellbar ist.

Diese Aufgabe wird gelöst durch Bereitstellung eines rotfarbenen Effektpigments, welches ein nichtmetallisches plättchenförmiges Substrat und eine auf dem Substrat aufgebrachte Beschichtung umfasst, wobei die Beschichtung wenigstens ein Metalloxid, Metallhydroxid und/oder Metalloxidhydrat umfasst, die Metallionen des Metalloxids, Metallhydroxids und/oder Metalloxidhydrats wenigstens zwei unterschiedliche Metallionen, ausgewählt aus der Gruppe der Metalle, bestehend aus Fe, Sn, Ti und Zr umfassen, der Anteil an Eisenionen, bestimmt mittels RFA und berechnet als elementares Eisen, bei insgesamt wenigstens 17 Gew.-%, bezogen auf das Gesamtgewicht des rotfarbenen Effektpigments, und wobei der Bunttonwinkel $h^*_{15}$ im CIE-LCh-Farbraum in einem Bereich von 320° bis 360° und 0° bis 60° liegt.

Bevorzugte Weiterbildungen des rotfarbenen Effektpigments sind in den abhängigen Ansprüchen 2 bis 9 angegeben.

Des Weiteren wird die Aufgabe durch Bereitstellung eines Verfahrens zur Herstellung des erfindungsgemäßen rotfarbenen Effektpigments gelöst, wobei das Verfahren die folgenden Schritte umfasst:

(i) Optionales Aufbringen einer nicht kalzinierten Schicht, die Zinnoxid-, Zinnhydroxid- und/oder Zinnoxidhydrat umfasst oder daraus besteht, auf dem nichtmetallischen plättchenförmigen Substrat,

(ii) sequentielles Aufbringen von drei nicht kalzinierten Schichten A, B und C aus oder mit jeweils wenigstens einem Metalloxid, Metallhydroxid und/oder Metalloxidhydrat, wobei das Metallion der Schichten A und C jeweils wenigstens ein Metallion, ausgewählt aus der Gruppe der Metalle, bestehend aus Fe, Ti und Zr, umfasst oder ist, das Metallion der Schicht B wenigstens ein Metallion, ausgewählt aus der Gruppe der Metalle, bestehend aus Sn, Ti und Zr, umfasst oder ist, wenigstens ein Metallion der Schichten A und/oder C ein Eisenion umfasst oder ist, die Schichten A, B und C unmittelbar aufeinander angeordnet werden und wobei das in der Schicht B aufgebrachte wenigstens eine Metalloxid, Metallhydroxid und/oder Metalloxidhydrat in Bezug auf das Metallion verschieden von dem oder den Metallion(en) der Metalloxide, Metallhydroxide und/oder Metalloxidhydrate der Schicht A und Schicht C ist,

(iii) Kalzinieren des in Schritt (ii) erhaltenen Produktes bei einer Temperatur aus einem Bereich von 400°C bis 1000°C unter Erhalt des rotfarbenen Effektpigmentes.

Alternativ wird die Aufgabe durch Bereitstellung eines Verfahrens zur Herstellung des erfindungsgemäßen rotfarbenen Effektpigments gelöst, wobei das Verfahren die folgenden Schritte umfasst:

(i) Sequentielles Aufbringen von zwei nicht kalzinierten Schichten B und C aus oder mit jeweils wenigstens einem Metalloxid, Metallhydroxid und/oder Metalloxidhydrat auf ein kalziniertes einfach oder mehrfach beschichtetes nichtmetallisches Substrat, wobei das Metallion der Schicht B wenigstens ein Metallion, ausgewählt aus der Gruppe der Metalle, bestehend aus Ti, Sn und Zr, umfasst oder ist, das Metallion der Schicht C wenigstens ein Metallion, ausgewählt aus der Gruppe der Metalle, bestehend aus Fe, Ti und Zr, umfasst oder ist, wenigstens ein Metallion der Schicht, welche in Richtung Substrat unmittelbar an die Schicht B angrenzt, und/oder der Schicht C ein Eisenion umfasst oder ist, die Schichten B und C unmittelbar aufeinander angeordnet werden und wobei das in der Schicht B aufgebrachte wenigstens eine Metalloxid, Metallhydroxid und/oder Metalloxidhydrat in Bezug auf das Metallion verschieden von dem oder den Metallion(en) des Metalloxids, Metallhydroxids und/oder Metalloxidhydrats der Schicht C und der Schicht, welche in Richtung Substrat unmittelbar an die Schicht B angrenzt, ist, wobei das oder die Metallion(en) des Metalloxids, Metallhydroxids und/oder Metalloxidhydrats der Schicht, welche in Richtung Substrat unmittelbar an die Schicht B angrenzt, wenigstens ein Metallion, ausgewählt aus der Gruppe der Metalle, bestehend aus Fe, Ti und Zr, umfasst oder ist,

(ii) Kalzinieren des in Schritt (i) erhaltenen Produktes bei einer Temperatur aus einem Bereich von 400°C bis 1000°C unter Erhalt des rotfarbenen Effektpigmentes.

Gegenstand der Erfindung ist weiterhin die Verwendung des erfindungsgemäßen rotfarbenen Effektpigments in kosmetischen Formulierungen, Kunststoffen, Folien, Textilien, keramischen Materialien, Gläsern, Farben, Druckfarben, Tinten, Lacken, Pulverlacken und/oder als Lasermarkierungsadditiv.

Die der Erfindung zugrunde liegende Aufgabe wird des Weiteren durch Bereitstellung eines Gegenstandes gelöst, wobei der Gegenstand wenigstens ein erfindungsgemäßes rotfarbenes Effektpigment aufweist.

Mit dem Ausdruck "ein Metallion" bzw. "ein Eisenion" ist erfindungsgemäß nicht ein einzelnes Metallion bzw. Eisenion, sondern eine Vielzahl an Metallionen bzw. Eisenionen gemeint.

Die zu beschichtenden nichtmetallischen plättchenförmigen Substrate können aus der Gruppe, bestehend aus natürlichen Glimmerplättchen, synthetischen Glimmerplättchen, Eisenglimmerplättchen, Glasplättchen, Eisenoxidplättchen, $SiO_2$-Plättchen, $Al_2O_3$-Plättchen, Kaolinplättchen, Talkplättchen und Bismuthoxychloridplättchen, ausgewählt werden. Erfindungsgemäß können die rotfarbenen Effektpigmente auch auf Gemischen der vorstehend angegebenen nichtmetallischen plättchenförmigen Substrate basieren. Die vorgenannten nichtmetallischen plättchenförmigen Substrate können auch eine oder mehrere Schichten aus oder mit wenigstens einem hoch- und/oder niedrigbrechenden Metalloxid, Metallhydroxid und/oder Metalloxidhydrat aufweisen. So können als Substrate also auch einfach oder mehrfach beschichtete Perlglanzpigmente oder Interferenzpigmente verwendet werden. Gemäß einer bevorzugten Ausführungsform sind die erfindungsgemäß zu verwendenden Substrate unbeschichtete, nichtmetallische plättchenförmige Substrate.

Bevorzugt werden die nichtmetallischen plättchenförmigen Substrate aus der Gruppe, bestehend aus natürlichen Glimmerplättchen, synthetischen Glimmerplättchen, Glasplättchen, $SiO_2$-Plättchen, $Al_2O_3$-Plättchen und deren Gemische, ausgewählt. Besonders bevorzugt werden die nichtmetallischen plättchenförmigen Substrate aus der Gruppe, bestehend aus natürlichen Glimmerplättchen, synthetischen Glimmerplättchen, Glasplättchen und deren Gemische, ausgewählt. Ganz besonders bevorzugt sind als nichtmetallische plättchenförmige Substrate synthetische Glimmerplättchen und/oder Glasplättchen sowie deren Gemische. Insbesondere Glasplättchen sind als nichtmetallisches plättchenförmiges Substrat bevorzugt.

Die als Substrat verwendbaren Glasplättchen können im Hinblick auf ihre Zusammensetzung aus Silikatglas, wie Kalknatronglas, Bleikristallglas, E-Glas, A-Glas, C-Glas, ECR-Glas, Duranglas, Fensterglas, Laborglas, Aluminosilikatglas oder Borosilikatglas bestehen. Bevorzugt weisen die Glasplättchen eine Zusammensetzung entsprechend der Lehre, insbesondere entsprechend dem Hauptanspruch der EP 1 980 594 B1, besonders bevorzugt entsprechend der Lehre, insbesondere entsprechend dem jeweiligen Hauptanspruch der EP 1 829 833 B1 oder der EP 2 042 474 B1, auf. Die Herstellung der als Substrat einsetzbaren Glasplättchen erfolgt vorzugsweise nach dem in EP 289 240 B1 beschriebenen Verfahren.

Bei einer weiteren Ausführungsform können die Glasplättchen durch den Zusatz von wenigstens einem anorganischen Farbmittel bei ihrer Herstellung gezielt eingefärbt werden. Geeignete Farbmittel sind solche, die sich bei der jeweiligen Schmelztemperatur der Glaszusammensetzung nicht zersetzen. Der Anteil an Farbmittel liegt hierbei bevorzugt in einem Bereich von insgesamt 0,1 Gew.-% bis 50 Gew.-%, besonders bevorzugt in einem Bereich von insgesamt 1 Gew.-% bis 35 Gew.-% und ganz besonders bevorzugt in einem Bereich von insgesamt 5 Gew.-% bis 25 Gew.-%, jeweils bezogen auf das Gesamtgewicht der Glaszusammensetzung. Geeignete Farbmittel sind insbesondere elementare Edelmetalle, wie Au, Pd oder Pt, die Kationen oder komplexe Anionen der Elemente Cu, Cr, Mn, Fe, Ti und/oder Co sowie Mischungen der vorstehend aufgeführten Farbmittel.

Bei einer weiteren Ausführungsform liegt der Brechungsindex der als Substrat einsetzbaren Glasplättchen in einem Bereich von 1,45 bis 1,80, vorzugsweise in einem Bereich von 1,50 bis 1,70.

Bei einer weiteren Ausführungsform können die plättchenförmigen Substrate, insbesondere Glasplättchen, mit einer Schicht, die Siliziumoxid, Siliziumhydroxid und/oder Siliziumoxidhydrat umfasst oder daraus besteht, umhüllt sein. Beispielsweise kann durch die vorgenannte Beschichtung bei Verwendung von Glasplättchen die Glasoberfläche vor chemischer Veränderung, wie Quellung, Auslaugen von Glasbestandteilen oder Auflösung in aggressiven sauren Belegungslösungen, geschützt werden.

Die als Substrat verwendbaren synthetischen Glimmerplättchen können eine Zusammensetzung gemäß Hauptanspruch der CN 102718229 A oder gemäß Hauptanspruch der US 2014/0251184 A1 aufweisen. Sie können weiterhin gemäß den Angaben in der EP 0 723 997 A1, Seite 3 bis Seite 4 hergestellt werden.

Bei den als Substrat verwendbaren synthetischen Glimmerplättchen handelt es sich vorzugsweise um Fluorphlogopit der Formel $KMg_3AlSi_3O_{10}F_2$, $KMg_2½(Si_4O_{10})F_2$ oder $NaMg_2½(Si_4O_{10})F_2$, insbesondere um Fluorphlogopit der Formel $KMg_3AlSi_3O_{10}F_2$, welcher gemäß Röntgenfluoreszenzanalyse (RFA) vorzugsweise die in Tabelle 1 genannten Bestandteile als jeweiliges Metalloxid in den dort aufgeführten Bereichen umfasst.

Tabelle 1: Bevorzugte Zusammensetzungen von synthetischen Glimmerplättchen gemäß RFA

| Zusammensetzung synthetischer Glimmerplättchen, Angaben in Gew.-%, jeweils bezogen auf das Gesamtgewicht der synthetischen Glimmerplättchen | |
| --- | --- |
| $SiO_2$ | 38 bis 46 |
| $Al_2O_3$ | 10 bis 14 |
| $K_2O$ | 9 bis 13 |
| $Fe_2O_3$ | 0,01 bis 0,25 |
| MgO | 26 bis 34 |
| MnO | 0 bis 0,05 |
| $Na_2O$ | 0 bis 13 |

Die mittlere Dicke der zu beschichtenden nichtmetallischen plättchenförmigen Substrate liegt bevorzugt in einem Bereich von 50 nm bis 5000 nm, besonders bevorzugt in einem Bereich von 60 nm bis 3000 nm und ganz besonders bevorzugt in einem Bereich von 70 nm bis 2000 nm. Unter der mittleren Dicke wird erfindungsgemäß das arithmetische Mittel verstanden, sofern nicht anders angegeben.

Bei einer Ausführungsform liegt die mittlere Dicke für Glasplättchen als zu beschichtendes nichtmetallisches plättchenförmiges Substrat in einem Bereich von 750 nm bis 1500 nm, bevorzugt in einem Bereich von 850 nm bis 1400 nm und besonders bevorzugt in einem Bereich von 900 nm bis 1300 nm.

Dünnere plättchenförmige Substrate führen zu einer geringeren Gesamtdicke der erfindungsgemäßen rotfarbenen Effektpigmente. So sind als nichtmetallisches plättchenförmiges Substrat ebenfalls bevorzugt Glasplättchen, deren mittlere Dicke in einem Bereich von 50 nm bis 700 nm, weiter bevorzugt in einem Bereich von 101 nm bis 600 nm, besonders bevorzugt in einem Bereich von 160 nm bis 500 nm und ganz besonders bevorzugt in einem Bereich 200 nm bis 400 nm liegt.

Bei einer weiteren Ausführungsform liegt die mittlere Dicke der natürlichen oder synthetischen Glimmerplättchen als zu beschichtendes nichtmetallisches plättchenförmiges Substrat bevorzugt in einem Bereich von 80 nm bis 1300 nm, weiter bevorzugt in einem Bereich von 90 nm bis 1000 nm, besonders bevorzugt in einem Bereich von 99 nm bis 800 nm und ganz besonders bevorzugt in einem Bereich von 200 nm bis 600 nm.

Beschichtet man nichtmetallische plättchenförmige Substrate unterhalb einer mittleren Dicke von 50 nm mit beispielsweise hochbrechenden Metalloxiden, so werden extrem bruchempfindliche Pigmente erhalten, die schon beim Einarbeiten in das jeweilige Anwendungsmedium zerbrechen können, was wiederum eine signifikante Herabsetzung des Glanzes bedingt.

Oberhalb einer mittleren Substratdicke von 5000 nm können die Pigmente insgesamt zu dick werden. Damit geht eine schlechtere spezifische Deckfähigkeit einher, d.h. die abgedeckte Fläche pro Gewichtseinheit an erfindungsgemäßem rotfarbenen Effektpigment ist geringer. Außerdem orientieren sich derartig dicke Pigmente in geringerem Ausmaß planparallel zum Untergrund im Anwendungsmedium. Aus einer schlechteren Orientierung wiederum resultiert ein verminderter Glanz.

Auch im Hinblick auf die Haptik können insgesamt zu dicke Effektpigmente unvorteilhaft in einer Anwendung sein.

Bei einer Ausführungsform beträgt die relative Standardabweichung der Dickenverteilung der nichtmetallischen plättchenförmigen Substrate 15% bis 100%, bevorzugt 17% bis 70%, besonders bevorzugt 19% bis 61% und ganz besonders bevorzugt 21% bis 41%. Die relative Standardabweichung in [%] ist dabei der Quotient aus berechneter Standardabweichung und mittlerer Dicke.

Die mittlere Dicke des nichtmetallischen plättchenförmigen Substrates wird anhand eines gehärteten Lackfilmes, in dem die erfindungsgemäßen rotfarbenen Effektpigmente im Wesentlichen planparallel zum Untergrund ausgerichtet sind, gemäß den nachstehenden Angaben in Abschnitt IIj "Bestimmung der mittleren Dicke der nichtmetallischen plättchenförmigen Substrate, der mittleren Schichtdicke der Schichten 2 und 3, der mittleren Schichtdicke der gesamten Beschichtung, der mittleren Höhe $h_a$ der Abstandslage sowie der mittleren Höhe $h_H$ der Hohlräume" bestimmt. Hierzu wird ein Querschliff des gehärteten Lackfilmes unter einem Rasterelektronenmikroskop (REM) untersucht, wobei die Dicke des nichtmetallischen plättchenförmigen Substrates von wenigstens 100 Effektpigmenten bestimmt und statistisch gemittelt wird. Mit dem Begriff "mittlere" ist erfindungsgemäß stets der arithmetische Mittelwert gemeint, sofern nicht anders angegeben.

Die rasterelektronenmikroskopischen Aufnahmen wurden anhand von Querschliffen der erfindungsgemäßen rotfarbenen Effektpigmente mit dem Rasterelektronenmikroskop Supra 35 (Fa. Zeiss) erhalten.

Die erfindungsgemäßen rotfarbenen Effektpigmente umfassen optional eine Schicht 1, mit einer mittleren Schichtdicke von weniger als 10 nm, die Zinnoxid, Zinnhydroxid und/oder Zinnoxidhydrat umfasst oder daraus besteht. Die Schicht 1 kann optional mit einer direkt an die Schicht 1 angrenzenden Schicht, beispielsweise der Schicht 2, zumindest teilweise als Mischschicht vorliegen.

Bei den Schichten 2 und 3 der erfindungsgemäßen rotfarbenen Effektpigmente handelt es sich nach Kalzination vorzugsweise jeweils um eine hochbrechende Schicht, deren Brechungsindex bevorzugt bei n > 1,8, besonders bevorzugt bei n ≥ 1,9 und ganz besonders bevorzugt bei n ≥ 2,1 liegt. Die Auswahl der wenigstens zwei unterschiedlichen Metallionen, wobei wenigstens eines der zwei unterschiedlichen Metallionen Eisenionen sind, in den Schichten 2 und 3 erfolgt erfindungsgemäß so, dass das oder die sich daraus bildenden Metalloxid(e), Metallhydroxid(e) und/oder Metalloxidhydrat(e) in den Schichten 2 und/oder 3 vorzugsweise jeweils einen gemittelten Brechungsindex von n > 1,8 aufweist oder aufweisen.

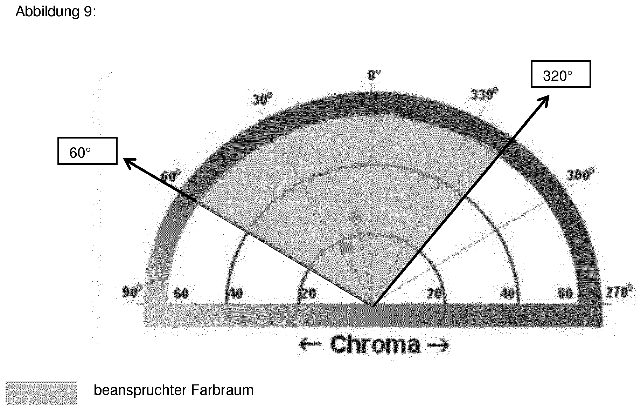

Das wenigstens eine Metalloxid, Metallhydroxid und/oder Metalloxidhydrat der Schichten 2 oder 3 umfasst wenigstens zwei unterschiedliche Metallionen, ausgewählt aus der Gruppe der Metalle, bestehend aus Fe, Sn, Ti und Zr, wobei eines der zwei unterschiedlichen Metallionen ein Eisenion ist. Erfindungsgemäß sind hierbei die Auswahl der wenigstens zwei unterschiedlichen Metallionen und der Anteil an Eisenionen so zu treffen, dass die resultierenden Effektpigmente rotfarben sind. Unter "rotfarbene Effektpigmente" wird im Rahmen dieser Erfindung verstanden, dass ihr Bunttonwinkel $h^*_{15}$ im CIE-LCh-Farbraum in einem Bereich von 320° bis 360° und 0° bis 60° weiter bevorzugt in einem Bereich von 330° bis 360° und 0° bis 55° besonders bevorzugt in einem Bereich von 335° bis 360° und 0° bis 45° und ganz besonders bevorzugt in einem Bereich von 340° bis 360° und 0° bis 40° liegt (Abbildung 9). Bevorzugt liegt das Chroma $C^*_{15}$ in den vorstehend genannten Bunttonwinkelbereichen bei > 28, besonders bevorzugt bei > 39 und ganz besonders bevorzugt bei > 46.

Der Bunttonwinkel $h^*_{15}$ sowie das Chroma $C^*_{15}$ werden hierbei anhand von Lackapplikationen auf Schwarz-Weiß-Deckungskarten (Byko-Chart 2853, Fa. Byk-Gardner) gemäß den nachfolgenden Angaben im Abschnitt IIb "Winkelabhängige Farbmessungen" bestimmt. Dazu werden die erfindungsgemäßen Effektpigmente in einer Pigmentierungshöhe von 6 Gew.-%, bezogen auf das Gesamtgewicht des Nasslacks, in einen konventionellen Nitrocelluloselack (Erco-Bronzemischlack 2615e farblos; Fa. Maeder Plastiklack AG) eingerührt, mit einer Spiralrakel in einer Nassfilmdicke von 40 $\mu$m, 76 $\mu$m oder 100 $\mu$m auf den Schwarz-Weiß-Deckungskarten appliziert, nachfolgend bei Raumtemperatur getrocknet und mit dem Mehrwinkelfarbmessgerät BYK-mac (Fa. Byk-Gardner) vermessen.

Tabelle 2: Nassfilmdicke in Abhängigkeit vom $D_{50}$-Wert der rotfarbenen Effektpigmente

| $D_{50}$-Wert | Spiralrakel |
|---|---|
| <40 $\mu$m | 40 $\mu$m |
| 40 $\mu$m - 85 $\mu$m | 76 $\mu$m |
| > 85 $\mu$m | 100 $\mu$m |

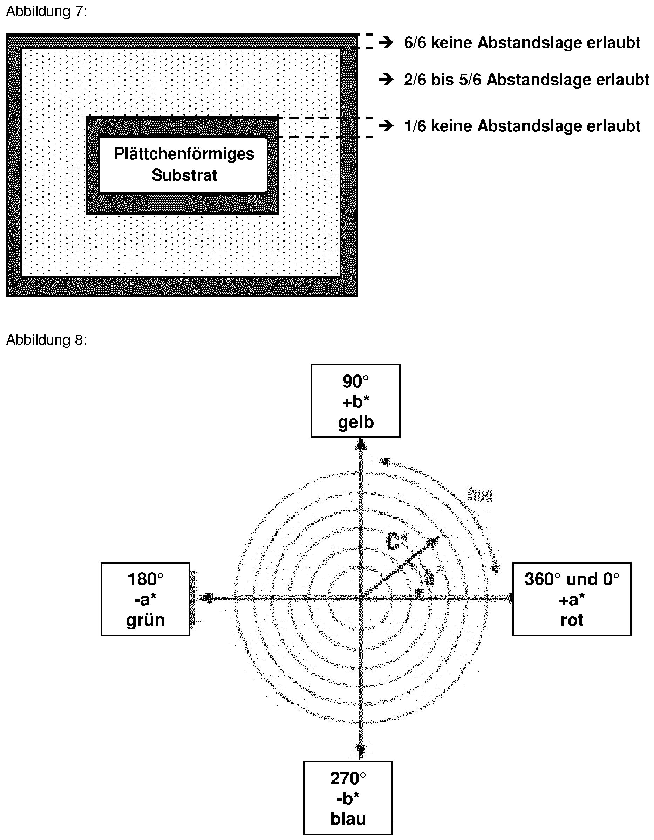

Beim CIE-LCh-Farbraum handelt es sich um den CIELab-Farbraum, wobei anstelle der kartesischen Koordinaten a*, b* die Zylinderkoordinaten C* (Buntheit, relative Farbsättigung, Entfernung von der L-Achse, englisch chroma) und h* (Bunttonwinkel, Winkel des Farbtons im CIELab-Farbkreis, engl. hue angle) angegeben werden (Abbildung 8).

$$D_q = \frac{L^{*110}_{schwarz}}{L^{*110}_{weiß}}$$

Der Deckungsquotient $D_q$, definiert als , der erfindungsgemäßen rotfarbenen Effektpigmente liegt bevorzugt bei $\geq$ 0,41, bevorzugt bei $\geq$ 0,45 besonders bevorzugt bei $\geq$ 0,50 und ganz besonders bevorzugt bei $\geq$ 0,55. Der Deckungsquotient wird hierbei anhand von Lackapplikationen auf Schwarz-Weiß-Deckungskarten (Byko-Chart 2853, Fa. Byk-Gardner) eines mit 6 Gew.-% des jeweiligen erfindungsgemäßen Effektpigments versetzten Nitrocelluloselacks (Erco-Bronzemischlack 2615e farblos; Fa. Maeder Plastiklack AG) gemäß den nachfolgenden Angaben im Abschnitt IIc "Deckungsvergleich" bestimmt. $L^{*110}_{schwarz}$ bzw. $L^{*110}_{weiß}$ sind dabei die unter einem Messwinkel von 110° auf schwarzem bzw. weißem Untergrund der Schwarz-Weiß-Deckungskarten, vorzugsweise mit dem Mehrwinkelfarbmessgerät BYK-mac der Fa. Byk-Gardner, gemessenen Helligkeitswerte.

Mit "wenigstens zwei unterschiedlichen Metallionen" ist erfindungsgemäß gemeint, dass wenigstens zwei Metallionen verschiedener Elemente vorliegen, beispielsweise Titan- und Eisenionen, oder Eisen-und Zinnionen, oder Eisen- und Zirkoniumionen, etc. Die verschiedenen Metallionen können dabei in einer Mischung von Metalloxiden und/oder Metallhydroxiden und/oder Metalloxidhydraten und/oder auch in Mischoxiden und/oder Mischhydroxiden und/oder Mischoxidhydraten in der Schicht 2 und/oder Schicht 3 des erfindungsgemäßen rotfarbenen Effektpigments vorliegen. Die Schicht 2 und/oder Schicht 3 können diese Mischung von Metalloxiden und/oder Metallhydroxiden und/oder Metalloxidhydraten und/oder Mischoxiden und/oder Mischhydroxiden und/oder Mischoxidhydraten umfassen oder daraus bestehen.

Der Anteil an Eisenoxid, Eisenhydroxid und/oder Eisenoxidhydrat liegt in dem erfindungsgemäßen rotfarbenen Effektpigment bevorzugt in einem Bereich von 17 Gew.-% bis 90 Gew.-%, besonders bevorzugt in einem Bereich von 20 Gew.-% bis 86 Gew.-% und ganz besonders bevorzugt in einem Bereich von 36 Gew.-% bis 78 Gew.-%, jeweils bestimmt mittels RFA, jeweils berechnet als elementares Eisen und jeweils bezogen auf das Gesamtgewicht des erfindungsgemäßen rotfarbenen Effektpigments.

Erfindungsgemäß liegt im rotfarbenen Effektpigment der Anteil an Metalloxid, Metallhydroxid und/oder Metalloxidhydrat, wobei das wenigstens eine Metallion ein Metallion aus der Gruppe der Metalle, bestehend aus Sn, Ti und Zr, umfasst oder ist, bevorzugt in einem Bereich von insgesamt 0,5 Gew.-% bis insgesamt 25 Gew.-%, weiter bevorzugt in einem Bereich von insgesamt 0,9 Gew.-% bis insgesamt 21 Gew.-%, besonders bevorzugt in einem Bereich von insgesamt 1,5 Gew.-% bis insgesamt 16 Gew.-% und ganz besonders bevorzugt in einem Bereich von insgesamt 2,0 Gew.-% bis insgesamt 12 Gew.-% und der Anteil an Eisenoxid, Eisenhydroxid und/oder Eisenoxidhydrat bevorzugt in einem Bereich von 25 Gew.-% bis 90 Gew.-%, weiter bevorzugt in einem Bereich von 28 Gew.-% bis 84 Gew.-%, besonders bevorzugt in einem Bereich von 30 Gew.-% bis 76 Gew.-% und ganz besonders bevorzugt in einem Bereich von 32 Gew.-% bis 71 Gew.-%, jeweils bestimmt mittels RFA, jeweils berechnet als Metalloxid und jeweils bezogen auf das Gesamtgewicht des rotfarbenen Effektpigments.

Bei einer Ausführungsform umfassen die erfindungsgemäßen rotfarbenen Effektpigmente kein Zinnoxid, Zinnhydroxid und/oder Zinnoxidhydrat in einer der Schichten 2 oder 3. In diesem Fall liegt der Anteil an Zinnoxid, Zinnhydroxid und/oder Zinnoxidhydrat im rotfarbenen Effektpigment bevorzugt in einem Bereich von 0,04 Gew.-% bis 1,5 Gew.-%, besonders bevorzugt in einem Bereich von 0,09 Gew.-% bis 1,3 Gew.-% und ganz besonders bevorzugt in einem Bereich von 0,1 Gew.-% bis 0,9 Gew.-%, jeweils bestimmt mittels RFA als Zinndioxid und jeweils bezogen auf das Gesamtgewicht des erfindungsgemäßen rotfarbenen Effektpigments.

Bei einer bevorzugten Ausführungsform umfasst wenigstens eine der Schichten 2 oder 3 Metalloxide, Metallhydroxide und/oder Metalloxidhydrate, wobei die Metallionen der Metalloxide, Metallhydroxide und/oder Metalloxidhydrate die Metalle Ti und Fe umfassen oder sind, wobei das Gewichtsverhältnis von Ti zu Fe, jeweils bestimmt mittels RFA und jeweils berechnet als elementares Metall, bevorzugt in einem Bereich von <8, weiter bevorzugt in einem Bereich von <5, besonders bevorzugt in einem Bereich von <2 und ganz besonders bevorzugt in einem Bereich von <1 liegt, und wobei der Anteil an Fe, bestimmt mittels RFA und berechnet als elementares Metall, bevorzugt in einem Bereich von 18 Gew.-% bis 85 Gew.-%, bezogen auf das Gesamtgewicht des erfindungsgemäßen rotfarbenen Effektpigments, liegt.

Bei einer besonders bevorzugten Ausführungsform umfasst wenigstens eine der Schichten 2 oder 3 Metalloxide, Metallhydroxide und/oder Metalloxidhydrate, wobei die Metallionen der Metalloxide, Metallhydroxide und/oder Metalloxidhydrate die Metalle Sn und Fe umfassen oder sind, wobei das Gewichtsverhältnis von Sn zu Fe, jeweils bestimmt mittels RFA und jeweils berechnet als elementares Metall, bevorzugt in einem Bereich von <5, weiter bevorzugt in einem Bereich von <2, besonders bevorzugt in einem Bereich von <1 und ganz besonders bevorzugt in einem Bereich von <0,5 liegt, und wobei der Anteil an Fe, bestimmt mittels RFA und berechnet als elementares Metall, bevorzugt in einem Bereich von 19 Gew.-% bis 83 Gew.-%, bezogen auf das Gesamtgewicht des erfindungsgemäßen rotfarbenen Effektpigments, liegt.

Bei einer weiteren besonders bevorzugten Ausführungsform umfasst wenigstens eine der Schichten 2 oder 3 Metalloxide, Metallhydroxide und/oder Metalloxidhydrate, wobei die Metallionen der Metalloxide, Metallhydroxide und/oder Metalloxidhydrate die Metalle Zr und Fe umfassen oder sind, wobei das Gewichtsverhältnis von Zr zu Fe, jeweils bestimmt mittels RFA und jeweils berechnet als elementares Metall, bevorzugt in einem Bereich von <0,5, weiter bevorzugt in einem Bereich von <0,2, besonders bevorzugt in einem Bereich von <0,12 und ganz besonders bevorzugt in einem Bereich von <0,09 liegt, und wobei der Anteil an Fe, bestimmt mittels RFA und berechnet als elementares Metall, bevorzugt in einem Bereich von 19 Gew.-% bis 61 Gew.-%, bezogen auf das Gesamtgewicht des erfindungsgemäßen rotfarbenen Effektpigments, liegt.

Umfasst das wenigstens eine Metalloxid, Metallhydroxid und/oder Metalloxidhydrat aus den Schichten 2 oder 3 wenigstens zwei unterschiedliche Metallionen, wobei eines der zwei unterschiedlichen Metallionen ein Eisenion ist, so liegen die zwei unterschiedlichen Metallionen vorzugsweise entweder homogen in den Schichten 2 und/oder 3 verteilt vor oder sie bilden hierin einen Gradienten aus. In Ausnahmefällen können die wenigstens zwei unterschiedlichen Metallionen auch inhomogen verteilt in den Schichten 2 und/oder 3 vorliegen.

Bei einer Ausführungsform umfasst eine der beiden Schichten 2 oder 3 lediglich eine Art Metallion, bevorzugt ausgewählt aus der Gruppe der Metalle, bestehend aus Fe, Sn, Ti und Zr, weiter bevorzugt bestehend aus Fe, Sn und Zr. Entsprechend weist die jeweils andere der beiden Schichten 3 oder 2 wenigstens zwei unterschiedliche Metallionen, bevorzugt ausgewählt aus der Gruppe der Metalle, bestehend aus Fe, Sn, Ti und Zr, weiter bevorzugt bestehend aus Fe, Sn und Zr auf.

Bei einer bevorzugten Ausführungsform umfassen sowohl die Schicht 2 als auch die Schicht 3 wenigstens ein Metalloxid, Metallhydroxid und/oder Metalloxidhydrat aus oder mit wenigstens zwei unterschiedlichen Metallionen, bevorzugt ausgewählt aus der Gruppe der Metalle, bestehend aus Fe, Sn, Ti und Zr, weiter bevorzugt bestehend aus Ti, Sn und Fe, wobei wenigstens eines der beiden unterschiedlichen Metallionen in Schicht 2 und Schicht 3 ein Eisenion ist.

Bei einer bevorzugten Ausführungsform sind die durch die Abstandslage unterbrochenen Schichten 2 und 3 in Bezug auf die jeweilige Zusammensetzung identisch.

Die Metalloxid-, Metallhydroxid- und/oder Metalloxidhydratgehalte der erfindungsgemäßen rotfarbenen Effektpigmente werden als jeweiliges Metalloxid mittels Röntgenfluoreszenzanalyse (RFA) bestimmt und können als jeweiliges elementares Metall berechnet werden. Hierzu wird das rotfarbene Effektpigment in eine Lithiumtetraboratglastablette eingearbeitet, in Festprobenmessbechern fixiert und daraus vermessen. Als Messgerät dient das Gerät Advantix ARL, Fa. Thermo Scientific.

Die mittlere Schichtdicke der Schicht 1 beträgt erfindungsgemäß weniger als 10 nm, bevorzugt weniger als 5 nm und besonders bevorzugt weniger als 3 nm, wobei die Schicht 1 das nichtmetallische plättchenförmige Substrat bzw. eine optional vorhandene Beschichtung vollständig umhüllt oder nicht vollständig umhüllt.

Die mittlere Schichtdicke der Schichten 2 und 3 der erfindungsgemäßen rotfarbenen Effektpigmente liegt jeweils bevorzugt in einem Bereich von 30 nm bis 300 nm, weiter bevorzugt jeweils in einem Bereich von 35 nm bis 290 nm, weiter bevorzugt jeweils in einem Bereich von 40 nm bis 270 nm, besonders bevorzugt jeweils in einem Bereich von 40 nm bis 275 nm und ganz besonders bevorzugt jeweils in einem Bereich von 45 nm bis 210 nm.

Bei einer Ausführungsform ist die mittlere Schichtdicke der Schichten 2 und 3 nahezu gleich. Unter einer "nahezu gleichen mittleren Schichtdicken" wird erfindungsgemäß verstanden, dass der Quotient aus der mittleren Schichtdicke der Schicht 2 und der mittleren Schichtdicke der Schicht 3 bevorzugt in einem Bereich von 0,3 bis 2,5, weiter bevorzugt in einem Bereich von 0,4 bis 2,1, besonders bevorzugt in einem Bereich von 0,5 bis 1,9 und ganz besonders bevorzugt in einem Bereich von 0,4 bis 1,6 liegt.

Bei einer weiteren Ausführungsform ist bei stofflich unterschiedlicher Zusammensetzung der Schichten 2 und 3 deren jeweilige optische Schichtdicke annähernd gleich, wobei die optische Schichtdicke der Schichten 2 und 3 der bekannten Lambda/4 Regel folgen kann oder auch nicht. Die optische Schichtdicke ist definiert als Produkt aus Brechungsindex und mittlerer Schichtdicke der jeweiligen Schicht.

Die mittlere Schichtdicke der gesamten Beschichtung der erfindungsgemäßen rotfarbenen Effektpigmente liegt vorzugsweise bei ≤ 600 nm. Bevorzugt liegt die mittlere Schichtdicke der gesamten Beschichtung in einem Bereich von 45 nm bis 550 nm, besonders bevorzugt in einem Bereich von 65 nm bis 480 nm und ganz besonders bevorzugt in einem Bereich von 75 nm bis 350 nm. Unter gesamter Beschichtung wird die komplette Beschichtung, die sich ausgehend von der Substratoberfläche senkrecht von dieser in einer Richtung erstreckt, verstanden.

Bei einer Ausführungsform beträgt die relative Standardabweichung der Schichtdickenverteilung der Schichten 2 und 3 2% bis 74%, bevorzugt 3% bis 63%, besonders bevorzugt 4% bis 57% und ganz besonders bevorzugt 5% bis 49% und die relative Standardabweichung der Schichtdickenverteilung der gesamten Beschichtung 0,3% bis 31%, bevorzugt 1% bis 27% besonders bevorzugt 1,2% bis 24% und ganz besonders bevorzugt 1,9% bis 22%. Die relative Standardabweichung in [%] ist dabei der Quotient aus berechneter Standardabweichung und mittlerer Dicke.

Die Abstandslage zwischen den Schichten 2 und 3 ist vorzugsweise im Wesentlichen parallel zur Oberfläche des nichtmetallischen plättchenförmigen Substrats angeordnet. Unter "im Wesentlichen parallel" wird im Rahmen dieser Erfindung verstanden, dass in einer rasterelektronenmikroskopischen Querschliffaufnahme eine durch eine Abstandslage gelegte Regressionsgerade in Bezug auf eine an die Oberfläche des nichtmetallischen plättchenförmigen Substrats gelegte Regressionsgrade eine Steigung von vorzugsweise nahe Null aufweist.

Die Position der Abstandslage innerhalb der gesamten Beschichtung kann variieren. Sind beispielsweise die mittleren Schichtdicken der Schichten 2 und 3 nahezu identisch, liegt die Abstandslage, in Bezug auf die gesamte Beschichtung, vorzugsweise aus optionaler Schicht 1 sowie den Schichten 2 und 3, etwa in der Mitte der gesamten Beschichtung, da die optionale Schicht 1 bevorzugt äußerst dünn, besonders bevorzugt nur wenige Atomlagen dick, ist. Die Abstandslage ist vorzugsweise, in Bezug auf die gesamte Beschichtung, zwischen dem ersten Sechstel und dem sechsten Sechstel der gesamten Beschichtung angeordnet. Mit dem ersten Sechstel wird hierbei der dem nichtmetallischen plättchenförmigen Substrat zugewandte Anteil und mit dem sechsten Sechstel der dem nichtmetallischen plättchenförmigen Substrat abgewandte Anteil der gesamten Beschichtung bezeichnet (Abbildung 7).

Die zwischen den Schichten 2 und 3 ausgebildete Abstandslage weist vorzugsweise Verbindungen, die auch als Abstandshalter bezeichnet werden können, auf, welche die beidseits der Abstandslage angrenzenden Schichten zum einen miteinander verbinden und zum anderen voneinander auf Abstand halten. Wie aus rasterelektronenmikroskopisch aufgenommenen Querschliffaufnahmen ersichtlich, können diese Verbindungen bzw. Abstandshalter, z.B. in Form von Stegen oder Säulen, im Winkel von etwa 90°, beispielsweise von 80° bis 100°, zur Oberfläche des nichtmetallischen plättchenförmigen Substrat angeordnet sein. Sie können aber auch jeden beliebigen anderen Winkel zwischen 5° und 175° einnehmen. Bevorzugt sind die Abstandshalter, insbesondere Stege, vorzugsweise die Längsachsen der Abstandshalter, bevorzugt Stege, in einem Winkel aus einem Bereich von 15° bis 150° und besonders bevorzugt in einem Winkel aus einem Bereich von 35° bis 135°, jeweils zur Oberfläche des nichtmetallischen plättchenförmigen Substrats angeordnet. Bei der Bestimmung des Winkels bildet die Substratebene den ersten Schenkel. Eine der Außenseiten des jeweils betrachteten Steges bildet den zweiten Schenkel. Ausgehend vom Winkelscheitel der beiden Schenkel wird der eingeschlossene Winkel bestimmt, wobei in der Draufsicht auf die rasterelektronenmikroskopisch aufgenommenen Querschliffaufnahmen 0° links und 180° rechts in der Substratebene liegend angenommen wird.

Die Verbindungen bzw. Abstandshalter können verschiedene geometrische Formen annehmen und sind vorzugsweise gleichmäßig über die gesamte Abstandslage verteilt. Beispielsweise können die Verbindungen bzw. Abstandshalter netzartig, gitterartig, leiterartig, schwammartig oder wabenförmig vorliegen. Teilweise können auch Strukturelemente erkennbar sein, die denen in einem photonischen oder inversen photonischen Kristall, wie beispielsweise aus EP 2 371 908 A2, EP 1 546 063 A1 oder EP 1 121 334 A1 bekannt, ähnlich sind.

Die Verbindungen bzw. Abstandshalter umfassen wenigstens ein Metalloxid, Metallhydroxid und/oder Metalloxidhydrat. Bei einer bevorzugten Ausführungsform umfassen die Verbindungen bzw. Abstandshalter eine identische stoffliche Zusammensetzung wie die sich beidseits der Abstandslage befindlichen Schichten. Auch kann alternativ innerhalb der Verbindungen bzw. Abstandshalter ein Gradient zwischen verschiedenen Metalloxiden, Metallhydroxiden und/oder Metalloxidhydraten ausgebildet sein.

Bei einer bevorzugten Ausführungsform umfassen die Verbindungen bzw. Abstandshalter ein Metalloxid, Metallhydroxid und/oder Metalloxidhydrat, wobei die Metallionen der Metalloxide, Metallhydroxide und/oder Metalloxidhydrate wenigstens zwei Metallionen, ausgewählt aus der Gruppe der Metalle, bestehend aus Fe, Sn, Ti und Zr, weiter bevorzugt aus der Gruppe, bestehend aus Fe, Ti, und Sn, und besonders bevorzugt aus der Gruppe, bestehend aus Fe und Sn, umfassen oder sind, und wenigstens eines der beiden unterschiedlichen Metallionen ein Eisenion ist.

Die Erfinder gehen davon aus, dass die Verbindungen bzw. Abstandshalter auch eine mechanische Stabilisierung der angrenzenden Schichten und somit des erfindungsgemäßen rotfarbenen Effektpigments bewirken können. Vermutlich bildet sich aufgrund der Anzahl an Verbindungen bzw. Abstandshaltern, der verschiedenartigen Winkel und geometrischen Formen, welche die Verbindungen bzw. Abstandshalter innerhalb der Abstandslage einnehmen können, und deren vorzugsweise gleichmäßig flächigen Verteilung innerhalb der Abstandslage, ein mechanisch sehr stabiles Effektpigment aus. Die Haftung zwischen der gesamten Beschichtung und dem nichtmetallischen plättchenförmigen Substrat ist bei den erfindungsgemäßen rotfarbenen Effektpigmenten sehr gut. Selbst extreme Scherbedingungen, wie sie im sogenannten Waring Blender Test auftreten, überstehen die erfindungsgemäßen rotfarbenen Effektpigmente ohne nachweisbare Beschädigung. Die Durchführung des Waring Blender Tests ist nachfolgend in Abschnitt IIf "Waring Blender Test" beschrieben.

Neben ihrer überraschend guten mechanischen Stabilität verfügen die erfindungsgemäßen rotfarbenen Effektpigmente über eine ausgezeichnete Chemikalienbeständigkeit, wie dies gemäß den nachfolgenden Ausführungen im Abschnitt IIg "Bestimmung der Chemikalienbeständigkeit" erläutert wird.

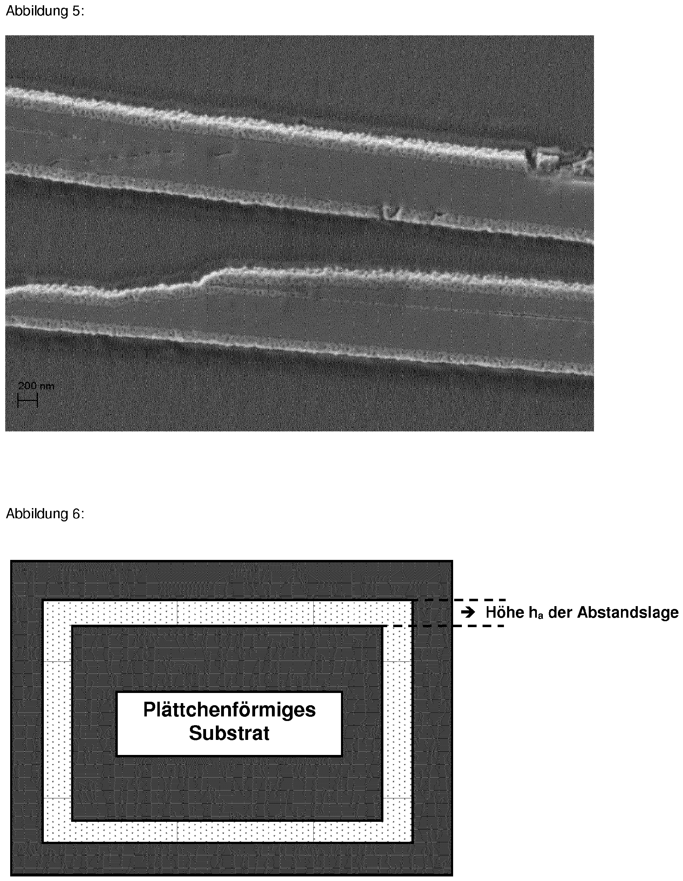

Die Abstandslage der erfindungsgemäßen rotfarbenen Effektpigmente besitzt bevorzugt eine mittlere Höhe $h_a$ aus einem Bereich von 3 nm bis 120 nm, weiter bevorzugt aus einem Bereich von 6 nm bis 99 nm, weiter bevorzugt aus einem Bereich von 14 nm bis 76 nm, weiter bevorzugt aus einem Bereich von 21 nm bis 69 nm, besonders bevorzugt aus einem Bereich von 22 nm bis 62 nm und ganz besonders bevorzugt aus einem Bereich von 26 nm bis 56 nm (Abbildung 6).

Zur Bestimmung der mittleren Höhe $h_a$ der Abstandslage, der mittleren Schichtdicke der Schichten 2 und 3 sowie der mittleren Schichtdicke der gesamten Beschichtung wird anhand von rasterelektronenmikroskopischen Querschliffaufnahmen die obere und untere Substratoberfläche jeweils als Basislinie herangezogen. Mit oberer und unterer Substratoberfläche ist in der rasterelektronenmikroskopischen Querschliffaufnahme jeweils die längere Seite des nichtmetallischen plättchenförmigen Substrats gemeint. Die Basislinie wird in die rasterelektronenmikroskopische Querschliffaufnahme entlang der Oberfläche des nichtmetallischen plättchenförmigen Substrats gelegt. Die rasterelektronenmikroskopischen Querschliffaufnahmen wurden mit Hilfe der Bildverarbeitungssoftware AxioVision 4.6.3. (Fa. Zeiss) untersucht.

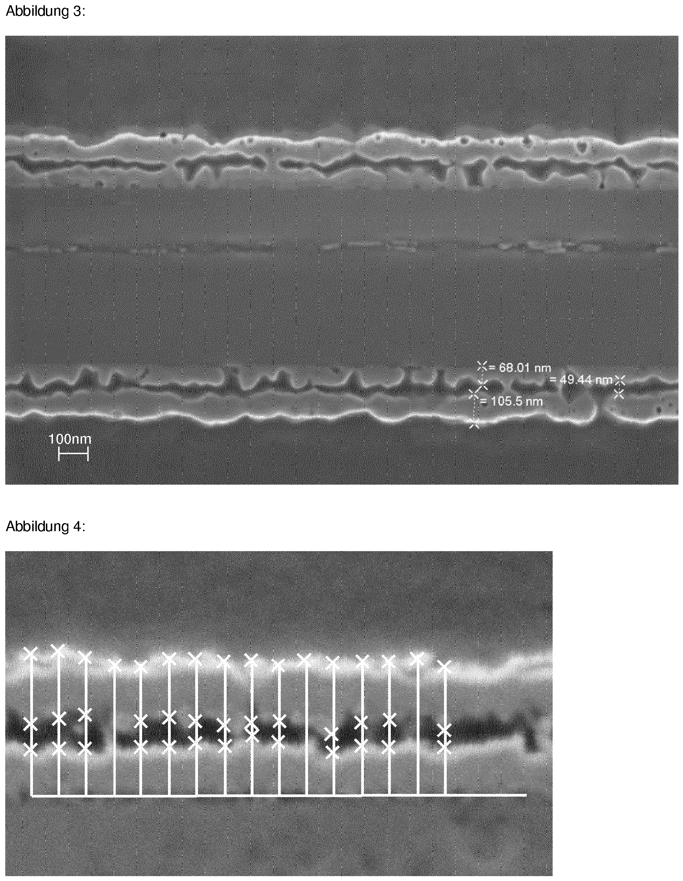

Im 90° Winkel zu diesen beiden Basislinien werden im 50 nm Abstand so viele parallele Linien eingezogen, dass über das in der rasterelektronenmikroskopischen Querschliffaufnahme gezeigte Effektpigment ein Raster gelegt ist (Abbildung 4). Die Vergrößerung der rasterelektronenmikroskopischen Querschliffaufnahme beträgt vorzugsweise mindestens 50000-fach, bezogen auf Polaroid 545 (4" x 5"). Ausgehend von der jeweiligen Basislinie vom nichtmetallischen plättchenförmigen Substrat werden in Richtung der jeweils außenliegenden Schicht 3 bzw. der jeweils äußersten Schicht die Schnittpunkte zwischen den senkrecht zu der jeweiligen Basislinie angeordneten parallelen Linien mit den jeweiligen Grenzflächen der optionalen Schicht 1 zur Schicht 2, der Schicht 2 zur Abstandslage, der Abstandslage zur Schicht 3 und der Schicht 3 zur Umgebung bzw. zu einer etwaig weiter aufgebrachten Schicht manuell vermessen. Hierbei kann es vorkommen, dass eine der im 50 nm Abstand eingezeichneten Linien direkt über einer Verbindungsstelle bzw. einem Abstandshalter zu liegen kommt. In diesem Fall wird nur der jeweilige Schnittpunkt der Linie an der Grenzfläche Schicht 3 zur Umgebung bzw. zu einer etwaig weiter aufgebrachten Schicht erfasst.

Aus diesen Messwerten ergeben sich die Schichtdicken der Schichten 2 und 3, die Schichtdicke der gesamten Beschichtung, die Schichtdicke optional weiter vorhandener Schichten sowie die Höhe $h_a$ der Abstandslage durch Differenzbildung. Die Schichtdicke der Schicht 2 ergibt sich aus der Differenz der jeweiligen vermessenen Schnittpunkte an den jeweiligen Grenzflächen Schicht 2 zur Abstandslage und entweder der optionalen Schicht 1 zur Schicht 2 oder der Basislinie zur Schicht 2, sofern das nichtmetallische plättchenförmige Substrat nicht mit weiteren Schichten vorbelegt ist. Die Schichtdicke der Schicht 3 ergibt sich aus der Differenz der jeweiligen vermessenen Schnittpunkte der Schicht 3 zur Umgebung bzw. einer etwaig weiter aufgebrachten Schicht und der Abstandslage zur Schicht 3. Die Schichtdicke der gesamten Beschichtung ergibt sich aus der Differenz der jeweiligen Schnittpunkte der Schicht 3 zur Umgebung bzw. einer etwaig weiter aufgebrachten Schicht zur Umgebung und der jeweiligen Basislinie. Die Höhe $h_a$ der Abstandslage ergibt sich aus der Differenz der jeweiligen vermessenen Schnittpunkte Abstandslage zur Schicht 3 und Schicht 2 zur Abstandslage. Die Schichtdicken etwaig weiter aufgebrachter Schichten sind analog zu bestimmen und bei der Differenzbildung entsprechend zu berücksichtigen.

Aus den auf die Weise bestimmten Einzelwerten der Schichtdicken bzw. der Höhe $h_a$ werden die jeweiligen arithmetischen Mittelwerte gebildet, um die oben angegebenen Werte der mittleren Schichtdicken bzw. der mittleren Höhe $h_a$ zu bestimmen. Für eine aussagekräftige Statistik werden die oben beschriebenen Messungen an mindestens 100 der senkrecht zur Basislinien angeordneten parallelen Linien durchgeführt.

Die Höhe $h_{ma}$ bezeichnet die Mitte der Abstandslage. Sie ergibt sich als Summe aus der Schichtdicke der optionalen Schicht 1, der Schicht 2 und der halben Höhe $h_a$ der Abstandslage. Die relative Höhe $h_{Rma}$ der Mitte der Abstandslage bildet sich aus dem Verhältnis von $h_{ma}$ und der Schichtdicke der gesamten Beschichtung. Die Standardabweichung der relativen Höhe $\sigma h_{Rma}$ liegt bevorzugt in einem Bereich von 0,2% bis 18%, weiter bevorzugt in einem Bereich von 0,3% bis 15%, besonders bevorzugt in einem Bereich von 0,4% bis 11% und ganz besonders bevorzugt in einem Bereich von 0,5% bis 8%. Die Standardabweichung der relativen Höhe $\sigma h_{Rma}$ ist ein Maß dafür, dass die Abstandslage in einer definierten Position parallel zur Oberfläche des nichtmetallischen plättchenförmigen Substrats innerhalb der gesamten Beschichtung angeordnet ist.

Weisen die erfindungsgemäßen rotfarbenen Effektpigmente wenigstens eine weitere Abstandslage auf, so werden auch deren Höhe(n) $h_{ma}$ sowie deren relative Höhe(n) der Mitte der wenigstens einen weiteren Abstandslage $h_{Rma}$ über das vorstehend beschriebene Verfahren anhand von rasterelektronenmikroskopisch aufgenommenen Querschliffaufnahmen ermittelt. Die vorstehend angegebenen Werte für die Standardabweichung der relativen Höhe $\sigma h_{Rma}$ gelten für weitere Abstandslagen entsprechend.

Dem Fachmann ist bekannt, dass beispielsweise mit Titandioxid beschichtete Perlglanzpigmente in der Beschichtung Poren aufweisen, die über die gesamte Beschichtung statistisch verteilt sind (Abbildung 5). Diese Perlglanzpigmente weisen keine Abstandslage auf. Die Abstandslage sowie die sich innerhalb der Abstandslage befindlichen Hohlräume der erfindungsgemäßen rotfarbenen Effektpigmente sind dagegen nicht statistisch über die gesamte Beschichtung verteilt, sondern sind innerhalb der gesamten Beschichtung parallel zur Oberfläche des nichtmetallischen plättchenförmigen Substrats angeordnet.

Die Abstände der Mittelpunkte der statistisch verteilten Poren zur Substratoberfläche wurden ebenfalls anhand rasterelektronenmikroskopischer Querschliffaufnahmen nach dem vorstehend beschriebenen Verfahren bestimmt. Hierzu wurden im 90° Winkel zu der oberen und unteren Basislinie, die den beiden Oberflächen des plättchenförmigen Substrats entsprechen, im 50 nm Abstand so viele parallele Linien eingezogen, dass über das in der rasterelektronenmikroskopischen Querschliffaufnahme gezeigte Perlglanzpigment ohne Abstandslage ein Raster gelegt war. Sofern eine der parallelen Linien über einer oder mehreren Poren zu liegen kam, wurde deren Höhe(n), deren Porenmittelpunkt(e) und der Abstand des Porenmittelpunkts oder der Porenmittelpunkte zur Substratoberfläche bestimmt. Aus der statistischen Verteilung der Porenmittelpunkte lässt sich ebenfalls eine Standardabweichung ermitteln.

Die Standardabweichung der Abstände der Mittelpunkte der statistisch verteilten Poren zur Substratoberfläche liegt in Perlglanzpigmenten aus dem Stand der Technik, also bei Perlglanzpigmenten ohne Abstandslage, bei > 20%. Die Standardabweichung der Abstände der Mittelpunkte der statistisch verteilten Poren zur Substratoberfläche unterscheidet sich somit im Wert deutlich von der Standardabweichung der relativen Höhe der Mitte der Abstandslage der erfindungsgemäßen rotfarbenen Effektpigmente.

Somit kann die Standardabweichung der Abstände der Porenmittelpunkte zur Substratoberfläche von Perlglanzpigmenten ohne Abstandslage der Standardabweichung der relativen Höhe der Mitte der Abstandslage von erfindungsgemäßen rotfarbenen Effektpigmenten gegenübergestellt werden.

Weiterhin wird mit Hilfe der oben beschriebenen Linien, welche im 50 nm Abstand in eine rasterelektronenmikroskopische Aufnahme eingezeichnet werden, die Anzahl der Verbindungen bzw. Abstandshalter pro Mikrometer sowie die Steganzahldichte, definiert als die Anzahl der Verbindungen bzw. Abstandshalter pro Anzahl der Linien in Prozent, bestimmt.

Weisen die erfindungsgemäßen rotfarbenen Effektpigmente mehr als eine Abstandslage innerhalb der gesamten Beschichtung auf, so wird das soeben beschriebene Verfahren zur Vermessung der einzelnen Schichten und der Abstandslagen entsprechend übertragen.

Bei einer Ausführungsform liegt die Standardabweichung der relativen Höhe der Abstandslage $\sigma h_{Rma}$ bei < 25%, bevorzugt in einem Bereich von 0,5% bis 23% besonders bevorzugt 1% bis 21% und ganz besonders bevorzugt 13% bis 60%.

Bei einer bevorzugten Ausführungsform weisen die erfindungsgemäßen rotfarbenen Effektpigmente innerhalb der wenigstens einen Abstandslage eine Anzahl an Verbindungen bzw. Abstandshaltem pro Mikrometer aus einem Bereich von 0 bis 11, weiter bevorzugt aus einem Bereich von 0 bis 9, besonders bevorzugt aus einem Bereich von 1 bis 7 und ganz besonders bevorzugt aus einem Bereich von 1 bis 3 auf.

Bei einer bevorzugten Ausführungsform weisen die erfindungsgemäßen rotfarbenen Effektpigmente innerhalb der wenigstens einen Abstandslage eine Steganzahldichte, definiert als Anzahl an Verbindungen bzw. Abstandshaltern pro Anzahl der Linien in Prozent, von < 85%, bevorzugt aus einem Bereich von 1% bis 75%, besonders bevorzugt aus einem Bereich von 1% bis 63% und ganz besonders bevorzugt aus einem Bereich von 1% bis 49% auf.

Oberhalb einer Steganzahldichte von 85% wird im Sinne dieser Erfindung nicht mehr von einer Abstandslage gesprochen, da der hohe Anteil an Verbindungen bzw. Abstandshaltern dann zu einer weitestgehend durchgängigen Beschichtung führt.

Bei einer weiteren bevorzugten Ausführungsform umfassen die erfindungsgemäßen rotfarbenen Effektpigmente wenigstens eine, im Wesentlichen parallel zur Oberfläche des nichtmetallischen plättchenförmigen Substrats angeordnete Abstandslage, wobei die wenigstens eine Abstandslage jeweils eine mittlere Höhe $h_a$ aus einem Bereich von 5 nm bis 115 nm, besonders bevorzugt aus einem Bereich von 8 nm bis 76 nm und ganz besonders bevorzugt aus einem Bereich von 16 nm bis 67 nm aufweist.

Bei einer besonders bevorzugten Ausführungsform weisen die erfindungsgemäßen rotfarbenen Effektpigmente wenigstens eine Abstandslage der mittleren Höhe $h_a$ aus einem Bereich von 5 nm bis 112 nm, bevorzugt aus einem Bereich von 9 nm bis 96 nm und ganz besonders bevorzugt aus einem Bereich von 15 nm bis 76 nm auf, wobei innerhalb der wenigstens einen Abstandslage die Anzahl an Verbindungen bzw. Abstandshaltern pro Mikrometer aus einem Bereich von 0 bis 8, bevorzugt aus einem Bereich von 0 bis 6, besonders bevorzugt aus einem Bereich von 0,1 bis 5 und ganz besonders bevorzugt aus einem Bereich von 0,3 bis 4 ausgewählt ist.

Die Abstandslage umfasst neben den vorstehend beschriebenen Verbindungen bzw. Abstandshaltern Hohlräume. Diese Hohlräume werden räumlich durch die Schichten 2 und 3 sowie die Verbindungen bzw. Abstandshalter begrenzt. Die Hohlräume optional weiter vorhandener Abstandslagen weisen analoge Begrenzungen auf.

Eine energiedispersive Röntgenmikroanalyse (EDX-Analyse) dieser Hohlräume lässt keinen Schluss auf feste oder flüssige Materie zu, so dass die Erfinder mit den zum jetzigen Zeitpunkt zur Verfügung stehenden Analysemethoden davon ausgehen, dass die Hohlräume innerhalb der Abstandslage ein Gas, vermutlich Luft, umfassen. Die Verbindungen bzw. Abstandshalter hingegen umfassen wenigstens ein Metalloxid, Metallhydroxid und/oder Metalloxidhydrat, wie vorstehend ausgeführt.

Die Hohlräume innerhalb der Abstandslage der erfindungsgemäßen rotfarbenen Effektpigmente können eine mittlere Höhe $h_H$ aus einem Bereich von 2 nm bis 119 nm, bevorzugt aus einem Bereich von 6 nm bis 105 nm, besonders bevorzugt aus einem Bereich von 11 nm bis 85 nm und ganz besonders bevorzugt aus einem Bereich von 18 nm bis 53 nm, einnehmen. Unter der Höhe $h_H$ wird der größte Abstand zwischen der untersten und obersten Hohlraumbegrenzung verstanden. Sie wird nach dem oben für die Höhe $h_a$ beschriebenen Verfahren bestimmt, indem in rastelektronenmikroskopisch aufgenommenen Querschliffaufnahmen im 90° Winkel zur Oberfläche des nichtmetallischen plättchenförmigen Substrates im 50 nm Abstand parallele Linien eingezeichnet werden. Die Differenz der beiden Schnittpunkte dieser Linien mit der oberen und unteren Hohlraumbegrenzung stellt die Höhe $h_H$ dar. Auch hier werden für eine aussagekräftige Statistik die oben beschriebenen Messungen an mindestens 100 Linien durchgeführt.

Mithin stellt die mittlere Höhe $h_a$ einen Maximalwert für die mittlere Höhe $h_H$ dar. Demnach können innerhalb der Abstandslage auch mehrere Hohlräume übereinander vorliegen.

Die mittlere Höhe der Abstandslage $h_a$ sowie die mittlere Höhe der Hohlräume $h_H$ wird anhand eines gehärteten Lackfilmes, in dem die erfindungsgemäßen rotfarbenen Effektpigmente im Wesentlichen planparallel zum Untergrund ausgerichtet sind, gemäß den Ausführungen in Abschnitt IIj "Bestimmung der mittleren Dicke der nichtmetallischen plättchenförmigen Substrate, der mittleren Schichtdicke der Schichten 2 und 3, der mittleren Schichtdicke der gesamten Beschichtung, der mittleren Höhe $h_a$ der Abstandslage sowie der mittleren Höhe $h_H$ der Hohlräume" bestimmt. Hierzu wird ein Querschliff des gehärteten Lackfilmes unter einem Rasterelektronenmikroskop (REM), wie vorstehend für $h_a$ beschrieben, untersucht. Alternativ zu diesen Querschliffen können die erfindungsgemäßen rotfarbenen Effektpigmente mittels des FIB-Verfahrens (FIB = focused ion beam) angeschnitten werden. Dazu wird ein feiner Strahl aus hochbeschleunigten Ionen (z.B. Gallium, Xenon, Neon oder Helium) mittels einer Ionenoptik auf einen Punkt fokussiert und zeilenweise über die zu bearbeitende Effektpigmentoberfläche geführt. Die Ionen geben beim Aufprall auf die Effektpigmentoberfläche einen Großteil ihrer Energie ab und zerstören die Beschichtung an diesem Punkt, was zu einem zeilenweisen Materialabtrag führt. Auch anhand der dann aufgenommenen rasterelektronenmikroskopischen Aufnahmen kann nach dem oben beschriebenen Verfahren die mittlere Höhe $h_a$, die mittlere Schichtdicke der Schichten 2 und 3 sowie die mittlere Schichtdicke der gesamten Beschichtung ermittelt werden. Auch die mittlere Dicke des nichtmetallischen plättchenförmigen Substrats kann anhand von rasterelektronenmikroskopischen Aufnahmen der durch das FIB-Verfahren angeschnittenen Effektpigmente bestimmt werden.

Bei einer weiteren Ausführungsform umfassen die erfindungsgemäßen rotfarbenen Effektpigmente innerhalb der Abstandslage, verteilt über das gesamte Effektpigment und gemessen anhand rasterelektronenmikroskopischer Querschliffaufnahmen, einen Flächenanteil an Hohlräumen aus einem Bereich von 51% bis 99%, bevorzugt aus einem Bereich von 63% bis 96%, besonders bevorzugt aus einem Bereich von 76% bis 95% und ganz besonders bevorzugt aus einem Bereich von 84% bis 94% und einen Flächenanteil an Verbindungen bzw. Abstandhaltern aus einem Bereich von 1% bis 49%, bevorzugt aus einem Bereich von 4% bis 37%, besonders bevorzugt aus einem Bereich von 5% bis 24% und ganz besonders bevorzugt aus einem Bereich von 6% bis 16%.

Weiterhin ist bevorzugt, dass in der Abstandslage das durch die Verbindungen bzw. Abstandshalter eingenommene Gesamtvolumen kleiner ist als das durch die Hohlräume eingenommene Gesamtvolumen.

Bevorzugt beträgt in der Abstandslage das durch die Verbindungen bzw. Abstandshalter eingenommene Gesamtvolumen weniger als 50 Vol.-%, weiter bevorzugt weniger als 30 Vol.-%, besonders bevorzugt weniger als 20 Vol.-% und ganz besonders bevorzugt weniger als 10 Vol.-% des durch die Hohlräume eingenommenen Gesamtvolumens.

Bei den erfindungsgemäßen rotfarbenen Effektpigmenten sind die innerhalb der Abstandslage liegenden Hohlräume im Unterschied zu den Poren der Lehre nach EP 1 422 268 A2 ausdrücklich gewünscht. Gemäß EP 1 422 268 A2 ist eine Beschichtung mit geringer Porosität und möglichst kleinen Poren zum Erhalt von Pigmenten mit hohem Chroma und hoher Brillanz erforderlich. Die Pigmente gemäß EP 1 422 268 A2 weisen keine Abstandslage auf. Erfindungsgemäß nehmen die nicht willkürlich innerhalb der gesamten Beschichtung verteilten, sondern sich im Wesentlichen parallel zur Oberfläche des nichtmetallischen plättchenförmigen Substrats innerhalb der Abstandslage befindlichen Hohlräume keinen negativen Einfluss auf die optischen Eigenschaften der erfindungsgemäßen rotfarbenen Effektpigmente. Im Gegenteil, die erfindungsgemäßen rotfarbenen Effektpigmente zeichnen sich gegenüber einschichtig beschichteten Pigmenten durch einen höheren Glanz sowie ein höheres Chroma aus, gleiches nichtmetallisches plättchenförmiges Substrat, gleiche Teilchengröße und vergleichbare Beschichtung selbstverständlich vorausgesetzt.

Dabei können in Abhängigkeit der Beschichtungsdicke sowie der Art der Beschichtung unterschiedliche Interferenzfarben und/oder unterschiedliche Absorptionsfarben erhalten werden.

Erklären lassen sich der höhere Glanz und das höhere Chroma aus dem maximalen Brechungsindexunterschied zwischen der Abstandslage und den daran angrenzenden Schichten, was gemäß Fresnelschen Gesetz jeweils zu einer maximalen Lichtreflexion an diesen Grenzflächen führt. Für die Hohlräume wird hierbei der Brechungsindex von Luft von annähernd 1 zugrunde gelegt. Ein an der Abstandslage auftreffender Lichtstrahl wird an deren Grenzflächen teilreflektiert, wobei die jeweilige Intensität der Reflexion nach dem Fresnelschen Gesetz abhängig vom Brechungsindexunterschied der angrenzenden Schichten zur Abstandslage ist. Da an jeder einzelnen Grenzfläche eine solche Teilreflexion stattfindet, nimmt auch die Gesamtreflexion mit der Anzahl der Grenzflächen zu. Bei den erfindungsgemäßen rotfarbenen Effektpigmenten wird ein Lichtstrahl somit mehrfach teilreflektiert, was sich in einem deutlich stärkeren Glanz und einer stärkeren Intensität der Interferenzfarbe im Vergleich zu herkömmlichen, einfach beschichteten Pigmenten auswirkt.

Sind die Hohlräume innerhalb der gesamten Beschichtung statistisch verteilt, also nicht im Wesentlichen parallel zum nichtmetallischen plättchenförmigen Substrat, variiert die optische Weglänge innerhalb der gesamten Beschichtung. Dies hat zur Folge, dass die Interferenzbedingungen nicht ausreichend erfüllt sind und damit keine Verstärkung oder Auslöschung erfolgt.

Der Glanz der erfindungsgemäßen rotfarbenen Effektpigmente wird anhand von Schwarz-Weiß-Deckungskarten mit Hilfe eines Micro-Tri-Gloss Glanzmessgerätes, Fa. Byk-Gardner, gemäß den nachfolgenden Ausführungen im Abschnitt IId "Glanzmessungen" bestimmt. Das Chroma der erfindungsgemäßen rotfarbenen Effektpigmente wird ebenfalls anhand von Schwarz-Weiß-Deckungskarten mit dem Mehrwinkelfarbmessgerät BYK-mac (Fa. Byk-Gardner) gemäß den nachfolgenden Ausführungen im Abschnitt IIb "Winkelabhängige Farbmessungen" bestimmt. Weitere optische Effekte, wie Glitzer und Körnigkeit, werden gemäß den nachfolgenden Ausführungen im Abschnitt IIe "Effektmessungen" bestimmt.

Bei einer Ausführungsform umfassen die erfindungsgemäßen rotfarbenen Effektpigmente neben den vorstehend beschriebenen Schichten 1, 2 und 3 weitere hoch- und/oder niedrigbrechende Schichten, die, betrachtet vom nichtmetallischen plättchenförmigen Substrat aus, entweder unterhalb der optionalen Schicht 1 bzw. der Schicht 2 und/oder oberhalb der Schicht 3 angeordnet sein können. Diese weiteren Schichten können Metalloxide, Metallhydroxide, Metalloxidhydrate umfassen, wobei die Metallionen der Metalloxide, Metallhydroxide, Metalloxidhydrate wenigstens ein Metallion, ausgewählt aus der Gruppe der Metalle, bestehend aus Ti, Fe, Sn, Mn, Zr, Ca, Sr, Ba, Ni, Ag, Zn, Cu, Ce, Cr und Co, bevorzugt ausgewählt aus der Gruppe der Metalle, bestehend aus Ti, Fe, Sn, Zr, Ag, Zn, Cu, Ce, Cr und besonders bevorzugt ausgewählt aus der Gruppe der Metalle, bestehend aus Ti, Fe und Sn umfassen oder sind. Außerdem können diese weiteren Schichten semitransparente Metalle, ausgewählt aus der Gruppe, bestehend aus Ag, Al, Cr, Ni, Au, Pt, Pd, Cu, Zn und Ti, bevorzugt ausgewählt aus der Gruppe, bestehend aus Ag, Au und Cu, jeweils deren Legierungen und/oder Mischungen hiervon umfassen.

Bei einer Ausführungsform kann jede der Schichten der erfindungsgemäßen rotfarbenen Effektpigmente mit einer Dotierung versehen sein, wobei die Dotierung Metalloxide, Metallhydroxide und/oder Metalloxidhydrate umfassen kann, und die Metallionen der Metalloxide, Metallhydroxide, und/oder Metalloxidhydrate wenigstens ein Metallion, ausgewählt aus der Gruppe der Metalle, bestehend aus Ca, Mg, Al, Ce, Zr oder Sn, bevorzugt Al, Zr oder Sn, umfasst oder ist. Der Anteil an Dotierung liegt bevorzugt bei insgesamt $\leq 1$ Gew.-%, besonders bevorzugt bei insgesamt $\leq 0,5$ Gew.-% und ganz besonders bevorzugt bei insgesamt $\leq 0,2$ Gew.-%, jeweils bezogen auf das Gesamtgewicht der rotfarbenen Effektpigmente.

Bei einer weiteren Ausführungsform kann die gesamte Beschichtung der erfindungsgemäßen rotfarbenen Effektpigmente neben der Abstandslage zwischen den Schichten 2 und 3 wenigstens eine weitere Abstandslage umfassen, welche auch im Wesentlichen parallel zur Oberfläche des nichtmetallischen plättchenförmigen Substrats angeordnet ist. Vorzugsweise weisen die erfindungsgemäßen rotfarbenen Effektpigmente nicht mehr als vier Abstandslagen innerhalb der gesamten Beschichtung auf, da deren optische Qualität dann abnimmt. Erfindungsgemäß befindet sich, auch wenn das erfindungsgemäße rotfarbene Effektpigment mehr als eine Abstandlage umfasst, in Bezug auf die gesamte Beschichtung weder im ersten Sechstel noch im sechsten Sechstel der gesamten Beschichtung eine Abstandslage.

Die erfindungsgemäßen rotfarbenen Effektpigmente können eine beliebige mittlere Teilchengröße $D_{50}$ aufweisen. Die Dso-Werte der erfindungsgemäßen rotfarbenen Effektpigmente liegen vorzugsweise in einem Bereich von 3 $\mu$m bis 350 $\mu$m. Bevorzugt liegen die $D_{50}$-Werte der erfindungsgemäßen rotfarbenen Effektpigmente in einem Bereich von 4 $\mu$m bis 211 $\mu$m, weiter bevorzugt in einem Bereich von 6 $\mu$m bis 147 $\mu$m, besonders bevorzugt in einem Bereich von 7 $\mu$m bis 99 $\mu$m und ganz besonders bevorzugt in einem Bereich von 8 $\mu$m bis 56 $\mu$m. Äußerst bevorzugt weisen die erfindungsgemäßen rotfarbenen Effektpigmente einen $D_{50}$-Wert aus einem Bereich von 3 $\mu$m bis 15 $\mu$m oder aus einem Bereich von 10 $\mu$m bis 35 $\mu$m oder aus einem Bereich von 25 $\mu$m bis 45 $\mu$m oder aus einem Bereich von 30 $\mu$m bis 65 $\mu$m oder aus einem Bereich von 40 $\mu$m bis 140 $\mu$m oder aus einem Bereich von 135 $\mu$m bis 250 $\mu$m auf.

Die $D_{10}$-Werte der erfindungsgemäßen rotfarbenen Effektpigmente umfassen vorzugsweise einen Bereich von 1 $\mu$m bis 120 $\mu$m. Besonders bevorzugt liegen die $D_{10}$-Werte der erfindungsgemäßen rotfarbenen Effektpigmente in einem Bereich von 1 $\mu$m bis 5 $\mu$m oder in einem Bereich von 5 $\mu$m bis 25 $\mu$m oder in einem Bereich von 10 $\mu$m bis 30 $\mu$m oder in einem Bereich von 20 $\mu$m bis 45 $\mu$m oder in einem Bereich von 25 $\mu$m bis 65 $\mu$m oder in einem Bereich von 75 bis 110 $\mu$m.

Die $D_{90}$-Werte der erfindungsgemäßen rotfarbenen Effektpigmente umfassen vorzugsweise einen Bereich von 6 $\mu$m bis 500 $\mu$m. Besonders bevorzugt liegen die $D_{90}$-Werte der erfindungsgemäßen rotfarbenen Effektpigmente in einem Bereich von 8 $\mu$m bis 250 $\mu$m oder in einem Bereich von 10 $\mu$m bis 150 $\mu$m oder in einem Bereich von 40 $\mu$m bis 70 $\mu$m oder in einem Bereich von 68 $\mu$m bis 110 $\mu$m oder in einem Bereich von 120 $\mu$m bis 180 $\mu$m oder in einem Bereich von 400 bis 490 $\mu$m.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der vermessenen Effektpigmente einen volumengemittelten Durchmesser aufweisen, der gleich oder kleiner als der jeweils angegebene Wert ist. Hierbei wird die Größenverteilungskurve der erfindungsgemäßen rotfarbenen Effektpigmente mit dem Gerät Mastersizer 2000 der Fa. Malvern gemäß Herstellerangaben bestimmt. Die Auswertung der Streulichtsignale erfolgt nach der Fraunhofer-Theorie, welche auch Brechungs- und Absorptionsverhalten der Partikel beinhaltet.

Bei einer bevorzugten Ausführungsform besitzen die erfindungsgemäßen rotfarbenen Effektpigmente einen

$$\Delta D = \frac{D_{90} - D_{10}}{D_{50}}$$

Span $\Delta D$, definiert als , aus einem Bereich von 0,7 bis 3,0 bevorzugt aus einem Bereich von 0,7 bis 2,3 weiter bevorzugt aus einem Bereich von 0,8 bis 1,8, besonders bevorzugt aus einem Bereich von 0,8 bis 1,4 und ganz besonders bevorzugt aus einem Bereich von 0,85 bis 1,1. Die Vorteile einer engen Größenklassierung in Bezug auf Farbreinheit und/oder Glanz der resultierenden Effektpigmente werden beispielsweise in EP 2 217 664 A1, EP 2 346 950 A1, EP 2 356 181 A1, EP 2 346 949 A1 oder EP 2 367 889 A1 beschrieben.

Die erfindungsgemäßen rotfarbenen Effektpigmente können wie folgt hergestellt werden:

- Suspendieren der nichtmetallischen plättchenförmigen Substrate in Wasser bei einer Temperatur aus einem Bereich von 50°C bis 100°C,
- optional Aufbringen einer nicht kalzinierten Schicht, die Zinnoxid, Zinnhydroxids und/oder Zinnoxidhydrat umfasst oder daraus besteht, durch Zugabe eines wasserlöslichen Zinnsalzes bei gleichzeitiger Zugabe einer mineralischen Lauge,
- Sequenzielles Aufbringen von drei nicht kalzinierten Schichten A, B und C in Form von Metalloxiden, Metallhydroxiden und/oder Metalloxidhydraten durch sequenzielle Zugabe von drei wasserlöslichen Metallsalzen, jeweils bei gleichzeitiger Zugabe mineralischer Lauge, wobei das zweite wasserlösliche Metallsalz - zur Erzeugung der Schicht B - in Bezug auf das Metallion verschieden von den beiden anderen wasserlöslichen Metallsalzen zur Erzeugung der Schicht A bzw. Schicht C ist, das jeweilige wasserlösliche Metallsalz zur Erzeugung der Schichten A und C jeweils wenigstens ein Metallion, ausgewählt aus der Gruppe der Metalle, bestehend aus Fe, Ti und Zr, umfasst oder ist, das wasserlösliche Metallsalz zur Erzeugung der Schicht B wenigstens ein Metallion, ausgewählt aus der Gruppe der Metalle, bestehend aus Sn, Ti und Zr, umfasst oder ist, und das wasserlösliche Metallsalz bzw. die wasserlöslichen Metallsalze zur Erzeugung der Schichten A und/oder C wenigstens ein Eisenion umfasst oder ist bzw. umfassen oder sind,
- Abtrennen der beschichteten Substrate von der/den Beschichtungslösung(en), optional Waschen und/oder optional Trocknen der beschichteten Substrate,
- Kalzinieren der beschichteten Substrate bei Temperaturen aus einem Bereich von 400°C bis 1100°C, bevorzugt aus einem Bereich von 625°C bis 930°C und besonders bevorzugt aus einem Bereich von 750°C bis 890°C unter Erhalt der erfindungsgemäßen rotfarbenen Effektpigmente umfassend wenigstens eine Abstandslage.

Bei einer bevorzugten Ausführungsform werden die erfindungsgemäßen rotfarbenen Effektpigmente nach dem vorstehenden Verfahren hergestellt.

Das Aufbringen, vorzugsweise Abscheiden, der jeweiligen Metalloxide, Metallhydroxide und/oder Metalloxidhydrate erfolgt vorzugsweise bei konstantem pH-Wert in einem Bereich von pH 1,4 bis 10,0 in Abhängigkeit vom Metallsalz.

Zusätzlich zu den wenigstens drei sequenziell aufgebrachten, vorzugsweise abgeschiedenen, Metalloxiden, Metallhydroxiden und/oder Metalloxidhydraten können selbstverständlich weitere Metalloxide, Metallhydroxide und/oder Metalloxidhydrate vorher und/oder nachfolgend aufgebracht werden, so dass unterhalb bzw. oberhalb der Schichtenfolge [optionale Schicht 1/Schicht 2/Abstandslage/Schicht 3] weitere Schichten angeordnet sein können.

Beim Kalzinieren diffundieren überraschenderweise vermutlich die in der Schicht B vorhandenen Metallionen in die Schicht A und/oder Schicht C unter Ausbildung von gemischten Metalloxiden und/oder gemischten Metallhydroxiden und/oder Metalloxidhydraten und/oder Mischungen von Metalloxiden und/oder Metallhydroxiden und/oder Metalloxidhydraten in der Schicht A und/oder Schicht C. Aufgrund der Diffusion der Metallionen aus der Schicht B in die Schicht A und/oder Schicht C werden beim Kalzinieren die erfindungsgemäßen Schichten 2 und 3 sowie die dazwischenliegende Abstandslage ausgebildet, wobei wenigstens eine der beiden Schichten 2 und 3 wenigstens zwei unterschiedliche Metallionen umfasst. Aus den ursprünglich drei nacheinander abgeschiedenen Schichten A, B und C entstehen somit beim Kalzinieren die Schichten 2 und 3 sowie die dazwischen liegende Abstandslage, wobei wenigstens eine der beiden Schichten 2 und 3 wenigstens zwei unterschiedliche Metallionen, ausgewählt aus der Gruppe der Metalle, bestehend aus Fe, Sn, Ti und Zr, und wenigstens eine der Schichten 2 oder 3 Eisenionen umfasst.

Es wird angenommen, dass unter anderem die unterschiedliche Mobilität der Metalloxide, Metallhydroxide und/oder Metalloxidhydrate zueinander beim Kalzinieren für die Ausbildung der Abstandslage verantwortlich ist. Hierbei konkurriert die Mobilität der in der Schicht B enthaltenen Metallionen mit der Mobilität der in den Schichten A und/oder C enthaltenen Metallionen, vorausgesetzt die Metallionen diffundieren aus der Schicht B in wenigstens eine der angrenzenden Schichten A und/oder C und die Metallionen aus wenigstens einer der Schichten A und/oder C diffundieren in die Schicht B. Die Erfinder gehen zum gegenwärtigen Zeitpunkt davon aus, dass, sofern die Mobilität der in der Schicht B enthaltenen Metallionen während der Kalzination höher ist als die Mobilität der in den Schichten A und/oder C enthaltenen Metallionen, dies eine der möglichen Erklärungen für die Ausbildung der Abstandslage ist. Des Weiteren wird davon ausgegangen, dass ein Konzentrationsgefälle in Bezug auf die Metallionen die Ausbildung einer Abstandslage begünstigt, d.h. wenn mehr mobile Metallionen aus der Schicht B in eine der angrenzenden Schichten A und/oder C diffundieren können als umgekehrt. Zusammenfassend wurde festgestellt, dass die Ausbildung einer Abstandslage durch ein komplexes Zusammenspiel unterschiedlichster weiterer Faktoren, wie beispielsweise entropischen und/oder enthalpischen Effekten, während der Kalzination hervorgerufen wird, welche jedoch noch nicht abschließend geklärt sind. Für die Ausbildung wenigstens einer weiteren Abstandslage gelten vorstehende Überlegungen selbstverständlich entsprechend.

Das erste und das dritte der drei sequenziell aufgebrachten, vorzugsweise abgeschiedenen, Metalloxide, Metallhydroxide und/oder Metalloxidhydrate umfasst wenigstens ein Metallion, ausgewählt aus der Gruppe der Metalle, bestehend aus Fe, Ti, und Zr, wobei das erste und/oder das dritte aufgebrachte Metalloxid, Metallhydroxid und/oder Metalloxidhydrat Eisenionen umfasst. Das erste und das dritte Metalloxid, Metallhydroxid und/oder Metalloxidhydrat erzeugen nach dem Aufbringen die Schicht A bzw. Schicht C. Das zweite der drei sequenziell aufgebrachten, vorzugsweise abgeschiedenen, Metalloxide, Metallhydroxide und/oder Metalloxidhydrate erzeugt die Schicht B und umfasst wenigstens ein Metallion, ausgewählt aus der Gruppe der Metalle, bestehend aus Sn, Ti und Zr, das verschieden von den Metallionen der zur Erzeugung der Schicht A und Schicht C abgeschiedenen Metalloxide, Metallhydroxide und/oder Metalloxidhydrate ist. In der Schicht A und der Schicht C können die aufgebrachten, vorzugsweise abgeschiedenen, Metalloxide, Metallhydroxide und/oder Metalloxidhydrate in Bezug auf das oder die Metallion(en) gleich oder voneinander verschieden sein, wobei wenigstens eine der Schichten A oder C Eisenionen umfasst.

Alternativ können die erfindungsgemäßen rotfarbenen Effektpigmente wie folgt hergestellt werden:

- Suspendieren der kalzinierten einfach oder mehrfach beschichteten nichtmetallischen plättchenförmigen Substrate in Wasser bei einer Temperatur aus einem Bereich von 50°C bis 100°C, wobei das oder die Metallion(en) des Metalloxids, Metallhydroxids und/oder Metalloxidhydrats der Schicht, welche in Richtung Substrat unmittelbar an die aufzubringende Schicht B angrenzt, wenigstens ein Metallion, ausgewählt aus der Gruppe der Metalle, bestehend aus Fe, Ti und Zr, umfasst oder ist,

- sequenzielles Aufbringen von zwei nicht kalzinierten Schichten B und C in Form von Metalloxiden, Metallhydroxiden und/oder Metalloxidhydraten durch sequenzielle Zugabe von zwei wasserlöslichen Metallsalzen, jeweils bei gleichzeitiger Zugabe mineralischer Lauge, wobei das erste wasserlösliche Metallsalz - zur Erzeugung der Schicht B - in Bezug auf das Metallion verschieden von dem anderen wasserlöslichen Metallsalz zur Erzeugung der Schicht C und der Schicht, welche in Richtung Substrat unmittelbar an die Schicht B angrenzt, ist, das wasserlösliche Metallsalz zur Erzeugung der Schicht B wenigstens ein Metallion, ausgewählt aus der Gruppe der Metalle, bestehend aus Ti, Sn und Zr, umfasst oder ist, das wasserlösliche Metallsalz zur Erzeugung der Schicht C wenigstens ein Metallion, ausgewählt aus der Gruppe der Metalle, bestehend aus Fe, Ti, Zr, umfasst oder ist, wenigstens ein Metallion der Schicht, welche in Richtung Substrat unmittelbar an die Schicht B angrenzt, und/oder der Schicht C ein Eisenion umfasst oder ist,

- Abtrennen der beschichteten Substrate von der/den Beschichtungslösung(en), optional Waschen und/oder optional Trocknen der beschichteten Substrate,

- Kalzinieren der beschichteten Substrate bei Temperaturen aus einem Bereich von 400°C bis 1100°C, bevorzugt aus einem Bereich von 625°C bis 930°C und besonders bevorzugt aus einem Bereich von 750°C bis 890°C unter Erhalt der erfindungsgemäßen rotfarbenen Effektpigmente umfassend wenigstens eine Abstandslage.

Das Aufbringen, vorzugsweise Abscheiden, der jeweiligen Metalloxide, Metallhydroxide und/oder Metalloxidhydrate erfolgt auch hier vorzugsweise bei konstantem pH-Wert in einem Bereich von pH 1,4 bis 10,0 in Abhängigkeit vom Metallsalz.

Es wird vermutet, dass beim Kalzinieren die in der Schicht B vorhandenen Metallionen wenigstens in die Schicht C unter Ausbildung von gemischten Metalloxiden und/oder gemischten Metallhydroxiden und/oder Metalloxidhydraten und/oder Mischungen von Metalloxiden und/oder Metallhydroxiden und/oder Metalloxidhydraten in der Schicht C diffundieren. Aufgrund der Diffusion der Metallionen aus der Schicht B in die Schicht C werden beim Kalzinieren die erfindungsgemäße Schicht 3 sowie die Abstandslage ausgebildet. Aus den ursprünglich zwei nacheinander abgeschiedenen Schichten B und C entstehen somit beim Kalzinieren die Schicht 3 sowie die Abstandslage, wobei wenigstens die Schicht 3 wenigstens zwei unterschiedliche Metallionen, ausgewählt aus der Gruppe der Metalle, bestehend aus Fe, Sn, Ti und Zr, umfasst. Die Schicht 2 ist hier bereits vorhanden. Als Schicht 2 wird die äußerste Schicht des als Ausgangsmaterial eingesetzten kalzinierten einfach oder mehrfach beschichteten nichtmetallischen plättchenförmigen Substrats bezeichnet, wobei das oder die Metallion(en) des Metalloxids, Metallhydroxids und/oder Metalloxidhydrats der Schicht 2 wenigstens ein Metallion, ausgewählt aus der Gruppe der Metalle, bestehend aus Fe, Ti und Zr, und wenigstens eine der Schichten 2 oder 3 Eisenionen umfasst.

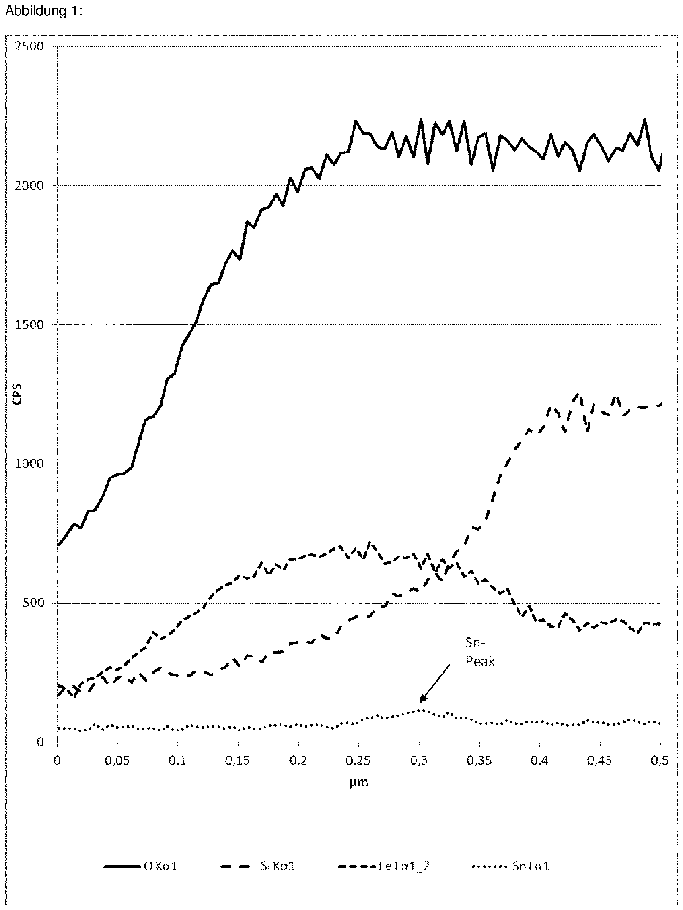

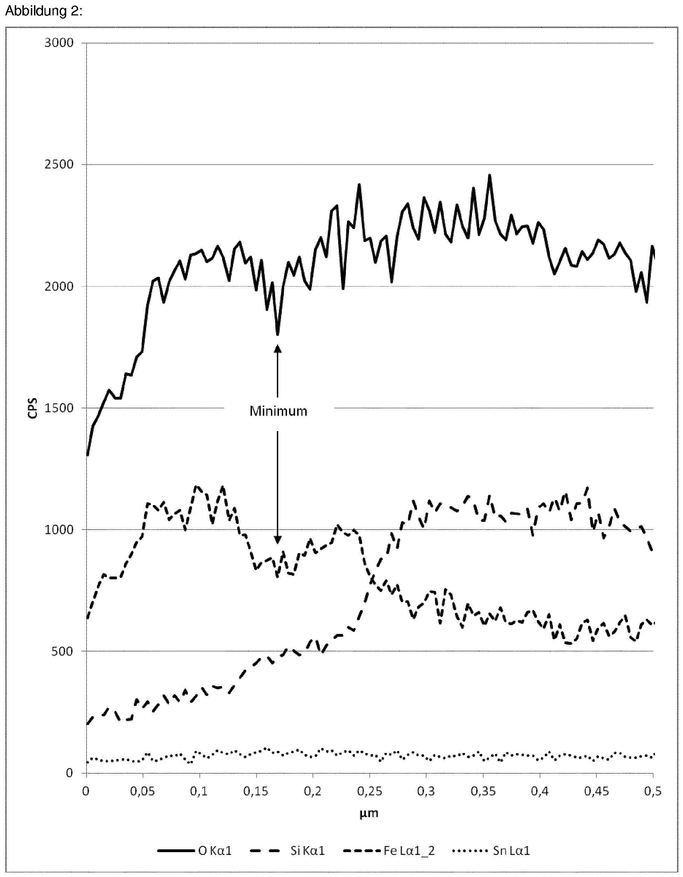

Das Konzentrationsprofil (Line-Scan) anhand von Querschliffen im Rasterelektronenmikroskop mit einem energiedispersiven Mikrobereichsanalysator (EDX) zeigt eine signifikante ortsabhängige Änderung der chemischen Zusammensetzung der Beschichtung vor und nach der Kalzination (Abbildungen 1 und 2).

In Abbildung 1 ist ein Konzentrationsprofil von Beispiel 1 nach Beschichtung und Trocknung, jedoch noch vor der Kalzination dargestellt. Anhand der Konzentrationskurven kann man erkennen, dass sich im Bereich von 0,3 μm ein Sn-Maximum (Sn-Peak) befindet. Dieser Sn-Peak zeigt an wo sich die aufgebrachte Sn-haltige Schicht innerhalb der Beschichtung vor der Kalzination befindet. Die Konzentrationskurve von Sauerstoff ist hingegen weitgehend homogen verteilt, ohne erkennbare Minima bzw. Maxima.

In Abbildung 2 ist ebenfalls ein Konzentrationsprofil von Beispiel 1 nach Beschichtung und Trocknung, jedoch nach Kalzination dargestellt. Es zeigt sich, dass der in Abbildung 1 vorhandene Sn-Peak verschwunden ist. Die Sn-Ionen sind in die umgebenden Fe-haltigen Schichten eindiffundiert. Hervorzuheben ist darüber hinaus ein Minimum der Sauerstoff- und Eisenkonzentrationskurven, welches die Position der Abstandslage indiziert.

Bei einer besonders bevorzugten Ausführungsform umfassen die zwei oder drei sequenziell aufgebrachten, vorzugsweise abgeschiedenen, Metalloxide, Metallhydroxide und/oder Metalloxidhydrate zur Erzeugung der Schichten B und C bzw. A, B und C, kein(e) Metallion(en), ausgewählt aus der Gruppe der Metalle, bestehend aus Si, Mg und Al.

Bei der sequenziellen Aufbringung von zwei nicht kalzinierten Schichten B und C auf ein bereits beschichtetes und gegebenenfalls kalziniertes Substrat umfasst diejenige Schicht, auf welche die Schicht B aufgebracht wird, erfindungsgemäß ein hochbrechendes Metalloxid, Metallhydroxid und/oder Metalloxidhydrat, wobei dessen wenigstens ein Metallion ein Metallion, ausgewählt aus der Gruppe der Metalle, bestehend aus Fe, Ti und Zr, umfasst oder ist.

Bei der sequenziellen Aufbringung von drei nicht kalzinierten Schichten A, B und C auf ein bereits beschichtetes und gegebenenfalls kalziniertes Substrat kann diejenige Schicht, auf welche die Schicht A aufgebracht wird, erfindungsgemäß ein hochbrechendes oder ein niedrigbrechendes Metalloxid, Metallhydroxid und/oder Metalloxidhydrat umfassen.

Bei einer bevorzugten Ausführungsform umfassen die erfindungsgemäßen rotfarbenen Effektpigmente einen Anteil an Eisenionen von insgesamt wenigstens 17 Gew.-%, bevorzugt aus einem Bereich von insgesamt 19 Gew.-% bis insgesamt 76 Gew.-%, weiter bevorzugt aus einem Bereich von insgesamt 21 Gew.-% bis insgesamt 65 Gew.-%, besonders bevorzugt aus einem Bereich von insgesamt 22 Gew.-% bis insgesamt 56 Gew.-%, besonders bevorzugt aus einem Bereich von insgesamt 23 Gew.-% bis insgesamt 47 Gew.-% und einen Anteil an Zinn-, Titan- und Zirkoniumionen von ingesamt ≤ 20 Gew.-%, bevorzugt aus einem Bereich von insgesamt 0,5 Gew.-% bis insgesamt 19 Gew.-%, weiter bevorzugt aus einem Bereich von insgesamt 0,8 Gew.-% bis insgesamt 14 Gew.-%, besonders bevorzugt aus einem Bereich von insgesamt 0,9 Gew.-% bis insgesamt 15 Gew.-%, ganz besonders bevorzugt aus einem Bereich von insgesamt 1,3 Gew.-% bis insgesamt 14 Gew.-%, jeweils bestimmt mittels RFA, jeweils berechnet als elementares Metall und jeweils bezogen auf das Gesamtgewicht des rotfarbenen Effektpigments.

Bei einer besonders bevorzugten Ausführungsform umfassen die erfindungsgemäßen rotfarbenen Effektpigmente einen Anteil an Eisenionen von insgesamt wenigstens 17 Gew.-%, bevorzugt aus einem Bereich von insgesamt 19 Gew.-% bis insgesamt 76 Gew.-%, weiter bevorzugt aus einem Bereich von insgesamt 21 Gew.-% bis insgesamt 65 Gew.-%, besonders bevorzugt aus einem Bereich von insgesamt 22 Gew.-% bis insgesamt 56 Gew.-%, besonders bevorzugt aus einem Bereich von insgesamt 23 Gew.-% bis insgesamt 47 Gew.-% und gleichzeitig einen Anteil an

    i. Zinnionen von insgesamt ≤ 17 Gew.-%, bevorzugt aus einem Bereich von insgesamt 0,3 Gew.-% bis insgesamt 11 Gew.-%, weiter bevorzugt aus einem Bereich von insgesamt 0,4 Gew.-% bis insgesamt 8,5 Gew.-%, besonders bevorzugt aus einem Bereich von insgesamt 0,5 Gew.-% bis 6 Gew.-%, ganz besonders bevorzugt aus einem Bereich von insgesamt 0,2 Gew.-% bis 7 Gew.-%, oder

    ii. Titanionen von insgesamt ≤ 15 Gew.-%, bevorzugt aus einem Bereich von insgesamt 0,1 Gew.-% bis insgesamt 14 Gew.-%, weiter bevorzugt aus einem Bereich von insgesamt 0,2 Gew.-% bis insgesamt 11 Gew.-%, besonders bevorzugt aus einem Bereich von insgesamt 0,5 Gew.-% bis insgesamt 9 Gew.-%, ganz besonders bevorzugt aus einem Bereich von insgesamt 0,8 Gew.-% bis insgesamt 5 Gew.-%, oder

    iii. Zirkoniumionen von insgesamt ≤ 18 Gew.-%, bevorzugt aus einem Bereich von insgesamt 0,1 Gew.-% bis insgesamt 13 Gew.-%, weiter bevorzugt aus einem Bereich von insgesamt 0,5 Gew.-% bis insgesamt 12 Gew.-%, besonderes bevorzugt aus einem Bereich von insgesamt 0,8 Gew.-% bis insgesamt 7 Gew.-%, ganz besonders bevorzugt aus einem Bereich von insgesamt 1,2 Gew.-% bis insgesamt 10 Gew.-%, oder

    iv. Zinn- und Titanionen von insgesamt ≤ 19 Gew.-%, bevorzugt aus einem Bereich von insgesamt 0,3 Gew.-% bis insgesamt 14 Gew.-%, weiter bevorzugt aus einem Bereich von insgesamt 0,5 Gew.-% bis insgesamt 13 Gew.-%, besonders bevorzugt aus einem Bereich von insgesamt 1,1 Gew.-% bis insgesamt 9 Gew.-%, ganz besonders bevorzugt aus einem Bereich von insgesamt 1,5 Gew.-% bis insgesamt 7 Gew.-%,

jeweils bestimmt mittels RFA, jeweils berechnet als elementares Metall und jeweils bezogen auf das Gesamtgewicht des rotfarbenen Effektpigments.

Bei einer Ausführungsform erfolgt die Kalzination der erfindungsgemäßen rotfarbenen Effektpigmente unter reduzierenden Bedingungen, vorzugsweise in Gegenwart von Formiergas ($N_2/H_2$). Mit einer Kalzination unter reduzierenden Bedingungen können niedrigere Helligkeitswerte L* einhergehen, als dies bei einer Kalzination unter Luft der Fall ist. In Abhängigkeit von den aufgebrachten Schichten A, B und C bzw. B und C können bei dieser Ausführungsform während der Kalzination reduzierte Metalloxide, beispielsweise Magnetit oder Ilmenit, ausgebildet werden.

Vorstehende Ausführungen werden im Folgenden beispielhaft anhand verschiedener Beschichtungen näher erläutert.

Wird beispielsweise nacheinander ein wasserlösliches Eisen(III)salz, ein wasserlösliches Zinn(IV)salz und wiederum ein wasserlösliches Eisen(III)salz zu einer Suspension eines, optional beschichteten, nichtmetallischen plättchenförmigen Substrats hinzu gegeben, entsteht beim Kalzinieren, betrachtet im REM-Querschliff ausgehend vom Substrat, auf die optional bereits vorhandene Beschichtung folgend, eine Schicht 2 umfassend ein Metalloxid, Metallhydroxid und/oder Metalloxidhydrat, wobei die Metallionen des Metalloxids, Metallhydroxids und/oder Metalloxidhydrats aus der Gruppe bestehend aus Fe Ti, Zr, und Sn sind, eine Abstandslage sowie eine Schicht 3, wobei die Metallionen des Metalloxids, Metallhydroxids und/oder Metalloxidhydrats der Schicht 3 aus der Gruppe bestehend aus Fe, Ti, Zr und, Sn sind. Wenigstens eine der Schichten 2 und 3 umfasst ein Metalloxid, Metallhydroxid und/oder Metalloxidhydrat enthaltend Eisenionen.

Weisen die erfindungsgemäßen rotfarbenen Effektpigmente zusätzlich zu den wenigstens zwei oder drei sequenziell aufgebrachten, vorzugsweise abgeschiedenen, Metalloxiden, Metallhydroxiden und/oder Metalloxidhydraten weitere Metalloxide, Metallhydroxide und/oder Metalloxidhydrate umfassende Schichten auf, so können sich auch innerhalb der weiteren Schichten weitere Abstandslagen ausbilden, sofern beispielsweise die oben, für die wenigstens zwei oder drei sequenziell aufgebrachten, vorzugsweise abgeschiedenen, Metalloxide, Metallhydroxide und/oder Metalloxidhydrate beschriebenen Verfahrensschritte eingehalten werden.

Die erfindungsgemäßen rotfarbenen Effektpigmente können optional mit wenigstens einer äußeren Schutzschicht versehen sein, die die Wetterstabilität und/oder chemische Stabilität weiter erhöht. Die Chemikalienstabilität sowie die Schwitzwasserstabilität wurden gemäß den nachfolgenden Ausführungen in den Abschnitten IIg "Bestimmung der Chemikalienbeständigkeit" "bzw. IIi "Schwitzwassertest" bestimmt.

Die optional vorhandene Schutzschicht umfasst Metalloxide, Metallhydroxide und/oder Metalloxidhydrate, deren Metallionen aus der Gruppe der Metalle, bestehend aus Si, Ce, Cr, Al, Zr, Zn und Mischungen davon, vorzugsweise aus der Gruppe der Metalle Si, Ce, Al, Zr und Mischungen davon, ausgewählt werden. Hierbei liegt der Anteil der optional vorhandenen Schutzschicht bevorzugt in einem Bereich von 0,1 Gew.-% bis 7,0 Gew.-%, besonders bevorzugt in einem Bereich von 0,2 Gew.-% bis 5,2 Gew.-% und ganz besonders bevorzugt in einem Bereich von 0,3 Gew.-% bis 3,1 Gew.-%, jeweils bezogen auf das Gesamtgewicht des erfindungsgemäßen rotfarbenen Effektpigments.

Die optional vorhandene Schutzschicht kann des Weiteren, beispielsweise durch Silane, oberflächenmodifiziert sein. Die Silane können dabei keine funktionelle Bindungsgruppe oder eine oder mehrere funktionelle Bindungsgruppe(n) aufweisen. Silane mit wenigstens einer funktionellen Bindungsgruppe werden im Folgenden auch als organofunktionelle Silane bezeichnet.

Beispielsweise können ein oder mehrere Silane auf dieser äußeren Schutzschicht aufgebracht sein. Bei den Silanen kann es sich um Alkylsilane mit verzweigten oder unverzweigten Alkylresten mit 1 bis 24 C-Atomen, vorzugsweise 6 bis 18 C-Atomen, handeln.

Bei einer weiteren bevorzugten Ausführungsform ist das Silan ohne funktionelle Bindungsgruppe ein Alkylsilan. Das Alkylsilan weist vorzugsweise die Formel $R_{(4-z)}Si(X)_z$ auf. Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

Bei einer weiteren Ausführungsform kann auch wenigstens ein organofunktionelles Silan, das eine chemische Anbindung an einen Kunststoff, ein Bindemittel eines Lackes oder einer Farbe, etc. ermöglicht zur Oberflächenmodifizierung eingesetzt werden. Diese Gruppen des organofunktionellen Silans können auch als Kupplungsgruppen oder funktionelle Bindungsgruppen bezeichnet werden und werden bevorzugt aus der Gruppe, die aus Hydroxy, Amino, Acryl, Methacryl, Vinyl, Epoxy, Isocyanat, Cyano und Mischungen davon besteht, ausgewählt.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Evonik hergestellt und unter dem Handelsnamen "Dynasylan" vertrieben. Weitere Produkte können von der Fa. Momentive (Silquest-Silane) oder von der Fa. Wacker, beispielsweise Standard-und α-Silane aus der GENIOSIL-Produktgruppe, bezogen werden.

Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m)ethoxysilan (Dynasylan MTMS bzw. MTES), 3-Mercaptopropyltrimethoxysilan (Dynasylan MTMO; Silquest A-189), 3-Glycidoxypropyltrimethoxysilan (Dynasylan GLYMO, Silquest A-187), Tris[3-(trimethoxysilyl)propyl]isocyanurat (Silquest Y-11597), Bis[3-(triethoxysilyl)propyl)]tetrasulfid (Silquest A-1289), Bis[3-(triethoxysilyl)propyldisulfid (Silquest A-1589), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), (Isocyanatomethyl)methyldimethoxysilan, (Isocyanatomethyl)trimethoxysilan, 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysilan, 3-Acryloxypropyltrimethoxysilan, 3-Acryloxypropyltripropoxysilan, 3-Methacryloxypropyltriethoxysilan, 3-Methacryloxypropyltriacetoxysilan, 3-Methacryloxypropylmethyldimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan oder Mischungen davon. Vorzugsweise werden als organofunktionelle Silane 3-Methacryloxypropyl-trimethoxysilan (Dynasylan MEMO, Silquest A-174NT), Vinyltri(m)ethoxysilan (Dynasylan VTMO bzw. VTEO, Silquest A-151 bzw. A-171), Methyltri(m)ethoxysilan (Dynasylan MTMS bzw. MTES), beta-(3,4-Epoxycyclohexyl)ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), 3-(Triethoxysilyl)propylbernsteinsäureanhydrid (GENIOSIL GF 20), Vinyltrimethoxysilan (GENIOSIL XL 10) und/oder Vinyltris(2-methoxyethoxy)silan (GENIOSIL GF 58) verwendet.

Es ist aber auch möglich, andere organofunktionelle Silane auf die erfindungsgemäßen rotfarbenen Effektpigmente aufzubringen.

Weiterhin lassen sich, beispielsweise kommerziell von Degussa erhältliche, wässrige Vorhydrolysate einsetzen. Hierzu gehören unter anderem wässriges Aminosiloxan (Dynasylan Hydrosil 1151), wässriges amino-/ alkylfunktionelles Siloxan (Dynasylan Hydrosil 2627 oder 2909), wässriges diaminofunktionelles Siloxan (Dynasylan Hydrosil 2776), wässriges epoxyfunktionelles Siloxan (Dynasylan Hydrosil 2926), amino-/ alkylfunktionelles Oligosiloxan (Dynasylan 1146), vinyl-/ alkylfunktionelles Oligosiloxan (Dynasylan 6598), oligomeres Vinylsilan (Dynasylan 6490) oder oligomeres kurzkettiges alkyl-funktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Pigments an verschiedenartige Bindemittel sehr gut geeignet.

Bevorzugt werden hierzu folgende Verbindungen genommen: 3-Aminopropyltrimethoxysilan (Dynasylan AMMO; Silquest A-1110), 3-Aminopropyltriethoxysilan (Dynasylan AMEO), [3-(2-Aminoethyl)-aminopropyl]trimethoxysilan (Dynasylan DAMO, Silquest A-1120), [3-(2-Aminoethyl)-aminopropyl]triethoxysilan, triaminofunktionelles Trimethoxysilan (Silquest A-1130), Bis-(gamma-trimethoxysilylpropyl)amin (Silquest A-1170), N-Ethyl-gamma-aminoisobutyltrimethoxysilan (Silquest A-Link 15), N-Phenyl-gamma-aminopropyltrimethoxysilan (Silquest Y-9669), 4-Amino-3,3-dimethylbutyltrimethoxysilan (Silquest A-1637), ((Cyclohexylamino)methyl)(diethoxy)methylsilan (GENIOSIL XL 924), N-Cyclohexyl-aminomethyltriethoxysilan (GENIOSIL XL 926), N-Phenylaminomethyl-trimethoxysilan (GENIOSIL XL 973) oder deren Mischungen.

Bei einer bevorzugten Ausführungsform weist die optional vorhandene Schutzschicht die in den jeweiligen Hauptansprüchen der WO 2006/021386 A1, WO 2012/130897 A1 oder WO 2014/053454 A1 offenbarte Zusammensetzung auf.

Weiterhin können die erfindungsgemäßen rotfarbenen Effektpigmente mit einer Oberflächenmodifizierung versehen sein, welche beispielsweise die Einarbeitung der Effektpigmente in unterschiedliche Medien erleichtert. Bei der Verwendung der erfindungsgemäßen rotfarbenen Effektpigmente beispielsweise in Pulverlacken, weisen die Effektpigmente vorzugsweise eine der in den Hauptansprüchen der EP 2 698 403 A1 oder der EP 2 576 702 A1 offenbarten Oberflächenmodifizierungen auf. Alternativ können die erfindungsgemäßen rotfarbenen Effektpigmente eine äußerste Beschichtung gemäß WO 2006/136435 A2, Anspruch 32, aufweisen, welche vorzugsweise durch das Sprühtrocknungsverfahren gemäß WO 2006/136435 A2, Anspruch 1, aufgebracht wird.

Bei der Verwendung der erfindungsgemäßen rotfarbenen Effektpigmente in kosmetischen Formulierungen kann beispielsweise ihre Einarbeitung in O/W-, W/O- oder W/Si-Emulsionssystemen durch eine hydrophobe Oberflächenbelegung, z.B. mit Triethoxy Caprylylsilan, erleichtert und eine längere Emulsionsstabilität erzielt werden.

Die erfindungsgemäßen rotfarbenen Effektpigmente lassen sich auch in Mischungen mit transparenten und/oder deckenden (an)organischen Weiß-, Bunt-, Schwarzpigmenten und/oder Metalleffektpigmenten und/oder Perlglanzpigmenten und/oder Füllstoffen in der jeweils gewünschten Anwendung einsetzen. In welcher Menge die erfindungsgemäßen rotfarbenen Effektpigmente zum Einsatz kommen, hängt von der jeweiligen Anwendung sowie vom zu erzielenden optischen Effekt ab.

Die erfindungsgemäßen rotfarbenen Effektpigmente können in kosmetischen Formulierungen, Kunststoffen, Folien, Textilien, keramischen Materialien, Gläsern, Farben, Druckfarben, Tinten, Lacken und Pulverlacken eingesetzt werden. Weiterhin können die erfindungsgemäßen rotfarbenen Effektpigmente auch für funktionale Applikationen, wie z.B. Lasermarkierung, Gewächshausfolien oder Agrarfolien, verwendet werden.

In kosmetischen Formulierungen, wie beispielsweise Körperpuder, Gesichtspuder, gepresstes oder loses Puder, Pudercreme, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotionen, Gele, Emulsionen, Nagellackkompositionen, können die erfindungsgemäßen rotfarbenen Effektpigmente mit für die jeweilige Anwendung geeigneten Roh-, Hilfs- und Wirkstoffen kombiniert werden. Die Gesamtkonzentration erfindungsgemäßen rotfarbenen Effektpigmente in der kosmetischen Formulierung kann zwischen 0,001 Gew.-% für Rinse-off-Produkte und 40,0 Gew.-% für Leave-on-Produkte, jeweils bezogen auf das Gesamtgewicht der Formulierung, liegen.

Bei einer weiteren Ausführungsform können die erfindungsgemäßen rotfarbenen Effektpigmente in kompakter Teilchenform vorliegen. Unter kompakter Teilchenform werden Pellets in Form von vorzugsweise Zylindern und/oder Kügelchen verstanden. Die Zylinder weisen hierbei bevorzugt einen Durchmesser aus einem Bereich von 0,2 cm bis 4,2 cm, besonders bevorzugt aus einem Bereich von 0,5 cm bis 2,3 cm und ganz besonders bevorzugt aus einem Bereich von 0,7 cm bis 1,7 cm und bevorzugt eine Länge aus einem Bereich von 0,2 cm bis 7,1 cm, besonders bevorzugt aus einem Bereich von 0,6 cm bis 5,3 cm und ganz besonders bevorzugt aus einem Bereich von 0,8 cm bis 3,7 cm auf. Die Kügelchen weisen bevorzugt einen Radius von $\leq$ 1 cm, besonders bevorzugt aus einem Bereich von 0,2 cm bis 0,7 cm und ganz besonders bevorzugt aus einem Bereich von 0,3 cm bis 0,5 cm auf.

Bei einer Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine auf dem Substrat aufgebrachte Beschichtung, wobei die Beschichtung wenigstens ein Metalloxid, Metallhydroxid und/oder Metalloxidhydrat umfasst, die Metallionen des Metalloxids, Metallhydroxids und/oder Metalloxidhydrats wenigstens zwei unterschiedliche Metallionen, ausgewählt aus der Gruppe der Metalle, bestehend aus Fe, Sn, Ti und Zr umfassen, der Anteil an Eisenionen, bestimmt mittels RFA und berechnet als elementares Eisen, bei insgesamt wenigstens 18 Gew.-%, bezogen auf das Gesamtgewicht des rotfarbenen Effektpigments, wobei der Bunttonwinkel $h^*_{15}$ im CIE-LCh-Farbraum in einem Bereich von 330° bis 360° und 0° bis 50° und wobei das Aspektverhältnis des rotfarbenen Effektpigments, definiert als $D_{50}$/Gesamtdicke, in einem Bereich von 1 bis 5000, bevorzugt in einem Bereich von 5 bis 1000, besonders bevorzugt in einem Bereich von 10 bis 790, liegt.

Bei einer weiteren Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine auf dem Substrat aufgebrachte Beschichtung, wobei die Beschichtung wenigstens ein Metalloxid, Metallhydroxid und/oder Metalloxidhydrat umfasst, die Metallionen des Metalloxids, Metallhydroxids und/oder Metalloxidhydrats wenigstens zwei unterschiedliche Metallionen, ausgewählt aus der Gruppe der Metalle, bestehend aus Fe, Sn, Ti und Zr umfassen, der Anteil an Eisenionen, bestimmt mittels RFA und berechnet als elementares Eisen, bei insgesamt wenigstens 18 Gew.-%, bezogen auf das Gesamtgewicht des rotfarbenen Effektpigments, wobei der Bunttonwinkel $h^*_{15}$ im CIE-LCh-Farbraum in einem Bereich von 340° bis 360° und 0° bis 50° und wobei die Gesamtdicke des rotfarbenen Effektpigments, definiert als Summe aus mittlerer Dicke des Substrats und mittlerer Dicke der gesamten Beschichtung, in einem Bereich von 100 nm bis 5000 nm, bevorzugt in einem Bereich von 200 nm bis 2500 nm, besonders bevorzugt in einem Bereich von 250 nm bis 1800 nm, liegt.

Bei einer weiteren Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung

    a) optional eine Schicht 1, mit einer mittleren Schichtdicke von weniger als 10 nm, die Zinnoxid, Zinnhydroxid und/oder Zinnoxidhydrat umfasst oder daraus besteht,
    b) eine Schicht 2 umfassend wenigstens ein Metalloxid, Metallhydroxid und/oder Metalloxidhydrat, wobei das Metallion wenigstens ein Metallion, ausgewählt aus der Gruppe der Metalle, bestehend aus Fe, Sn, Ti und Zr umfasst oder ist,
    c) eine Schicht 3 umfassend wenigstens ein Metalloxid, Metallhydroxid und/oder Metalloxidhydrat, wobei das Metallion wenigstens ein Metallion, ausgewählt aus der Gruppe der Metalle, bestehend aus Fe, Sn, Ti und Zr umfasst oder ist

umfasst, wobei wenigstens eine der Schichten 2 oder 3 wenigstens zwei unterschiedliche Metallionen aus den vorstehend aufgeführten Gruppen und wenigstens eine der Schichten 2 oder 3 Eisenionen umfasst, wobei der Anteil an Eisenionen, jeweils bestimmt über RFA und jeweils berechnet als elementares Metall, in einem Bereich von 22 Gew.-% bis 79 Gew.-%, bezogen auf das Gesamtgewicht des Effektpigments liegt, die Schichten 2 und 3 durch eine Abstandslage der mittleren Höhe $h_a$ aus einem Bereich von 5 nm bis 76 nm unterbrochen sind, die Standardabweichung der relativen Höhe $h_{Rma}$ in einem Bereich von 0,2% bis 11%, und die Steganzahldichte in einem Bereich von 0,5 % bis 79 % liegt.

Mit "unterbrochen" ist erfindungsgemäß gemeint, dass die Schichten 2 und 3 durch eine Abstandslage voneinander beabstandet sind bzw. auf Abstand gehalten werden.

Mit dem allgemeinen Ausdruck "Metalloxid, Metallhydroxid und/oder Metalloxidhydrat" ist erfindungsgemäß gemeint "Metalloxid und/oder Metallhydroxid und/oder Metalloxidhydrat". Dies gilt auch dann, wenn das Metall bzw. Metallion spezifiziert ist, beispielsweise als Titan(ion), Eisen(ion), Zinn(ion), Zirkonium(ion), etc.

Gemäß einer bevorzugten Ausführungsform liegt die optionale Schicht 1 direkt auf dem nichtmetallischen plättchenförmigen Substrat, die Schicht 2 folgt direkt auf die Schicht 1 und die Schicht 3 folgt auf die Schicht 2, wobei die Schichten 2 und 3 durch eine Abstandslage unterbrochen sind.

Gemäß einer weiteren Ausführungsform liegt die Schicht 2 direkt auf dem mit wenigstens einem Metalloxid, Metallhydroxid und/oder Metalloxidhydrat beschichteten nichtmetallischen plättchenförmigen Substrat und die Schicht 3 folgt auf die Schicht 2, wobei die Schichten 2 und 3 durch eine Abstandslage unterbrochen sind. Die außenliegenden Metalloxid-, Metallhydroxid- und/oder Metalloxidhydratschicht des nichtmetallischen plättchenförmigen Substrats umfasst hierbei wenigstens ein Metallion, ausgewählt aus der Gruppe der Metalle, bestehend aus Fe, Ti und Zr.

Bei einer weiteren Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung

a) optional eine Schicht 1, mit einer mittleren Schichtdicke von weniger als 10 nm, die Zinnoxid, Zinnhydroxid und/oder Zinnoxidhydrat umfasst oder daraus besteht,
b) eine Schicht 2 umfassend wenigstens ein Metalloxid, Metallhydroxid und/oder Metalloxidhydrat wobei das Metallion wenigstens ein nichtfärbendes Metallion, ausgewählt aus der Gruppe der Metalle, bestehend aus Fe und Sn, umfasst oder ist,
c) eine Schicht 3 umfassend wenigstens ein Metalloxid, Metallhydroxid und/oder Metalloxidhydrat, wobei das Metallion wenigstens ein nichtfärbendes Metallion, ausgewählt aus der Gruppe der Metalle, bestehend aus Fe und Sn, umfasst oder ist

umfasst und wenigstens eine der Schichten 2 oder 3 wenigstens zwei unterschiedliche Metallionen aus den vorstehend aufgeführten Gruppen und wenigstens eine der Schichten 2 oder 3 Eisenionen umfasst, die Schichten 2 und 3 durch eine Abstandslage unterbrochen sind, und wobei die Effektpigmente einen Span $\Delta D$ aus einem Bereich von 0,8 bis 2,5 aufweisen.

Bei einer bevorzugten Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung

a) optional eine Schicht 1, mit einer mittleren Schichtdicke von weniger als 10 nm, die Zinnoxid, Zinnhydroxid und/oder Zinnoxidhydrat umfasst oder daraus besteht,
b) eine Schicht 2 umfassend wenigstens ein Metalloxid, Metallhydroxid und/oder Metalloxidhydrat aus oder mit wenigstens einem Metallion, ausgewählt aus der Gruppe der Metalle, bestehend aus Ti, Fe, Sn und Zr,
c) eine Schicht 3 umfassend wenigstens ein Metalloxid, Metallhydroxid und/oder Metalloxidhydrat aus oder mit wenigstens einem Metallion, ausgewählt aus der Gruppe der Metalle, bestehend aus Ti, Fe, Sn und Zr

umfasst und wenigstens eine der Schichten 2 oder 3 wenigstens zwei unterschiedliche Metallionen aus den vorstehend aufgeführten Gruppen und wenigstens eine der Schichten 2 oder 3 Eisenionen umfasst, wobei der Anteil an Eisenionen, jeweils bestimmt über RFA und jeweils berechnet als elementares Metall, in einem Bereich von insgesamt 21 Gew.-% bis 78 Gew.-%, bevorzugt in einem Bereich von 25 Gew.-% bis 72 Gew.-%, jeweils bezogen auf das Gesamtgewicht des Effektpigments liegt, die Schichten 2 und 3 durch eine Abstandslage unterbrochen sind und das Effektpigment eine Chemikalienbeständigkeit mit einem dE von <5 bevorzugt <3 aufweist.

Bei einer besonders bevorzugten Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung

a) optional eine Schicht 1, mit einer mittleren Schichtdicke von weniger als 10 nm, die Zinnoxid, Zinnhydroxid und/oder Zinnoxidhydrat umfasst oder daraus besteht,
b) eine Schicht 2 umfassend wenigstens ein Metalloxid, Metallhydroxid und/oder Metalloxidhydrat, wobei die Metallionen wenigstens zwei Metallionen, ausgewählt aus der Gruppe der Metalle, bestehend aus Fe und Sn, umfassen oder sind,
c) eine Schicht 3 umfassend wenigstens ein Metalloxid, Metallhydroxid und/oder Metalloxidhydrat , wobei die Metallionen wenigstens zwei Metallionen, ausgewählt aus der Gruppe der Metalle, bestehend aus Fe und Sn, umfassen oder sind

umfasst und die Schichten 2 und 3 durch eine Abstandslage unterbrochen sind, wobei die Beschichtung weitere hoch- und/oder niedrigbrechende Schichten umfasst und das Effektpigment wenigstens eine weitere, im Wesentlichen parallel zur Oberfläche des nichtmetallischen plättchenförmigen Substrats verlaufende Abstandslage der mittleren Höhe $h_a$ aus einem Bereich von 4 nm bis 100 nm, bevorzugt aus einem Bereich von 9 nm bis 74 nm, umfasst.

Bei einer weiteren Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung

a) optional eine Schicht 1, mit einer mittleren Schichtdicke von weniger als 10 nm, die Zinnoxid, Zinnhydroxid und/oder Zinnoxidhydrat umfasst oder daraus besteht,
b) eine Schicht 2 umfassend wenigstens ein Metalloxid, Metallhydroxid und/oder Metalloxidhydrat, wobei das Metallion wenigstens ein Metallion, ausgewählt aus der Gruppe der Metalle, bestehend aus Zr, Sn und Fe, umfasst oder ist,
c) eine Schicht 3 umfassend wenigstens ein Metalloxid, Metallhydroxid und/oder Metalloxidhydrat, wobei das Metallion wenigstens ein Metallion, ausgewählt aus der Gruppe der Metalle, bestehend aus Zr, Sn und Fe, umfasst oder ist

umfasst, wobei wenigstens eine der Schichten 2 oder 3 wenigstens zwei unterschiedliche Metallionen aus den vorstehend aufgeführten Gruppen und wenigstens eine der Schichten 2 oder 3 Eisenionen umfasst, der Quotient aus der mittleren Schichtdicke der Schicht 2 und der mittleren Schichtdicke der Schicht 3 bevorzugt in einem Bereich von 0,5 bis 3,0 und die Standardabweichung der relativen Höhe $h_{Rma}$ in einem Bereich von 0,2% bis 11% liegt.

Bei einer weiteren Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung wenigstens eine, im Wesentlichen parallel zur Oberfläche des nichtmetallischen plättchenförmigen Substrats liegende Abstandslage aufweist und das Effektpigment erhältlich ist durch i) optionales Aufbringen einer nicht kalzinierten Zinnoxid-, Zinnhydroxid- und/oder Zinnoxidhydratschicht auf dem nichtmetallischen plättchenförmigen Substrat, ii) Aufbringen von drei nicht kalzinierten Metalloxiden, Metallhydroxiden und/oder Metalloxidhydraten, wobei das zweite dieser nicht kalzinierten Metalloxide, Metallhydroxide und/oder Metalloxidhydrate stofflich verschieden von den anderen ist und derart beschaffen ist, dass es in wenigstens eines der anderen nicht kalzinierten Metalloxide, Metallhydroxide und/oder Metalloxidhydrate diffundieren kann, wobei das erste und dritte der drei nicht kalzinierten Metalloxide, Metallhydroxide und/oder Metalloxidhydrate jeweils wenigstens ein Metallion, ausgewählt aus der Gruppe der Metalle, bestehend aus Fe, Ti und Zr, das erste und/oder dritte der drei nicht kalzinierten Metalloxide, Metallhydroxide und/oder Metalloxidhydrate Eisenionen und das zweite der drei nicht kalzinierten Metalloxide, Metallhydroxide und/oder Metalloxidhydrate wenigstens ein Metallion, ausgewählt aus der Gruppe der Metalle, bestehend aus Sn, Ti und Zr, umfassen oder sind sowie iii) Kalzinieren des in Schritt ii) erhaltenen Produktes, optional unter reduzierenden Bedingungen, bei einer Temperatur aus einem Bereich von 400°C bis 980°C.

Bei einer ganz besonders bevorzugten Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine darauf aufgebrachte Beschichtung, wobei die Beschichtung wenigstens eine, im Wesentlichen parallel zur Oberfläche des nichtmetallischen plättchenförmigen Substrats liegende Abstandslage der mittleren Höhe $h_a$ aus einem Bereich von 14 nm bis 51 nm aufweist und das Effektpigment erhältlich ist durch i) optionales Aufbringen einer nicht kalzinierten Zinnoxid-, Zinnhydroxid- und/oder Zinnoxidhydratschicht unter Verwendung eines wasserlöslichen Zinn(IV)salzes auf dem nichtmetallischen plättchenförmigen Substrat, ii) sequentielles Aufbringen einer ersten Schicht A unter Verwendung eines wasserlöslichen Eisen(III)salzes, einer zweiten Schicht B unter Verwendung eines wasserlöslichen Zinn(IV)- und/oder Titan(IV)salzes, einer dritten Schicht C unter Verwendung eines wasserlöslichen Eisen(III)salzes und iii) Kalzinieren des in Schritt ii) erhaltenen Produktes bei einer Temperatur aus einem Bereich von 400°C bis 910°C.

Bei einer bevorzugten Ausführungsform umfasst die Beschichtung der erfindungsgemäßen rotfarbenen Effektpigmente, jeweils vor Trocknung und/oder Kalzination, wenigstens eine hochbrechende Schicht aus oder mit Titanoxid, Titanhydroxid und/oder Titanoxidhydrat und wenigstens zwei nicht benachbarte, hochbrechende Schichten aus oder mit Eisenoxid, Eisenhydroxid und/oder Eisenoxidhydrat, wobei das Gewichtsverhältnis im Effektpigment von Titan zu Eisen bei <1 bevorzugt in einem Bereich von 0,01 bis 0,8 und besonders bevorzugt in einem Bereich von 0,1 bis 0,5 und ganz besonders bevorzugt in einem Bereich von 0,05 bis 0,19 liegt.

Bei einer weiter bevorzugten Ausführungsform umfasst die Beschichtung der erfindungsgemäßen rotfarbenen Effektpigmente, jeweils vor Trocknung und/oder Kalzination, wenigstens eine hochbrechende Schicht aus oder mit Zinnoxid, Zinnhydroxid und/oder Zinnoxidhydrat und wenigstens zwei hochbrechende Schichten aus oder mit Eisenoxid, Eisenhydroxid und/oder Eisenoxidhydrat, wobei das Gewichtsverhältnis im Effektpigment von Zinn zu Eisen bei <1, bevorzugt in einem Bereich von 0,01 bis 0,9 und besonders bevorzugt in einem Bereich von 0,1 bis 0,8 liegt. Bei dieser Ausführungsform ist es insbesondere bevorzugt, dass zunächst wenigstens eine hochbrechende Schicht von Eisenoxid, Eisenhydroxid und/oder Eisenoxidhydrat und anschließend wenigstens eine hochbrechende Schicht von Zinnoxid, Zinnhydroxid und/oder Zinnoxidhydrat sowie eine weitere hochbrechende Schicht von Eisenoxid, Eisenhydroxid und/oder Eisenoxidhydrat direkt auf dem plättchenförmigen Substrat oder direkt auf der jeweils obersten, substratnahen Schicht aufgebracht ist. Des Weiteren ist es auch möglich, vor Aufbringung der wenigstens einen hochbrechenden Schicht aus Eisenoxid, Eisenhydroxid, Eisenoxidhydrat eine Schicht oder Vorbelegung mit Metalloxiden, Metallhydroxiden, Metalloxidhydraten, wobei das Metallion ein Metallion ausgewählt aus der Gruppe der Metalle, bestehend aus Sn und Si, umfasst oder ist, direkt auf dem nichtmetallischen plättchenförmigen Substrat oder direkt auf der jeweils obersten, substratnahen Schicht abzuscheiden, wobei die Schichtdicke wenige Nanometer, bevorzugt weniger als 10 nm, besonders bevorzugt weniger als 5 nm und ganz besonders bevorzugt weniger als 3 nm, betragen kann und diese das Substrat nicht vollständig umschließend muss. Das Zinnoxid, Zinnhydroxid und/oder Zinnoxidhydrat kann mit dem Eisenoxid, Eisenhydroxid und/oder Eisenoxidhydrat zumindest teilweise in einer Mischschicht vorliegen.

Bei einer weiteren Ausführungsform betrifft die vorliegende Erfindung ein rotfarbenes Effektpigment umfassend ein nichtmetallisches plättchenförmiges Substrat, bevorzugt ein synthetisches Glimmerplättchen oder ein Glasplättchen, und eine auf dem Substrat aufgebrachte Beschichtung, wobei die Beschichtung wenigstens eine, im Wesentlichen parallel zur Oberfläche des nichtmetallischen plättchenförmigen Substrats liegende Abstandslage aufweist und das Effektpigment unter reduzierenden Bedingungen kalziniert wurde oder in der gesamten Beschichtung, vorzugsweise als äußerste Schicht direkt unter einer optional vorhandenen Schutzschicht liegend, eine semitransparente Metallschicht aufweist.

Bei einer Ausführungsform umfasst die Beschichtung der erfindungsgemäßen rotfarbenen Effektpigmente anstelle des wenigstens einen Metalloxids, Metallhydroxids und/oder Metalloxidhydrats die entsprechenden Metallsuboxide, Metallfluoride, Metallnitride, Metalloxynitride, Metalloxyhalide und/oder Metallsulfide.

Bei einer Ausführungsform umfasst die Beschichtung der erfindungsgemäßen rotfarbenen Effektpigmente zusätzlich zu dem wenigstens einen Metalloxid, Metallhydroxid und/oder Metalloxidhydrat wenigstens ein Metallsuboxid, Metallfluorid, Metallnitrid, Metalloxynitrid, Metalloxyhalid und/oder Metallsulfid.

Im Folgenden wird die Erfindung durch einige Beispiele näher erläutert, die Beispiele beschränken die Erfindung jedoch nicht. Alle %-Angaben der Beispiele und Vergleichsbeispiele sind als Gew.-% zu verstehen.

**I Herstellung der erfindungsgemäßen rotfarbenen Effektpigmente**

Beispiel 1

200 g synthetische Glimmerplättchen (Fluorphlogopitplättchen) mit einer Partikelgrößenverteilung nach MALVERN Mastersizer MS 2000: $D_{10}$=11 $\mu$m, $D_{50}$=21 $\mu$m, $D_{90}$=36 $\mu$m wurden in 1300 ml VE-Wasser suspendiert und unter turbulentem Rühren auf 85°C erhitzt. Der pH-Wert der Suspension wurde auf pH 2,6 eingestellt. Durch Zugabe von 570 g einer wässrigen Eisenchloridlösung mit einer Dichte von 1,42 g/cm$^3$ wurde eine Schicht Eisenoxid auf der Oberfläche der synthetischen Glimmerplättchen abgeschieden. Nachdem 60 min nachgerührt wurde, wurde der pH-Wert der Suspension auf pH 2,2 gesenkt und sodann eine Lösung von 500 ml SnCl$_4$ mit einer Konzentration von 30 g Sn/ L in die Suspension hinzudosiert.

Danach wurde erneut 120 Minuten nachgerührt und anschließend 750 g einer wässrigen Eisenchloridlösung mit einer Dichte von 1,42 g/cm$^3$ zugegeben. 60 Minuten nach beendeter Zugabe wurde die Suspension abfiltriert und der Filterkuchen gewaschen. Der Filterkuchen wurde gegebenenfalls getrocknet und bei 800°C 45 Minuten kalziniert. Es wurden extrem chromatische, hochglänzende rot interferierende, Effektpigmente mit roter Absorptionsfarbe und sehr guter Deckkraft erhalten.

Beispiel 2

200 g synthetische Glimmerplättchen (Fluorphlogopitplättchen) mit einer Partikelgrößenverteilung nach MALVERN Mastersizer MS 2000: $D_{10}$=10 $\mu$m, $D_{50}$=22 $\mu$m, $D_{90}$=40 $\mu$m wurden in 1300 ml VE-Wasser suspendiert und unter turbulentem Rühren auf 85°C erhitzt. Der pH-Wert der Suspension wurde auf pH 2,6 eingestellt. Durch Zugabe von 570 g einer wässrigen Eisenchloridlösung mit einer Dichte von 1,42 g/cm$^3$ wurde eine Schicht Eisenoxid auf der Oberfläche der synthetischen Glimmerplättchen abgeschieden.

Der pH-Wert der Suspension wurde im Anschluss auf pH 1,9 gesenkt und sodann eine Lösung von 250 ml TiCl$_4$ (200 g TiO$_2$/l VE-Wasser) in die Suspension hinzudosiert.

Danach wurde erneut 120 Minuten nachgerührt und anschließend 600 g einer wässrigen Eisenchloridlösung mit einer Dichte von 1,42 g/cm$^3$ zugegeben. 60 Minuten nach beendeter Zugabe wurde die Suspension abfiltriert und der Filterkuchen gewaschen. Der Filterkuchen wurde gegebenenfalls getrocknet und bei 400°C 60 Minuten kalziniert. Es wurden extrem chromatische, hochglänzende rot interferierende, Effektpigmente mit roter Absorptionsfarbe und sehr guter Deckkraft erhalten.

Beispiel 3

200 g synthetische Glimmerplättchen (Fluorphlogopitplättchen) mit einer Partikelgrößenverteilung nach MALVERN Mastersizer MS 2000: $D_{10}$=7 µm, $D_{50}$=20 µm, $D_{90}$=35 µm wurden in 1300 ml VE-Wasser suspendiert und unter turbulentem Rühren auf 85°C erhitzt. Der pH-Wert der Suspension wurde auf pH 2,6 eingestellt und 530 g einer wässrigen Eisenchloridlösung mit einer Dichte von 1,42 g/cm$^3$ zugegeben. Nachdem 60 min nachgerührt wurde, wurde der pH-Wert der Suspension auf pH 2,2 gesenkt und sodann eine Lösung von 17,5 ml SnCl$_4$ mit einer Konzentration von 315 g Sn/ L in die Suspension hinzudosiert.
Danach wurde erneut 120 Minuten nachgerührt und anschließend 1000 g einer wässrigen Eisenchloridlösung mit einer Dichte von 1,38 g/cm$^3$ zugegeben. 60 Minuten nach beendeter Zugabe wurde die Suspension abfiltriert und der Filterkuchen gewaschen. Der Filterkuchen wurde gegebenenfalls getrocknet und bei 780°C 45 Minuten kalziniert. Es wurden extrem chromatische, hochglänzende rot interferierende, Effektpigmente mit roter Absorptionsfarbe und sehr guter Deckkraft erhalten.

Beispiel 4

200 g synthetische Glimmerplättchen (Fluorphlogopitplättchen) mit einer Partikelgrößenverteilung nach MALVERN Mastersizer MS 2000: $D_{10}$=9 µm, $D_{50}$=19 µm, $D_{90}$=37 µm wurden in 1300 ml VE-Wasser suspendiert und unter turbulentem Rühren auf 85°C erhitzt. Der pH-Wert der Suspension wurde auf pH 2,2 eingestellt und sodann eine Lösung von 50 ml SnCl$_4$ mit einer Konzentration von 30 g Sn/ L bei konstantem pH-Wert zudosiert. Anschließend wurde 60 Minuten nachgerührt und der pH-Wert auf 1,9 adjustiert und 800 ml TiCl$_4$-Lösung (200 g TiO2/l VE-Wasser) zugegeben. Nach 60 minütigem Nachrühren wurde der pH-Wert der Suspension auf pH 2,2 gesenkt und sodann eine Lösung von 500 ml SnCl$_4$ mit einer Konzentration von 30 g Sn/L in die Suspension hinzudosiert.
Danach wurde erneut 120 Minuten nachgerührt und anschließend 1200 g einer wässrigen Eisenchloridlösung mit einer Dichte von 1,38 g/cm$^3$ zugegeben. 60 Minuten nach beendeter Zugabe wurde die Suspension abfiltriert und der Filterkuchen gewaschen. Der Filterkuchen wurde gegebenenfalls getrocknet und bei 830°C 45 Minuten kalziniert. Es wurden extrem chromatische, hochglänzende rot interferierende, Effektpigmente mit roter Absorptionsfarbe und sehr guter Deckkraft erhalten.

Beispiel 5

150 g synthetische Glimmerplättchen (Fluorphlogopitplättchen) mit einer Partikelgrößenverteilung nach MALVERN Mastersizer MS 2000: $D_{10}$=8 µm, $D_{50}$=20 µm, $D_{90}$=36 µm wurden in 1200 ml VE-Wasser suspendiert und unter turbulentem Rühren auf 85°C erhitzt. Der pH-Wert der Suspension wurde auf pH 2,6 eingestellt und 350 g einer wässrigen Eisenchloridlösung mit einer Dichte von 1,42 g/cm$^3$ zugegeben. Nachdem 60 Minuten nachgerührt wurde, wurde der pH-Wert der Suspension auf pH 3,5 eingestellt und sodann eine Lösung von 110 ml Zirkonchlorid w(ZrCl$_4$) = 20,0 Gew. % in die Suspension hinzudosiert.
Danach wurde erneut 120 Minuten nachgerührt, der pH-Wert auf 2,6 adjustiert und anschließend 700 g einer wässrigen Eisenchloridlösung mit einer Dichte von 1,38 g/cm$^3$ zugegeben. 60 Minuten nach beendeter Zugabe wurde die Suspension abfiltriert und der Filterkuchen gewaschen. Der Filterkuchen wurde gegebenenfalls getrocknet und bei 800°C 60 Minuten kalziniert. Es wurden extrem chromatische, hochglänzende rot interferierende, Effektpigmente mit roter Absorptionsfarbe und sehr guter Deckkraft erhalten.

Beispiel 6

200 g synthetische Borosilikatglasplättchen mit einer Partikelgrößenverteilung nach MALVERN Mastersizer MS 2000: $D_{10}$=34 µm, $D_{50}$=57 µm, $D_{90}$=95 µm wurden in 1300 ml VE-Wasser suspendiert und unter turbulentem Rühren auf 85°C erhitzt. Nacheinander erfolgt nun die Zugabe von 40 ml SnCl$_4$ (Konzentration 53 g/l) bei pH 2,2, 122 ml FeCl$_3$ (Dichte 1,42 g/cm$^3$) bei pH 2,6, 19 ml TiCl$_4$ (Konz. 200 g TiO$_2$/l) bei pH 1,9 und schließlich 292 ml FeCl$_3$ (Dichte 1,42 g/cm$^3$) bei pH 2,6. Der pH-Wert wurde dabei jeweils mittels synchroner Zugabe von wässriger NaOH (35%) konstant gehalten. 60 Minuten nach beendeter Zugabe wurde die Suspension abfiltriert und der Filterkuchen gewaschen. Der Filterkuchen wurde gegebenenfalls getrocknet und bei 650°C 60 Minuten kalziniert. Es wurden extrem chromatische, hochglänzende rot glitzernde, Effektpigmente mit roter Absorptionsfarbe und roter Reflexionsfarbe erhalten.

Beispiel 7

Das Endprodukt aus Beispiel 4 wurde unter reduzierenden Bedingungen bei 450°C 15 Minuten kalziniert. Es wurden hochchromatische, glänzende, rot interferierende Effektpigmente mit dunkler Absorptionsfarbe erhalten.

Beispiel 8

100 g des aus Beispiel 1 erhaltenen Effektpigments wurden in 850 ml VE-Wasser suspendiert und unter turbulentem Rühren auf 85°C erhitzt. Der pH-Wert wurde mit verdünnter Salzsäure auf pH 4,2 gesenkt. Dann wurde eine Lösung aus 0,93 g $Ce(NO_3)_3$ x 6 $H_2O$ gelöst in 40 ml VE-Wasser zudosiert. Gleichzeitig wurde durch Zutropfen einer 10%igen NaOH-Lösung der pH-Wert konstant gehalten. Nachdem die Lösung vollständig zugegeben wurde, wurde eine Stunde nachgerührt und im Anschluss daran der pH-Wert mit verdünnter Natronlauge auf pH 10 eingestellt. Danach wurden 5,7 g Dynasylan 1146 verdünnt mit 24,3 g VE-Wasser in die Suspension zugegeben, 180 Minuten nachgerührt, die Suspension abfiltriert und der Filterkuchen mit VE-Wasser nachgewaschen. Der Filterkuchen wurde bei 95°C unter Vakuum getrocknet.

Vergleichsbeispiel 1

Rotes Effektpigment basierend auf natürlichen Glimmerplättchen, beschichtet mit Eisenoxid, Iriodin 504 Rot, Fa. Merck.

Vergleichsbeispiel 2

Rotes Effektpigment basierend auf $SiO_2$-Plättchen, beschichtet mit Eisenoxid, Iriodin 4504 Lava Red, Fa. Merck.

Vergleichsbeispiel 3

200 g synthetische Glimmerplättchen (Fluorphlogopitplättchen) mit einer Partikelgrößenverteilung nach MAL-VERN Mastersizer MS 2000: $D_{10}$=10 $\mu m$, $D_{50}$=22 $\mu m$, $D_{90}$=40 $\mu m$ wurden in 1300 ml VE-Wasser suspendiert und unter Rühren auf 85°C erhitzt. Der pH-Wert der Suspension wurde auf pH 2,6 eingestellt und sodann eine Lösung von 60 ml $FeCl_3$ (280 g $Fe_2O_3$/l VE-Wasser) in die Suspension hinzudosiert. Anschließend wurde die Suspension noch weitere 60 min gerührt ehe der pH-Wert mit Lauge auf 7,5 angehoben und weitere 20 min nachgerührt wurde. Eine Wasserglaslösung (185 g Wasserglaslösung, 24% $SiO_2$, gemischt mit 207 g VE-Wasser) wurde sodann langsam in die Suspension eingeleitet. Danach wurde noch 60 min nachgerührt und der pH-Wert wieder auf 2,6 eingestellt. Sodann wurde eine Lösung von 260 ml $FeCl_3$ (280 g $Fe_2O_3$/l VE-Wasser) in die Suspension hinzudosiert. Zuletzt wurde 60 Minuten nachgerührt, die Suspension dann abfiltriert und der Filterkuchen gewaschen. Der Filterkuchen wurde bei 780°C 45 Minuten kalziniert. Es wurde ein rot interferierendes, rot absorbierendes Mehrschichtperlglanzpigment mit vergleichsweise geringem Chroma erhalten.

Vergleichsbeispiel 4

Rotes Effektpigment basierend auf Glasplättchen, beschichtet mit Eisenoxid, MIRAGE Sparkling Red, Fa. Eckart.

Vergleichsbeispiel 5

In Anlehnung an Beispiel 1 der US 8,58,5818 B1 wurde eine Suspension aus synthetischem Mica der Fraktion 20-80$\mu m$ mit einem $D_{50}$ von 35 $\mu m$ in 1785 mL VE-Wasser auf 83°C geheizt und der pH-Wert mit HCl auf 1,4 eingestellt. 50 g einer 20%igen $SnCl_4$-Lösung wurden mit einer Dosiergeschwindigkeit von 3,2 g/min zudosiert und mit NaOH der pH-Wert konstant gehalten. Anschließend wurde 60 Minuten nachgerührt und dann 36 g einer 40%igen $TiCl_4$-Lösung mit 3g/min zugegeben. Auch hier wurde der pH-Wert mit NaOH konstant gehalten. Nachfolgend wurde der pH-Wert auf 3,0 eingestellt bevor eine Eisenchloridlösung w($FeCl_3$)=39% mit einer Geschwindigkeit von 1,2 g/min bei konstantem pH-Wert zugegeben wurde. Nachdem 320 mL $FeCl_3$ zugegeben waren, wurde die Suspension filtriert, gewaschen und bei 850°C kalziniert. Es wurde ein bronzefarbenes Pigment mit mäßigen Effekteigenschaften erhalten. Im REM-Querschliff ist keine Abstandslage erkennbar.

**II Charakterisierung der rotfarbenen Effektpigmente und Pigmente der Vergleichsbeispiele**

IIa Teilchengrößenmessung

Die Größenverteilungskurve der erfindungsgemäßen rotfarbenen Effektpigmente sowie der Pigmente der Vergleichsbeispiele wurde mit dem Gerät Mastersizer 2000, Fa. Malvern, gemäß Herstellerangaben bestimmt. Hierzu wurden ca. 0,1 g des jeweiligen Pigments als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die Mittelwerte gebildet. Die Auswertung der Streulichtsignale erfolgte dabei nach der Fraunhofer Methode.

Unter der mittleren Teilchengröße $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50% der Pigmente einen Durchmesser aufweisen, der gleich oder kleiner als der angegebene Wert, beispielsweise 20 $\mu$m, ist. Entsprechend gibt der $D_{10}$- bzw. $D_{90}$-Wert an, dass 10% bzw. 90 % der Pigmente einen Durchmesser aufweisen, der gleich oder kleiner als der jeweilige Messwert ist.

$$\Delta D = \frac{D_{90} - D_{10}}{D_{50}}$$

Der Span $\Delta$D, definiert als , gibt die Breite der Teilchengrößenverteilung an.

Tabelle 3: Teilchengrößen

| Beispiel/Vergleichsbeispiel | $D_{10}$ [$\mu$m] | $D_{50}$ [$\mu$m] | $D_{90}$ [$\mu$m] | Span |
|---|---|---|---|---|
| Beispiel 1 | 10,0 | 21,9 | 40,2 | 1,382 |
| Beispiel 2 | 8,8 | 20,1 | 37,6 | 1,429 |
| Beispiel 3 | 8,3 | 20,7 | 40,8 | 1,567 |
| Beispiel 4 | 9,2 | 20,7 | 39,7 | 1,475 |
| Beispiel 5 | 10,1 | 21,8 | 40,3 | 1,384 |
| Beispiel 6 | 27,2 | 54,0 | 97,4 | 1,299 |
| Beispiel 7 | 8,4 | 20,4 | 40,7 | 1,583 |
| Vergleichsbeispiel 1 | 10,8 | 21,9 | 41,5 | 1,402 |
| Vergleichsbeispiel 2 | 9,7 | 19,3 | 35,5 | 1,337 |
| Vergleichsbeispiel 3 | 17,2 | 28,9 | 47,0 | 1,031 |
| Vergleichsbeispiel 4 | 29,4 | 56,4 | 98,0 | 1,219 |
| Vergleichsbeispiel 5 | 17,4 | 36,2 | 63,9 | 1,285 |

IIb Winkelabhängige Farbmessungen

Zur Messung der Farb- und Helligkeitswerte wurden die erfindungsgemäßen Effektpigmente und die Pigmente der Vergleichsbeispiele in einer Pigmentierungshöhe von 6 Gew.-%, bezogen auf das Gesamtgewicht des Nasslacks, in einen konventionellen Nitrocelluloselack (Erco-Bronzemischlack 2615e farblos; Fa. Maeder Plastiklack AG)) eingerührt. Dabei wurden die jeweiligen Pigmente vorgelegt und anschließend mit einem Pinsel in den Lack dispergiert. Der fertige Lack wurde auf einem Rakelabzugsgerät (RK Print Coat Instr. LTd. Citenco Abziehgerät Modell K 101) mit einer Spiralrakel in einer Nassfilmdicke von 40 $\mu$m, 76 $\mu$m (Beispiel 6) oder 100 $\mu$m auf Schwarz-Weiß-Deckungskarten (Byko-Chart 2853, Fa. Byk-Gardner) appliziert und nachfolgend bei Raumtemperatur getrocknet.

Die Auswahl der Spiralrakel erfolgt gemäß Tabelle 2 in Abhängigkeit vom Dso-Wert der jeweils zu applizierenden Pigmente bzw. Substrate.

Mit dem Mehrwinkelfarbmessgerät BYK-mac (Fa. Byk-Gardner) wurden bei einem konstanten Einfallswinkel von 45° (gemäß Herstellerangaben) bei verschiedenen Beobachtungswinkeln relativ zum Glanzwinkel die Farbwerte auf schwarzem Untergrund der Deckungskarte bestimmt. Zur Charakterisierung der Farbintensität wurde der Chroma-wert $C^{*}_{15}$ herangezogen, welcher bei einem Messwinkel von 15° entfernt vom Glanzwinkel auf dem schwarzen Untergrund der Schwarz-Weiß-Deckungskarte gemessen wurde.

Stark reflektierende Proben (Idealfall Spiegel) reflektieren nahezu das gesamte eintreffende Licht im sogenannten Glanzwinkel. Je näher die Lackapplikation am Glanzwinkel gemessen wird, desto stärker erscheint die Interferenzfarbe.

Tabelle 4: Farb- und Helligkeitswerte der erfindungsgemäßen rotfarbenen Effektpigmente

| Beispiel/ | NC-Lack 6%ig 40μm BykMac | | | | |
| Vergleichsbeispiel | L*110°s[1]) | a*15°s | b*15°s | C*15°s | h*15°s |
|---|---|---|---|---|---|
| Beispiel 1 | 70,7 | 52,8 | 32,0 | 61,7 | 31,2 |
| Beispiel 2 | 64,2 | 40,2 | 27,6 | 48,8 | 34,5 |
| Beispiel 3 | 75,7 | 50,1 | 23,3 | 55,3 | 24,9 |
| Beispiel 4 | 59,6 | 31,9 | 22,7 | 39,1 | 35,4 |
| Beispiel 5 | 63,7 | 46,2 | 29,8 | 54,9 | 32,8 |
| Beispiel 6 | 67,4 | 40,1 | 36,7 | 54,3 | 42,5 |
| Beispiel 7 | 60,2 | 26,4 | 15,5 | 30,6 | 30,4 |
| Vergleichsbeispiel 1 | 74,5 | 38,6 | 11,4 | 40,2 | 16,5 |
| Vergleichsbeispiel 2 | 73,7 | 48,7 | 29,1 | 56,8 | 30,8 |
| Vergleichsbeispiel 3 | 59,6 | 20,1 | 9,6 | 22,3 | 25,4 |
| Vergleichsbeispiel 4 | 37,4 | 13,9 | 8,7 | 16,4 | 32,1 |
| Vergleichsbeispiel 5 | 83,0 | 5,8 | 24,4 | 25,1 | 76,7 |
| [1]) Gemessen auf schwarzem Untergrund der Schwarz-Weiß-Deckungskarte. | | | | | |

IIc Deckungsvergleich

Zur Bestimmung des Deckungsquotienten $D_q$, definiert als $D_q = \dfrac{L^{*110}_{schwarz}}{L^{*110}_{weiß}}$ , wurden Lackapplikationen analog IIb jedoch mit einer Pigmentierungshöhe von 10 Gew.-% und mit einer Spiralrakel in einer Nassfilmdicke von 100 μm auf Schwarz-Weiß-Deckungskarten (Byko-Chart 2853, Fa. Byk-Gardner) appliziert und nachfolgend bei Raumtemperatur getrocknet. Anschließend wurden die Helligkeitswerte L*110° der Lackapplikationen mit dem Mehrwinkelfarbmessgerät BYK-mac (Fa. Byk-Gardner) bei einem Messwinkel von 110° auf schwarzem und auf weißem Untergrund der Schwarz-Weiß-Deckungskarte aufgenommen. Die Messgeometrie 110° bezieht sich, bei einem konstanten Einfallswinkel von 45°, auf die Differenz zum Glanzwinkel. Der Betrachtungswinkel wird von der spekularen Reflexion in der Beleuchtungsebene weg gemessen.

Die erfindungsgemäßen Effektpigmente weisen eine gute Deckkraft auf. Ihr Deckungsquotient $D_q$ liegt vorzugsweise bei ≥ 0,41. Der Deckungsquotient $D_q$ der erfindungsgemäßen plättchenförmigen rotfarbenen Effektpigmente der Beispiele 1 bis 6 liegt, wie Tabelle 6 zu entnehmen ist, jeweils deutlich über 0,41.

IId Glanzmessungen

Der Glanz ist ein Maß für die gerichtete Reflexion. Zur Bestimmung des Glanzes wurden die Lackapplikationen aus IIb auf dem weißen Untergrund der Schwarz-Weiß-Deckungskarte mit Hilfe eines Micro-Tri-Gloss Glanzmessgerätes, Fa. Byk-Gardner, bei einem Messwinkel von 60° bezogen auf die Vertikale vermessen. Die Glanzwerte der erfindungsgemäßen rotfarbenen Effektpigmente sowie der Pigmente der Vergleichsbeispiele sind in Tabelle 5 aufgeführt.

Die erfindungsgemäßen plättchenförmigen rotfarbenen Effektpigmente aus den Beispielen 1 bis 6 zeigen zum Teil deutlich höhere Glanzwerte als die einschichtig beschichteten Pigmente aus den Vergleichsbeispielen 1 und 2.

Die Glanzmessungen aus Tabelle 5 bestätigen die sehr hohe Reflektivität der erfindungsgemäßen Pigmente im Vergleich zum Stand der Technik.

Tabelle 5: Glanzwerte

| Beispiel/ Vergleichsbeispiel | Glanz 60° (w) |
|---|---|
| Beispiel 1 | 46,6 |
| Beispiel 2 | 53,9 |
| Beispiel 3 | 37,6 |
| Beispiel 4 | 42,4 |
| Beispiel 5 | 42,7 |
| Beispiel 6 | 51,4 |
| Beispiel 7 | 50,4 |
| Vergleichsbeispiel 1 | 41,8 |
| Vergleichsbeispiel 2 | 32,3 |

IIe Effektmessungen

Um den optischen Effekt der erfindungsgemäßen rotfarbenen Effektpigmente objektiv zu beschreiben, wurden Effektmessungen mit dem Spektralphotometer BYK-mac (Fa. Byk-Gardner) anhand der Lackapplikationen aus IIb durchgeführt (vgl. Byk-Gardner, Katalog "Qualitätskontrolle für Lacke und Kunststoffe" 2011/2012, S. 97/ 98). Die entsprechenden Messwerte für die Glitzerintensität $S_i$, die Glitzerfläche $S_a$ und die Körnigkeit G sind in Tabelle 6 zusammengefasst.

Tabelle 6: Effektmessungen, Deckungsquotient und Glanzwerte

| Beispiel/Vergleichsbeispiel | $S_i$ 15° (s)[1] | $S_a$ 15° (s)[1] | G (s)[1] | $D_q$ 110°[2] |
|---|---|---|---|---|
| Beispiel 1 | 7,33 | 30,04 | 4,77 | 0,939 |
| Beispiel 2 | 6,35 | 27,85 | 4,68 | 0,923 |
| Beispiel 3 | 5,69 | 25,00 | 4,23 | 0,955 |
| Beispiel 4 | 8,61 | 31,31 | 4,41 | 0,922 |
| Beispiel 5 | 6,23 | 29,01 | 4,04 | 0,967 |
| Beispiel 6 | 21,70 | 37,58 | 8,12 | 0,590 |
| Vergleichsbeispiel 1 | 4,70 | 19,38 | 4,15 | 0,800 |
| Vergleichsbeispiel 2 | 4,28 | 18,57 | 3,45 | / |
| Vergleichsbeispiel 3 | 7,12 | 27,03 | 4,92 | / |
| Vergleichsbeispiel 4 | 21,81 | 27,46 | 5,05 | / |
| Vergleichsbeispiel 5 | / | / | / | 0,673 |
| [1] Gemessen auf schwarzem Untergrund der Schwarz-Weiß-Deckungskarte. [2] Gemessen an 10%igem NC-Lack Abzug 100 μm Nassfilmdicke | | | | |

Die Effektwerte $S_i$, $S_a$ sowie G der erfindungsgemäßen plättchenförmigen rotfarbenen Effektpigmente aus den Beispielen 1 bis 6 sind zumeist höher als die Werte der Vergleichsbeispiele. Die erzielbaren optischen Effekte der erfindungsgemäßen plättchenförmigen rotfarbenen Effektpigmente sind deutlich stärker als bei herkömmlichen einschichtig beschichteten Effektpigmenten aus den Vergleichsbeispielen 1 und 2 ausgeprägt.

IIf Waring Blender

In der Industrie werden viele Lacke in Kreislaufsystemen verarbeitet. Hierbei werden die Lackkomponenten hohen Scherkräften ausgesetzt. Der Waring Blender Test simuliert nun diese Bedingungen und dient zur Bewertung der Ringleitungs- bzw. der Scherstabilität. Gerade Pigmente, deren Beschichtung nicht ausreichend auf dem Trägermaterial verankert ist, zeigen bei diesem Test starke Abweichungen der Chromawerte im Vergleich zu den unbehandelten Applikationen. Der Waring Blender Test kann somit als Maß für die Zwischenhaftung der Pigmentbeschichtung gegenüber Scherkräften verstanden werden.

Hierzu wurden die erfindungsgemäßen rotfarbenen Effektpigmente bzw. die Pigmente der Vergleichsbeispiele gemäß nachstehendem Ansatz eingewogen und schrittweise mit einem konventionellen Acryllack in einem 880 ml Becher angeteigt. Danach wurde die Viskosität mit Butylacetat/ Xylol 1:1 auf 17" im DIN 4 mm-Becher eingestellt. Es wurden insgesamt 600 g Lack hergestellt, wovon 400 g in ein doppelwandiges 1 kg-Gefäß mit Wasserkühlung eingefüllt und unter dem Dispermaten (Fa. Waring Blender) mit einem speziellen Aufsatz verrührt wurden. Die Rührzeit betrug 8 Minuten bei 13.500 U/Min, dann wurden 200 g Lack entnommen und der Rest wurde weitere 12 Minuten gerührt.

Ansatz: 6 % Pigment

8 % Butylacetat 85

86 % Acryllack, farblos

30 % Verdünnung Butylacetat 85/ Xylol 1:1

Jeweils 200 g des unbehandelten und des behandelten Lacks wurden mit einem Spritzautomaten und der Sata-Spritzpistole LP-90 nach folgender Einstellung auf ein Prüfblech appliziert.

Einstellung: Nadel: 1.3.4

Druck: 4 bar

Gänge: Die Zahl der Spritzgänge wurde so gewählt, dass eine trockene Lackschichtdicke von 15-20 $\mu$m vorlag.

Konventionsweise gelten Effektpigmente dann als scherstabil, wenn in der Applikation nach dem Waring Blender Test die Glanz- als auch die Farbdifferenz, nahe am Glanzwinkel gemessen, relativ gering ist. Der $\Delta C^*_{15}$-Wert zur unbehandelten Probe sollte idealerweise kleiner 2 sein.

In Tabelle 7 ist die Farbänderung $\Delta C^*_{15}$ sowie die Glanzänderung $\Delta$Glanz 60°der dem Waring Blender Test unterzogenen Probe zur unbehandelten Probe anhand der erfindungsgemäßen Beispiele 1 und 3 aufgezeigt.

Tabelle 7: Glanz- und Farbdifferenz im Waring Blender Test

|  | $\Delta$C*(15°) | $\Delta$Glanz (60°) |
|---|---|---|
| Beispiel 1 | 1,15 | -1,3 |
| Beispiel 3 | 2,31 | -2,6 |

Die erfindungsgemäßen rotfarbenen Effektpigmente aus den Beispielen 1 und 3 erfüllen die Kriterien des Tests. Der Farbunterschied ist vernachlässigbar gering. Auch unter dem Mikroskop konnten kaum Veränderungen wie Abplatzungen der Beschichtung oder sonstige entstandene Oberflächendefekte festgestellt werden.

Die erfindungsgemäßen rotfarbenen Effektpigmente zeigen sich trotz ihrer Abstandslage extrem scherstabil.

IIg Bestimmung der Chemikalienbeständigkeit

Die Chemikalienbeständigkeit der erfindungsgemäßen rotfarbenen Effektpigmente und der Pigmente der Vergleichsbeispiele wurde anhand von Lackapplikationen auf Kunststoffpanels bestimmt. 6 g des jeweiligen Pigments wurden in eine Mischung aus 90 g eines konventionellen farblosen Acryllacks und 10 g Butylacetat 85 eingerührt. Danach wurde die Viskosität mit einem Gemisch aus Butylacetat 85 und Xylol im Verhältnis 1:1 auf 17" im DIN 4 mm-Becher eingestellt.

Jeweils 100 g dieses Lackes wurden analog zu IIf mit einem Spritzautomaten auf die Panels deckend appliziert. Nach der Beschichtung wurden die Panels 30 Minuten bei 80°C eingebrannt.

24 Stunden später wurden die Panels bis zur Hälfte in 10%ige Natronlauge getaucht. Nach einer Einwirkzeit von 7 Tagen wurden die Panels mit VE-Wasser abgewaschen und nach 2 Stunden Trockenzeit dann auf Beschädigung und/oder Verfärbungen hin visuell beurteilt. Weiterhin wurden Verfärbungen mit Hilfe des BYK-mac (Fa. Byk-Gardner) vermessen. Zur Charakterisierung der Farbänderung wurde der $\Delta$E-Wert der belasteten Probe gegen die entsprechende unbelastete Probe bei einem Messwinkel von 15° herangezogen. Die Ergebnisse sind in nachstehender Tabelle 8 wiedergegeben.

Tabelle 8: Farbänderung $\Delta$E

| Beispiel/Vergleichsbeispiel | $\Delta$E(15°) |
|---|---|
| Beispiel 1 | 1,8 |

(fortgesetzt)

| Beispiel/Vergleichsbeispiel | ∆E(15°) |
|---|---|
| Vergleichsbeispiel 3 | 13,31 |

Pigmente mit einem ∆E(15°) <3 können als chemikalienstabil angesehen werden. Das erfindungsgemäße rotfarbene Effektpigmente aus Beispiel 1 liegt unterhalb, während das Pigment aus Vergleichsbeispiel 3 den Grenzwert deutlich überschreitet.

IIh Röntgenfluoreszenzanalyse (RFA)

Die Metalloxid-, Metallhydroxid- und/oder Metalloxidhydratgehalte der erfindungsgemäßen rotfarbenen Effektpigmente sowie der Pigmente der Vergleichsbeispiele wurde mittels Röntgenfluoreszenzanalyse (RFA) bestimmt. Hierzu wurden die jeweiligen Pigmente in eine Lithiumtetraboratglastablette eingearbeitet, in Festprobenmessbechern fixiert und daraus vermessen. Als Messgerät diente das Gerät Advantix ARL, Fa. Thermo Scientific. Die Messwerte sind in Tabelle 9 widergegeben. Dabei wurden die Angaben der verschiedenen Gehalte für Titan als $TiO_2$, für Eisen als $Fe_2O_3$, für Zr als $ZrO_2$, für Si als $SiO_2$ und für Zinn als $SnO_2$ angegeben.

Tabelle 9: Mittlere Höhe $h_a$ der Abstandslage und RFA-Werte

| Beispiel/ Vergleichsbeispiel | REM Mittlere Höhe $h_a$ [nm] | RFA (als Oxid) Ti [%] | Fe [%] | Sn [%] | Zr [%] | Si [%] |
|---|---|---|---|---|---|---|
| Beispiel 1 | 46 | / | 55,9 | 3,8 | / | / |
| Beispiel 2 | 20 | 9,1 | 51,1 | / | / | / |
| Beispiel 3 | 43 | / | 59,5 | 1,4 | / | / |
| Beispiel 4 | 56 | 25,8 | 34,4 | 3,4 | | / |
| Beispiel 5 | 32 | / | 54,2 | / | 4,1 | / |
| Beispiel 6 | 38 | 2,3 | 30,5 | 0,42 | | / |
| Vergleichsbeispiel 1 | Keine Abstandslage | / | 48,5 | / | / | / |
| Vergleichsbeispiel 2 | Keine Abstandslage | 0,04 | 55,7 | / | / | 42,9 |
| Vergleichsbeispiel 3 | Keine Abstandslage | / | / | / | / | / |
| Vergleichsbeispiel 4 | Keine Abstandslage | 1,1 | 18 | 0,5 | / | / |
| Vergleichsbeispiel 5 | Keine Abstandslage | 2,1 | 21,7 | 2 | / | / |

IIi Schwitzwassertest

Zur Bestimmung der Schwitzwasserbeständigkeit wurden die erfindungsgemäßen rotfarbenen Effektpigmente bzw. die Pigmente der Vergleichsbeispiele in ein Wasserlacksystem eingearbeitet und die Prüfapplikationen durch Spritzlackierung auf Aluminiumblechen hergestellt. Der Basislack wurde mit einem handelsüblichen 1K-Klarlack überlackiert und anschließend eingebrannt. Diese Applikationen wurden nach DIN 50 017 (Kondenswasser-Konstantklimate) geprüft. Die Haftfestigkeit wurde mittels Gitterschnitt nach DIN EN ISO 2409 sofort nach Testende im Vergleich zur unbelasteten Probe geprüft. Hierbei bedeutet Gt 0 keine Veränderung und Gt 5 eine sehr starke Veränderung.
Das Quellverhalten wurde unmittelbar nach Schwitzwasserbelastung in Anlehnung an die DIN 53230 visuell beurteilt. Hierbei bedeutet die Kennzahl 0: keine Veränderung und die Kennzahl 5: sehr starke Veränderung.
Schließlich wurde der DOI (distinctness of image) anhand eines Wave-scan II Fa. Byk-Gardner) bestimmt.

Tabelle 10: Schwitzwasserergebnisse

| Probe | Glanz 20° vor SW-Test | Glanz 20° nach SW-Test | Glanzverlust | Gitterschnittsofort | Quellung visuell |
|---|---|---|---|---|---|
| Beispiel 8 | 91 | 91 | 0 | 1 | 0 |

(fortgesetzt)

| Probe | Glanz 20° vor SW-Test | Glanz 20° nach SW-Test | Glanzverlust | Gitterschnittsofort | Quellung visuell |
|---|---|---|---|---|---|
| Vergleichsbeispiel 2 | 89 | 42 | 53% | 5 | 4 |

Das Pigment aus Vergleichsbeispiel 2 wies ein starkes Quellverhalten und eine schlechte Zwischenschichthaftung auf. Der DOI war aufgrund der hohen Feinstruktur nach Schwitzwasserbelastung nicht mehr messbar.

Das erfindungsgemäße rotfarbenen Effektpigment aus Beispiel 8 hingegen zeigte sich stabil und wies vor und nach dem Test nahezu keine Veränderungen auf.

Ilj Bestimmung der mittleren Dicke der nichtmetallischen plättchenförmigen Substrate, der mittleren Schichtdicke der Schichten 2 und 3, der mittleren Schichtdicke der gesamten Beschichtung, der mittleren Höhe $h_a$ der Abstandslage sowie der mittleren Höhe $h_H$ der Hohlräume

Hierzu wurden die erfindungsgemäßen rotfarbenen Effektpigmente 10%ig in einem 2K Klarlack Autoclear Plus HS der Fa. Sikkens GmbH mit einem Hülsenpinsel eingearbeitet, mit Hilfe einer Spiralrakel (26 $\mu$m Nassfilmdicke) auf eine Folie appliziert und getrocknet. Nach 24 h Abtrocknungszeit wurden von diesen Rakelabzügen Querschliffe angefertigt. Die Querschliffe wurden im REM vermessen, wobei zur Bestimmung der mittleren Dicke der nichtmetallischen plättchenförmigen Substrate mindestens 100 Einzelpigmente vermessen wurden, um eine aussagefähige Statistik zu erhalten.

Zur Bestimmung der mittleren Schichtdicke der Schichten 2 und 3, der mittleren Dicke der gesamten Beschichtung, der mittleren Höhe $h_a$ der Abstandslage sowie der mittleren Höhe $h_H$ der Hohlräume wurde die obere und untere Substratoberfläche, d.h. die im REM-Querschliff erkennbar jeweils längere Seite des nichtmetallischen plättchenförmigen Substrats, jeweils als Basislinie herangezogen. Die Basislinie wurde hierbei in die rasterelektronenmikroskopische Querschliffaufnahme entlang der Oberfläche des plättchenförmigen Substrats in der Querschliffaufnahme gelegt, indem die zwei Schnittpunkte nichtmetallisches plättchenförmiges Substrat-optionale Schicht 1 bzw. nichtmetallisches plättchenförmiges Substrat - Schicht 2 vom linken und rechten Rand der rasterelektronenmikroskopischen Querschliffaufnahme miteinander durch eine Gerade verbunden wurden. Die rasterelektronenmikroskopischen Querschliffaufnahmen wurden mit Hilfe der Bildverarbeitungssoftware AxioVision 4.6.3. (Fa. Zeiss) untersucht.

Im 90° Winkel zu diesen beiden Basislinien wurden im 50 nm Abstand so viele parallele Linien eingezogen, dass über die komplette rasterelektronenmikroskopische Querschliffaufnahme des Effektpigments ein Raster gelegt war (Abbildung 4). Die Vergrößerung der rasterelektronenmikroskopischen Querschliffaufnahme betrug vorzugsweise mindestens 50000-fach, bezogen auf Polaroid 545 (4" x 5"). Ausgehend von der jeweiligen oberen und unteren Basislinie vom nichtmetallischen plättchenförmigen Substrat jeweils in Richtung Schicht 3 wurden die Abstände zwischen den Schnittpunkte dieser Linien an den jeweiligen Grenzflächen der optionalen Schicht 1 zur Schicht 2, Schicht 2 zur Abstandslage, Abstandslage zur Schicht 3 und Schicht 3 zur Umgebung manuell vermessen. Hierbei kam es vor, dass eine der im 50 nm Abstand eingezeichneten Linien direkt über einer Verbindungsstelle bzw. einem Abstandshalter lag. In diesem Fall wurde nur der jeweilige Schnittpunkt der Linie an der Grenzfläche Schicht 3 zur Umgebung erfasst. Aus diesen Messwerten ergaben sich die Schichtdicken der Schichten 2 und 3, die Dicke der gesamten Beschichtung, die Schichtdicke optional weiter vorhandener Schichten sowie die Höhe $h_a$ der Abstandslage durch Differenzbildung.

Für die Bestimmung der mittleren Höhe $h_H$ der Hohlräume wurden die Schnittpunkte dieser parallelen Linien mit der oberen und unteren Hohlraumbegrenzung innerhalb der Abstandslage herangezogen. Aus den auf die Weise bestimmten Einzelwerten der Schichtdicken, der Höhe $h_a$ sowie der Höhe $h_H$ wurden die jeweiligen arithmetischen Mittelwerte gebildet, um die oben angegebenen Werte der mittleren Schichtdicken, der mittleren Höhe $h_H$ bzw. der mittleren Höhe $h_a$ zu bestimmen. Für eine aussagekräftige Statistik wurden die oben beschriebenen Messungen an mindestens 100 Linien durchgeführt.

Mit dem Begriff "mittlere" ist in allen Fällen der arithmetische Mittelwert gemeint.

Querschliffe der Pigmente der Vergleichsbeispiele, welche keine Abstandslage, aber gegebenenfalls statistisch verteilte Poren innerhalb der Beschichtung aufweisen, wurden ebenfalls nach dem vorstehend beschriebenen Verfahren anhand rasterelektronenmikroskopischer Querschliffaufnahmen untersucht. Hierbei wurden, sofern eine der parallelen Linien über einer oder mehreren Poren zu liegen kam, die Höhe der Pore(n), deren Porenmittelpunkt(e) und der Abstand des Porenmittelpunkts oder der Porenmittelpunkte zur Substratoberfläche bestimmt.

Alternativ zu Querschliffen können die erfindungsgemäßen rotfarbenen Effektpigmente mittels des FIB-Verfahrens (FIB = focused ion beam) angeschnitten werden. Dazu wird ein feiner Strahl aus hochbeschleunigten Ionen (z.B. Gallium, Xenon, Neon oder Helium) mittels einer Ionenoptik auf einen Punkt fokussiert und zeilenweise über die zu bearbeitende Effektpigmentoberfläche geführt. Die Ionen geben beim Aufprall auf die Effektpigmentoberfläche einen Großteil ihrer Energie ab und zerstören die Beschichtung an diesem Punkt, was zu einem zeilenweisen Materialabtrag führt. Auch anhand der dann aufgenommenen rasterelektronenmikroskopischen Aufnahmen kann nach dem oben beschriebenen Verfahren die mittlere Höhe $h_a$, die mittlere Schichtdicke der Schichten 2 und 3 sowie die mittlere Schichtdicke der gesamten Beschichtung ermittelt werden. Auch die mittlere Dicke des nichtmetallischen plättchenförmigen Substrats kann anhand von rasterelektronenmikroskopischen Aufnahmen der durch das FIB-Verfahren angeschnittenen Effektpigmente bestimmt werden.

Die Vorteile der erfindungsgemäßen rotfarbenen Effektpigmente ergeben sich daher in der Summe verschiedener Eigenschaften. Die erfindungsgemäßen rotfarbenen Effektpigmente weisen eine hohe Transparenz, eine sehr gute mechanische und chemische Stabilität sowie einen hohen Glanz und Farbstärke auf. Keines der Vergleichspigmente weist in der Gesamtbetrachtung die genannten Eigenschaften sämtlich in zufriedenstellender Weise auf.

Tabelle 11: Charakterisierung der Beschichtung

| Beispiel/ Vergleichsbeispiel | $d_{S2}$ [nm] | $d_{S3}$ [nm] | $d_{S2}/d_{S3}$ | $h_{ma}$ [nm] | $h_{Rma}$ | $\sigma h_{Rma}$ [%] | $n_S$ | $S_D$ [%] | $A_H$ [%] |
|---|---|---|---|---|---|---|---|---|---|
| Beispiel 1 | 67 | 107 | 0,63 | 90 | 0,41 | 3,10 | 0,4 | 2,1 | 97,9 |
| Beispiel 2 | 11 | 82,3 | 1,44 | 128,3 | 0,61 | 4,50 | 6,2 | 30,9 | 69,1 |
| Beispiel 3 | 85 | 96 | 0,89 | 107 | 0,48 | 1,40 | 1,0 | 5,2 | 94,8 |
| Beispiel 4 | 11 | 113 | 1,00 | 141 | 0,500 | 4,20 | 0,4 | 2,1 | 97,9 |
| Vergleichsbeispiel 1 | Keine Abstandslage | | | | | 24,7 | 13 | 65,0 | 35,0 |

$d_{S2}$ [nm] = mittlere Schichtdicke der Schicht 2
$d_{S3}$ [nm] = mittlere Schichtdicke der Schicht 3
$n_S$ = mittlere Anzahl Stege je $\mu$m
$A_H$ [%] = Flächenanteil Hohlraum
$S_D$ = Steganzahldichte [%]
$h_{ma}$ = Mitte der Abstandslage (Summe aus der Schichtdicke der optionalen Schicht 1, der Schicht 2 und der halben Höhe $h_a$)
$h_{Rma}$ = Relative Höhe der Abstandslage
$\sigma h_{Rma}$ [%] = Standardabweichung der relativen Höhe der Abstandslage

In Tabelle 11 ist die mittlere Höhe $h_a$ der Abstandslage der gemessenen Pigmente aufgezeigt. Alle erfindungsgemäßen rotfarbenen Effektpigmente weisen im Gegensatz zu den Pigmenten der Vergleichsbeispiele 1 bis 5 eine Abstandslage auf.

Die Pigmente aus den Vergleichsbeispielen 1 bis 5 weisen keine Abstandslage auf. In Tabelle 11 ist für das Vergleichsbeispiele 1 mit dem Wert in der Spalte $\sigma h_{Rma}$ [%] die Standardabweichung der Porenmittelpunkte zur Substratoberfläche gemeint.

Da das Pigment aus Vergleichsbeispiel 1 jedoch nur wenig statistisch verteilte Poren enthält, liegt die Steganzahldichte $S_D$ bei 65,0%. Die Standardabweichung der Porenmittelpunkte zur Substratoberfläche liegt bei 24,7%, wodurch belegt ist, dass die Poren statistisch verteilt innerhalb der gesamten Beschichtung vorliegen. Anders verhält es sich bei den erfindungsgemäßen rotfarbenen Effektpigmenten aus den Beispielen 1,bis 4. Hier ist die Standardabweichung der relativen Höhe der Mitte der Abstandslage $h_{Rma}$ jeweils < 5%, was aufzeigt, dass deren jeweilige Abstandslage in einer definierten Position innerhalb der Beschichtung liegt. Die Standardabweichung der Abstände der Porenmittelpunkte zur Substratoberfläche des Pigments aus dem Vergleichsbeispiel 1 kann somit der Standardabweichung der relativen Höhe der Mitte der Abstandslage der erfindungsgemäßen rotfarbenen Effektpigmente gegenübergestellt werden.

IIk Rasterelektronenmikroskopische Aufnahmen

Die rasterelektronenmikroskopischen Aufnahmen wurden anhand von Querschliffen der erfindungsgemäßen rotfarbenen Effektpigmente mit dem Rasterelektronenmikroskop Supra 35 (Fa. Zeiss) erhalten. Die energiedispersive Röntgenmikroanalyse (EDX-Analyse) wurde mit dem Gerät EDAX Sapphire, Fa. EDAX, durchgeführt.

**III Anwendungstechnische Beispiele**

Anwendungstechnisches Beispiel 1: Körperlotion

| INCI Name | Produktname | Gew.-% | Hersteller/ Lieferant |
|---|---|---|---|
| *Phase A* | | 85,80 | |
| | Effektpigment aus Beispiel 1 | 0,20 | |
| Aqua | Water | | |
| Glycerin | Glycerin 85% | 2,00 | H. Erhard Wagner |
| Xanthan Gum | Keltrol CG-T | 0,60 | CP Kelco |
| *Phase B* | | | |
| Isopropyl Palmitate | Isopropylpalmitat | 3,00 | H. Erhard Wagner |
| Glyceryl Stearate | Aldo MS K FG | 2,00 | Lonza |
| Cocos Nuifera Oil | Ewanol KR | 2,00 | H. Erhard Wagner |
| Cetearyl Alcohol | Tego Alkanol 1618 | 2,00 | Evonik |
| Dimethicone | Element 14 PDMS | 1,00 | Momentive |
| Sodium Polyacrylate | Cosmedia SP | 0,50 | BASF |
| *Phase C* | | | |
| Phenoxyethanol, Ethylhexylglycerin | Euxyl PE 9010 | 0,80 | Schülke & Meyr |
| Fragrance | Vitamin Bomb | 0,10 | Bell Europe |

Das Effektpigment aus Beispiel 1 kann in einem Bereich von 0,1 bis 2,5 Gew.-%, bezogen auf das Gesamtgewicht der Körper Lotion Formulierung, eingesetzt werden. Der Ausgleich auf 100 Gew.-% der Formulierung kann mit Wasser erfolgen.

Keltrol CG-T wurde in Phase A dispergiert und auf 75°C erhitzt. Phase B wurde separat auf 75°C erhitzt. Anschließend wurde Phase B langsam zu Phase A hinzugefügt. Unter Rühren wurde die Emulsion auf Raumtemperatur abgekühlt und Phase C einzeln zugegeben.

Anwendungstechnisches Beispiel 2: Creme Lidschatten

| INCI Name | Produktname | Gew.-% | Hersteller/ Lieferant |
|---|---|---|---|
| *Phase A* | | | |
| Microcrystalline Wax | TeCero-Wax 1030 K | 4,50 | Tromm Wachs |
| Copernicia Cerifera Cera | Carnaubawachs LT 124 | 4,50 | Tromm Wachs |
| Isohexadecane | Isohexadecane | 21,00 | Ineos |
| Cyclopentasiloxane, Dimethicone/ Vinyltrimethylsiloxysilicate Crosspolymer | Belsil RG 100 Silicone Elastomer Resin Gel | 8,00 | Wacker |
| Trimethylsiloxyphenyl Dimethicone | Belsil PDM 20 | 6,00 | Wacker |
| Dimethicone | Belsil DM 100 | 14,00 | Wacker |
| Caprylic/Capric Triglyceride | Miglyol 812 | 7,00 | Sasol |
| Cyclomethicone (and) Quatemium-90 Bentonite (and) Propylene Carbonate | Tixogel VSP-1438 | 5,00 | BYK |

(fortgesetzt)

| Phase B | | | |
|---|---|---|---|
| | Effektpigment aus Beispiel 3 | 30,00 | |

Das Effektpigment aus Beispiel 3 kann in einem Bereich von 5 bis 30,0 Gew.-%, bezogen auf das Gesamtgewicht der Lidschatten Formulierung, eingesetzt werden. Der Ausgleich auf 100 Gew.-% der Formulierung kann mit Isohexadecane erfolgen.

Phase A wurde gemischt und auf 85°C erhitzt, Phase B wurde anschließend unter Rühren zu Phase A hinzugegeben. Nach Abfüllen in ein entsprechendes Behältnis wird die Mischung auf Raumtemperatur abgekühlt.

Anwendungstechnisches Beispiel 3: Duschgel

| INCI Name | Produktname | Gew.-% | Hersteller/Lieferant |
|---|---|---|---|
| *Phase A* | | | |
| | Effektpigment aus Beispiel 5 | 0,10 | |
| Aqua | Wasser | 58,50 | |
| Acrylates Copolymer | Carbopol Aqua SF-1 | 5,50 | Lubrizol |
| *Phase B* | | | |
| Sodium Hydroxide | NaOH (10 Gew.-%) | 1,50 | |
| *Phase C* | | | |
| Sodium Laureth Sulfate | Zetesol NL-2 U | 22,00 | Zschimmer & Schwarz |
| Cocamidopropyl Betaine | Amphotensid B5 | 6,00 | Zschimmer & Schwarz |
| PEG-7 Glyceryl Cocoate | Emanon HE | 2,00 | Kao Corp. |
| Disodium Laureth Sulfosuccinate | Sectacin 103 Spezial | 2,00 | Zschimmmer & Schwarz |
| *Phase D* | | | |
| Phenoxyethanol (and) Piroctone Olamine | Nipaguard PO 5 | 0,60 | Clariant |
| Fragrance | Water Lily OA | 0,20 | Bell Flavors and Fragrances |
| Sodium Chloride | Sodium Chloride | 1,60 | VWR |

Das Effektpigment aus Beispiel 5 kann in einem Bereich von 0,01 bis 1,0 Gew.-%, bezogen auf das Gesamtgewicht der Duschgel Formulierung, eingesetzt werden. Der Ausgleich auf 100 Gew.-% der Formulierung kann mit Wasser erfolgen.

Phase A wurde gerührt, danach wurde Phase B zugegeben und gerührt bis ein homogenes Erscheinungsbild erreicht wurde. Phase C wurde separat eingewogen, kurz vermischt und zu Phase AB hinzugefügt. Anschließend wurde erneut gerührt und Phase D einzeln zugegeben.

Anwendungstechnisches Beispiel 4: Gepresster Lidschatten

| INCI Name | Produktname | Gew.-% | Hersteller/Lieferant |
|---|---|---|---|
| *Phase A* | | | |
| Talc | Talc Powder | 36,00 | VWR |

(fortgesetzt)

| INCI Name | Produktname | Gew.-% | Hersteller/Lieferant |
|---|---|---|---|
| *Phase A* | | | |
| Bentonite | Optigel CK-PC | 5,00 | BYK |
| Synthetic Fluorphlogopite | Synafil S 1050 | 13,00 | ECKART |
| Aluminium Starch Octenylsuccinate | Agenaflo OS 9051 | 10,00 | Agrana |
| Magnesium Stearate | Magnesium Stearate | 6,00 | VWR |
| | Effektpigment aus Beispiel 7 | 20,00 | |
| *Phase B* | | | |
| Cyclomethicone | Xiameter PMX-0345 | 5,00 | Dow Corning |
| Octyldodecyl Stearoyl Stearate | Ceraphyl 847 | 5,00 | Ashland |

Das Effektpigment aus Beispiel 7 kann in einem Bereich von 5,0 bis 40,0 Gew.-%, bezogen auf das Gesamtgewicht der Lidschatten Formulierung, eingesetzt werden. Der Ausgleich auf 100 Gew.-% der Formulierung kann mit Talc erfolgen.

Phase A wurde für 30s bei 2500 U/min in einem Hochgeschwindigkeitsmixer gemischt. Anschließend wurde Phase B zugegeben und die Mischung für 60s bei 3000 U/min im gleichen Mixer gemischt. Zuletzt wird die Pulvermischung mittels einer Lidschattenpresse bei 100 bar für 30 Sekunden in Form gepresst.

Anwendungstechnisches Beispiel 5: Mascara

| INCI Name | Produktname | Gew.-% | Hersteller/ Lieferant |
|---|---|---|---|
| *Phase A* | | | |
| Aqua | Water | 73,00 | |
| Bentonite (and) Xanthan Gum | Optigel WX-PC | 2,00 | BYK |
| *Phase B* | | | |
| Cetyl Alcohol (and) Glyceryl Stearate (and) PEG-75 Stearate (and) Ceteth-20 (and) Steareth-20 | Emulium Delta | 5,00 | Gattefosse |
| C10-18 Triglycerides | Lipocire A Pellets | 2,00 | Gattefosse |
| Ozokerite | Kahlwax 1899 | 2,00 | Kahl |
| Glyceryl Behenate | Compritol 888 CG Pastilles | 2,00 | Gattefosse |
| Butylene Glycol Cocoate | Cocoate BG | 4,00 | Gattefosse |
| *Phase C* | | | |
| | Effektpigment aus Beispiel 7 | 5,00 | |
| Phenoxyethanol (and) Piroctone Olamine | Nipaguard PO5 | 0,50 | Clariant |
| Glycine Soja (Soybean) Oil, Dicaprylyl Ether, Magnolia Grandiflora Bark Extract, Lauryl Alcohol | Follicusan DP | 3,00 | CLR Berlin |

(fortgesetzt)

| Phase C | | | |
|---|---|---|---|
| Water, Hydrolyzed Corn Starch, Beta Vulgaris (Beet) Root Extract | DayMoist CLR | 1,00 | CLR Berlin |
| Linoleic Acid (and) Linolenic Acid | Vitamin F forte | 0,50 | CLR Berlin |

Das Effektpigment aus Beispiel 7 kann in einem Bereich von 1,0 bis 10,0 Gew.-%, bezogen auf das Gesamtgewicht der Mascara Formulierung, eingesetzt werden. Der Ausgleich auf 100 Gew.-% der Formulierung kann mit dem Wasser aus Phase A erfolgen.

Phase A wurde unter hoher Scherung gerührt. Phase B wurde separat eingewogen. Phase A und Phase B wurden getrennt auf 85°C erhitzt, danach wurde Phase B zu Phase A hinzugefügt. Anschließend wurde Phase AB auf 45°C abgekühlt und während des Abkühlens Phase C unter Rühren nach und nach hinzugefügt.

Anwendungstechnisches Beispiel 6: Haargel

| INCI Name | Produktname | Gew.-% | Hersteller/ Lieferant |
|---|---|---|---|
| Phase A | | | |
| Sodium Magnesium Silicate (nano) | Laponite XLG | 2,00 | BYK |
| Aqua | Wasser | 94,80 | |
| Phase B | | | |
| | Effektpigment aus Beispiel 6 | 0,10 | |
| Citric Acid (and) Water | Citric Acid (10%) | 0,30 | |
| Glycerin, Water, Avena Strigosa Seed Extract, Lecithin, Potassium Sorbate, Citric Acid | Aquarich | 1,50 | Rahn AG |
| Fragrance | Lychee & Grape | 0,10 | Bell Europe |
| Methylisothiazolinone (and) Phenethyl Alcohol (and) PPG-2-Methyl Ether | Optiphen MIT Plus | 1,20 | Ashland |

Das Effektpigment aus Beispiel 6 kann in einem Bereich von 0,01 bis 2,0 Gew.-%, bezogen auf das Gesamtgewicht der Haargel Formulierung, eingesetzt werden. Der Ausgleich auf 100 Gew.-% der Formulierung kann mit Wasser erfolgen.

Das Laponite XLG wurde mit Wasser solange gerührt, bis die Phase A klar wurde. Danach wurde das Effektpigment aus Beispiel 6 der Phase B unter Rühren hinzugegeben. Anschließend wurden die restlichen Inhaltsstoffe der Phase B nach und nach zugegeben.

Anwendungstechnisches Beispiel 7: Körperpuder

| INCI Name | Produktname | Gew.-% | Hersteller/Lieferant |
|---|---|---|---|
| Phase A | | | |
| Synthetic Fluorphlogopite | Synafil S 1050 | 40,00 | Eckart |
| Polypropylene | Synafil W 1234 | 8,00 | Eckart |

(fortgesetzt)

| INCI Name | Produktname | Gew.-% | Hersteller/Lieferant |
|---|---|---|---|
| *Phase A* | | | |
| Bentonite | Optigel CK-PC | 10,00 | BYK |
| Talc | Talc Powder | 18,00 | VWR |
| Magnesium Stearate | Magnesium Stearate | 4,00 | Applichem |
| | Effektpigment aus Beispiel 5 | 20,00 | |

Das Effektpigment aus Beispiel 5 kann in einem Bereich von 0,2 bis 5,0 Gew.-%, bezogen auf das Gesamtgewicht der Körperpuder Formulierung, eingesetzt werden. Der Ausgleich auf 100 Gew.-% der Formulierung kann mit Synafil S 1050 erfolgen.

Phase A wurde gemischt und anschließend wurde der Puder in ein geeignetes Gefäß abgefüllt.

Anwendungstechnisches Beispiel 8: Lipgloss

| INCI Name | Produktname | Gew.-% | Hersteller/Lieferant |
|---|---|---|---|
| *Phase A* | | | |
| Hydrogenated Polyisobutene (and) Ethylene/Propylene/ Styrene Copolymer (and) Butylene/Ethylene/Styrene Copolymer | Versagel ME 750 | 75,30 | Penreco |
| Simmondsia Chinensis (Jojoba) Seed Oil | Jojoba Oil - Natural | 2,00 | BioChemica |
| Caprylyl Trimethicone | Silcare Silicone 31 M50 | 7,00 | Clariant |
| Stearyl Dimethicone | Silcare Silicone 41 M65 | 3,20 | Clariant |
| Hydrogenated Polydecene | Dekanex 2004 FG | 4,00 | IMCD |
| Isopropyl Myristate | Isopropyl Myristate | 4,50 | VWR |
| *Phase B* | | | |
| | Effektpigment aus Beispiel 6 | 4,00 | |

Das Effektpigment aus Beispiel 6 kann in einem Bereich von 0,10 bis 8,00 Gew.-%, bezogen auf das Gesamtgewicht der Lipgloss Formulierung, eingesetzt werden. Der Ausgleich auf 100 Gew.-% der Formulierung kann mit Versagel ME 750 erfolgen.

Phase A wurde auf 85°C erhitzt, anschließend wurde das Pigment aus Beispiel 6 der Phase B hinzugegeben, gerührt bis eine gleichmäßige Konsistenz entstand und dann in ein Lip Gloss Gefäß abgefüllt.

Anwendungstechnisches Beispiel 9: Lippenstift

| INCI Name | Produktname | Gew.-% | Hersteller/ Lieferant |
|---|---|---|---|
| *Phase A* | | | |
| Octyldodecanol | Eutanol G | 42,5 | BASF |
| Candelilla Cera | Kahlwax 2039 | 6,00 | Kahl |
| Copernicia Cerifera (Carnauba) Wax | Kahlwax 2442 | 6,00 | Kahl |
| Bis-Diglyceryl Polyacyladipate-2 | Softisan 649 | 10,00 | Sasol |
| Polyisobutene | Rewopal PIB 1000 | 10,00 | Evonik |
| Hydrogenated Polydecene | Silkflo 364 NF | 5,00 | Ineos |
| C10-18 Triglycerides | Lipocire A Pellets | 5,00 | Gattefosse |
| Acacia Decurrens/Jojoba/Sunflower Seed Wax/ Polyglyceryl-3 Esters | Hydracire S | 5,00 | Gattefosse |
| Tocopheryl Acetate | dl-alpha-Tocopheryl | 0,50 | IMCD |
| *Phase B* | | | |
| | Effektpigment aus Beispiel 10 | 10,00 | |

Das Effektpigment aus Beispiel 10 kann in einem Bereich von 0,5 bis 20,0 Gew.-%, bezogen auf das Gesamtgewicht der Lippenstift Formulierung, eingesetzt werden. Der Ausgleich auf 100 Gew.-% der Formulierung kann mit Eutanol G erfolgen.

Phase A wurde auf 85°C erhitzt, danach wurde Phase B zu Phase A gegeben und gemischt. Anschließend wurde diese Mischung bei einer Temperatur von 75°C in einem Lippenstiftform abgefüllt.

Anwendungstechnisches Beispiel 10: Flüssig Lidstrich

| INCI Name | Produktname | Gew.-% | Hersteller/ Lieferant |
|---|---|---|---|
| *Phase A* | | | |
| Aqua | Water | 56,90 | |
| Bentonite (and) Xanthan Gum | Optigel WX-PC | 1,40 | |
| *Phase B* | | | |
| Lecithin | Emulmetik 100 | 0,10 | Lucas Meyer |
| Copernicia Cerifera Cera | Kahlwax 2442 | 1,00 | Kahl |
| Stearic Acid | Stearic Acid | 3,50 | Lipo Chemicals |
| Hydrogenated Polyisobutene | Panalane L14 E | 5,00 | Ineos |
| Polysorbate 60 | Mulsifan CPS 60 | 1,50 | Zschimmer & Schwarz |

(fortgesetzt)

| Phase C | | | |
|---|---|---|---|
| | Effektpigment aus Beispiel 2 | 4,00 | |
| Polyurethane-35 | Baycusan C 1004 | 18,00 | Bayer Cosmetics |
| Aqua and Cl 77499 and Methylpropanediol and Ammonium Acrylates Copolymer and Simethicone and Caprylyl Glycol and Phenylpropanol Sodium Acrylates Copolymer | WorléeBase AQ 77499/1 | 8,00 | Worlee |
| Phenoxyethanol, Ethylhexylglycerin | Euxyl PE 9010 | 0,60 | Schülke & Mayr |

Das Effektpigment aus Beispiel 2 kann in einem Bereich von 0,5 bis 8,0 Gew.-%, bezogen auf das Gesamtgewicht der Lidstrich Formulierung, eingesetzt werden. Der Ausgleich auf 100 Gew.-% der Formulierung kann mit Wasser erfolgen.

Optigel WX-PC wurde in Wasser der Phase A dispergiert und 10 Minuten gerührt. Phase A und Phase B wurden separat auf 80°C erhitzt. Danach wurde Phase B langsam zu Phase A unter Rühren hinzugefügt. Nach dem Abkühlen auf 45°C wurden die Inhaltsstoffe der Phase C nach und nach hinzugefügt und in eine geeignete Verpackung abgefüllt.

Anwendungstechnisches Beispiel 11: Mousse

| INCI Name | Produktname | Gew.-% | Hersteller/Lieferant |
|---|---|---|---|
| Phase A | | | |
| Cyclopentasiloxane | Xiameter PMX-0245 Cyclosiloxane | 8,60 | Dow Corning |
| Hydrogenated Polyisobutene | MC 30 | 4,00 | Sophim |
| Dimethicone (and) Dimethicone Crosspolymer | Dow Corning 9041 Silicone Elastomer Blend | 37,14 | Dow Corning |
| Squalane | Squalane | 5,74 | Impag |
| Isononyl Isononanoate | Dermol 99 | 10,16 | Akzo International |
| Hydrogenated Jojoba Oil | Jojoba Butter LM | 2,15 | Desert Whale |
| Hydrogenated Jojaba Oi | Jojoba Butter HM | 1,00 | Desert Whale |
| C30-45 Alkyl Methicone (and) C30-45 Olefin | Dow Corning AMS-C30 Cosmetic Wax | 1,15 | Dow Corning |
| Stearyl Dimethicone | Dow Corning 2503 Cosmetic Wax | 0,47 | Dow Corning |
| Cyclopentasiloxane (and) Polypropylsilsesquioxane | Dow Corning 670 Fluid | 5,00 | Dow Corning |
| Phase B | | | |
| Dimethicone/ Vinyl Dimethicone Crosspolymer | Dow Corning 9506 Powder | 16,02 | Dow Corning |
| Silica Dimethyl Silylate | Covasilic 15 | 0,17 | LCW |
| Talc | Talc Powder | 5,00 | Sigma-Aldrich |
| | Effektpigment aus Beispiel 4 | 3,00 | |

(fortgesetzt)

| Phase D | | | |
|---|---|---|---|
| Phenoxyethanol, Ethylhexylglycerin | Euxyl PE 9010 | 0,40 | Schülke & Mayr |

Das Effektpigment aus Beispiel 4 kann in einem Bereich von 0,1 bis 8,0 Gew.-%, bezogen auf das Gesamtgewicht der Mousse Formulierung, eingesetzt werden. Der Ausgleich auf 100 Gew.-% der Formulierung kann mit Dow Corning 9041 Elastomer erfolgen.

Phase A wurde gemischt und solange erhitzt bis alles aufgeschmolzen war. Phase B wurden separat eingewogen und mit einem Hochgeschwindigkeitsmixer für 60s bei 2400 U/min gemischt. Die Hälfte der geschmolzenen Phase A wurde zur Phase B zugegeben und wieder im Mixer bei 2400 U/min für 30s gemischt. Anschließend wurde der übrige Teil der Phase B ebenfalls zur Phase A zugegeben und wieder bei 2400 U/min für 30s gemischt. Zuletzt wird Phase C zu Phase AB gegeben und wieder bei 2400 U/min für 30s im Hochgeschwindigkeitsmixer gemischt.

Anwendungstechnisches Beispiel 12: Nagellack

| INCI Name | Produktname | Gew.-% | Hersteller/Lieferant |
|---|---|---|---|
| Phase A | | | |
| | Effektpigment aus Beispiel 6 | 4,00 | |
| Phase B | | | |
| Butylacetat (and) Ethylacetat (and) Nitrocellulose (and) Isopropyl Alcohol | International Lacquers Nailpolish Base 15244 | 96,00 | International Lacquers |

Das Effektpigment aus Beispiel 6 kann in einem Bereich von 0,1 bis 8,0 Gew.-%, bezogen auf das Gesamtgewicht der Nagellack Formulierung, eingesetzt werden. Der Ausgleich auf 100 Gew.-% der Formulierung kann mit International Lacquers Nailpolish erfolgen.

Phase A und Phase B wurden gemischt und anschließend in ein angemessenes Behältnis abgefüllt.

Anwendungstechnisches Beispiel 13: Nagellack mit "Soft Touch"-Effekt

| INCI Name | Produktname | Gew.-% | Hersteller/Lieferant |
|---|---|---|---|
| Phase A | | | |
| | Effektpigment aus Beispiel 1 | 4,00 | |
| Polypropylene | Synafil W 1234 | 5,00 | Eckart |
| Phase B | | | |
| Butylacetat (and) Ethylacetat (and) Nitrocellulose (and) Isopropyl Alcohol | International Lacquers Nailpolish Base 15244 | 91,00 | International Lacquers |

Das Effektpigment aus Beispiel 1 kann in einem Bereich von 0,1 bis 8,0 Gew.-%, bezogen auf das Gesamtgewicht der Nagellack Formulierung, eingesetzt werden. Der Ausgleich auf 100 Gew.-% der Formulierung kann mit International Lacquers Nailpolish erfolgen.

Phase A wurde gemischt, zu Phase B hinzugefügt und anschließend wurde der Nagellack in ein angemessenes Behältnis abgefüllt.

Anwendungstechnisches Beispiel 14: wässriger Nagellack

Die Effektpigmente aus den Beispielen 1 bis 7 können in einem wässrigen Nagellack gemäß WO 2007/115675 A2 Beispiel 1 eingesetzt werden. Die Pigmentierungshöhe beträgt hierbei 0,1 bis 10,0 Gew.-%, bezogen auf das Gesamtgewicht der Formulierung.

Anwendungstechnisches Beispiel 15: Flüssig Lidschatten

| INCI Name | Produktname | Gew.-% | Hersteller/ Lieferant |
|---|---|---|---|
| *Phase A* | | | |
| Wasser | Aqua | 73,80 | |
| Glycerin | Glycerin | 3,00 | H. Erhard Wagner |
| *Phase B* | | | |
| PEG-800 | Polyglycol 35000 S | 0,60 | Clariant |
| Ammonium Acryloyldimehtyltaurate/ VP Copolymer | Aristoflex AVC | 0,80 | Clariant |
| Acrylates Copolymer | Worlee Micromer CEK 20/50 | 5,00 | Worlee |
| *Phase C* | | | |
| | Effektpigment aus Beispiel 7 | 10,00 | |
| Divinyldimethicone/ Dimethicone Copolymer C12-C13 Pareth-3, C12-C13 Pareth-23 | Dow Corning HMW 2220 Non-Ionic Emulsion | 6,00 | Dow Corning |
| Phenoxyethanol, Ethylhexylglycerin | Euxyl PE9010 | 0,80 | Schülke & Mayr |

Das Effektpigment aus Beispiel 7 kann in einem Bereich von 0,10 bis 20,00 Gew.-%, bezogen auf das Gesamtgewicht der Lidschatten Formulierung, eingesetzt werden. Der Ausgleich auf 100 Gew.-% der Formulierung kann mit Wasser erfolgen.

Phase A wurde gerührt, anschließend wurden die Inhaltsstoffe der Phase B einzeln zu Phase A hinzugegeben und gerührt bis eine gleichmäßige Konsistenz entstand. Danach wurden die Inhaltsstoffe der Phase C einzeln zu Phase AB gegeben und gerührt bis wieder eine gleichmäßige Konsistenz entstand.

Abbildung 1: Konzentrationsprofil (Line-Scan) anhand eines Querschliffes im Rasterelektronenmikroskop mit einem energiedispersiven Mikrobereichsanalysator (EDX) von Beispiel 1 vor Kalzination

Abbildung 2: Konzentrationsprofil (Line-Scan) anhand eines Querschliffes im Rasterelektronenmikroskop mit einem energiedispersiven Mikrobereichsanalysator (EDX) von Beispiel 1 nach Kalzination.

Abbildung 3: Rasterelektronenmikroskopische Querschliffaufnahme eines erfindungsgemäßen Effektpigments in 50000-facher Vergrößerung (bezogen auf Polaroid 545)

Abbildung 4: Ausschnitt der rasterelektronenmikroskopischen Querschliffaufnahme aus Abbildung 2 mit eingezeichneter Basislinie an der Grenzfläche nichtmetallisches plättchenförmiges Substrat - Beschichtung und senkrecht zur Basislinie angeordneten Linien. Mit "x" sind die Schnittpunkte an den Grenzflächen markiert

Abbildung 5: Rasterelektronenmikroskopische Querschliffaufnahme des mit Titandioxid beschichteten Perlglanzpigments SYMIC C261 (Fa. ECKART GmbH) in 20000-facher Vergrößerung (bezogen auf Polaroid 545)

Abbildung 6: Schematische Darstellung der Abstandslage

Abbildung 7: Schematische Darstellung der Position der Abstandslage

Abbildung 8: CIE-LCh-Farbraumschema

Abbildung 9: beanspruchter Farbbereich im CIE-LCh-Farbraum

**Patentansprüche**

1. Rotfarbenes Effektpigment umfassend ein nichtmetallisches plättchenförmiges Substrat und eine auf dem Substrat aufgebrachte Beschichtung, wobei die Beschichtung wenigstens ein Metalloxid, Metallhydroxid und/oder Metalloxidhydrat umfasst, die Metallionen des Metalloxids, Metallhydroxids und/oder Metalloxidhydrats wenigstens zwei unterschiedliche Metallionen, ausgewählt aus der Gruppe der Metalle, bestehend aus Fe, Sn, Ti und Zr umfassen, der Anteil an Eisenionen, bestimmt mittels RFA und berechnet als elementares Eisen, bei insgesamt wenigstens 17 Gew.-%, bezogen auf das Gesamtgewicht des rotfarbenen Effektpigments, und wobei der Bunttonwinkel $h^*_{15}$ im CIE-LCh-Farbraum in einem Bereich von 320° bis 360° und 0° bis 60° liegt.

2. Rotfarbenes Effektpigment nach Anspruch 1, wobei der Anteil an Zinn-, Titan- und Zirkoniumionen bei insgesamt $\leq$ 20 Gew.-%, jeweils bestimmt mittels RFA, jeweils berechnet als elementares Metall und jeweils bezogen auf das Gesamtgewicht des rotfarbenen Effektpigments, liegt.

3. Rotfarbenes Effektpigment nach einem der vorstehenden Ansprüche, wobei der Anteil an Metallionen, ausgewählt aus der Gruppe der Metalle, bestehend aus Sn, Ti und Zr,

   i. im Falle von Zinnionen bei insgesamt $\leq$ 17 Gew.-% oder
   ii. im Falle von Titanionen bei insgesamt $\leq$ 15 Gew.-% oder
   iii. im Falle von Zirkoniumionen bei insgesamt $\leq$ 18 Gew.-% oder
   iv. im Falle von Zinn- und Titanionen bei insgesamt $\leq$ 19 Gew.-%

   liegt, jeweils bestimmt mittels RFA, jeweils berechnet als elementares Metall und jeweils bezogen auf das Gesamtgewicht des rotfarbenen Effektpigments.

4. Rotfarbenes Effektpigment nach einem der vorstehenden Ansprüche, wobei die Beschichtung

   a) optional eine Schicht 1, mit einer mittleren Schichtdicke von weniger als 10 nm, die Zinnoxid, Zinnhydroxid und/oder Zinnoxidhydrat umfasst oder daraus besteht,
   b) eine Schicht 2, umfassend wenigstens ein Metalloxid, Metallhydroxid und/oder Metalloxidhydrat,
   c) eine Schicht 3, umfassend wenigstens ein Metalloxid, Metallhydroxid und/oder Metalloxidhydrat,

   aufweist, wenigstens eine der Schichten 2 oder 3 wenigstens zwei unterschiedliche Metallionen, ausgewählt aus der Gruppe der Metalle, bestehend aus Fe, Sn, Ti und Zr, umfasst, wobei wenigstens eine der Schichten 2 oder 3 Eisenionen umfasst und die Schichten 2 und 3 durch eine Abstandslage unterbrochen sind.

5. Rotfarbenes Effektpigment gemäß einem der vorstehenden Ansprüche, wobei das nichtmetallische plättchenförmige Substrat aus der Gruppe, bestehend aus natürlichen Glimmerplättchen, synthetischen Glimmerplättchen, Eisenglimmerplättchen, Glasplättchen, $SiO_2$-Plättchen, $Al_2O_3$-Plättchen, Kaolinplättchen, Talkplättchen, Bismuthoxychloridplättchen und deren Gemische, ausgewählt und das nichtmetallische plättchenförmige Substrat optional mit wenigstens einem Metalloxid, Metallhydroxid und/oder Metalloxidhydrat beschichtet ist.

6. Rotfarbenes Effektpigment gemäß einem der vorstehenden Ansprüche 4 oder 5, wobei die wenigstens eine Abstandslage eine mittlere Höhe $h_a$ aus einem Bereich von 3 nm bis 120 nm aufweist.

7. Rotfarbenes Effektpigment gemäß einem der vorstehenden Ansprüche 4 bis 6, wobei die wenigstens eine Abstandslage Verbindungen und Hohlräume aufweist.

8. Rotfarbenes Effektpigment gemäß einem der vorstehenden Ansprüche 4 bis 7, wobei die wenigstens eine Abstandslage eine Steganzahldichte von < 85% aufweist.

9. Rotfarbenes Effektpigment gemäß einem der vorstehenden Ansprüche 4 bis 8, wobei das Effektpigment weitere hoch- und/oder niedrigbrechende Schichten sowie optional wenigstens eine weitere Abstandslage umfasst.

10. Verfahren zur Herstellung des rotfarbenen Effektpigments gemäß einem der Ansprüche 1 bis 9, wobei das Verfahren die folgenden Schritten umfasst:

   (i) Optionales Aufbringen einer nicht kalzinierten Schicht, die Zinnoxid-, Zinnhydroxid- und/oder Zinnoxidhydrat umfasst oder daraus besteht, auf dem nichtmetallischen plättchenförmigen Substrat,

(ii) sequentielles Aufbringen von drei nicht kalzinierten Schichten A, B und C aus oder mit jeweils wenigstens einem Metalloxid, Metallhydroxid und/oder Metalloxidhydrat, wobei das Metallion der Schichten A und C jeweils wenigstens ein Metallion, ausgewählt aus der Gruppe der Metalle, bestehend aus Fe, Ti und Zr, umfasst oder ist, das Metallion der Schicht B wenigstens ein Metallion, ausgewählt aus der Gruppe der Metalle, bestehend aus Sn, Ti und Zr, umfasst oder ist, wenigstens ein Metallion der Schichten A und/oder C ein Eisenion umfasst oder ist, die Schichten A, B und C unmittelbar aufeinander angeordnet werden und wobei das in der Schicht B aufgebrachte wenigstens eine Metalloxid, Metallhydroxid und/oder Metalloxidhydrat in Bezug auf das Metallion verschieden von dem oder den Metallion(en) der Metalloxide, Metallhydroxide und/oder Metalloxidhydrate der Schicht A und Schicht C ist,

(iii) Kalzinieren des in Schritt (ii) erhaltenen Produktes bei einer Temperatur aus einem Bereich von 400°C bis 1000°C unter Erhalt des rotfarbenen Effektpigments.

**11.** Verfahren zur Herstellung des rotfarbenen Effektpigments gemäß einem der Ansprüche 1 bis 9, wobei das Verfahren die folgenden Schritten umfasst:

(i) Sequentielles Aufbringen von zwei nicht kalzinierten Schichten B und C aus oder mit jeweils wenigstens einem Metalloxid, Metallhydroxid und/oder Metalloxidhydrat auf ein kalziniertes einfach oder mehrfach beschichtetes nichtmetallisches Substrat, wobei das Metallion der Schicht B wenigstens ein Metallion, ausgewählt aus der Gruppe der Metalle, bestehend aus Ti, Sn und Zr, umfasst oder ist, das Metallion der Schicht C wenigstens ein Metallion, ausgewählt aus der Gruppe der Metalle, bestehend aus Fe, Ti und Zr, umfasst oder ist, wenigstens ein Metallion der Schicht, welche in Richtung Substrat unmittelbar an die Schicht B angrenzt, und/oder der Schicht C ein Eisenion umfasst oder ist, die Schichten B und C unmittelbar aufeinander angeordnet werden und wobei das in der Schicht B aufgebrachte wenigstens eine Metalloxid, Metallhydroxid und/oder Metalloxidhydrat in Bezug auf das Metallion verschieden von dem oder den Metallion(en) des Metalloxids, Metallhydroxids und/oder Metalloxidhydrats der Schicht C und der Schicht, welche in Richtung Substrat unmittelbar an die Schicht B angrenzt, ist, wobei das oder die Metallion(en) des Metalloxids, Metallhydroxids und/oder Metalloxidhydrats der Schicht, welche in Richtung Substrat unmittelbar an die Schicht B angrenzt, wenigstens ein Metallion, ausgewählt aus der Gruppe der Metalle, bestehend aus Fe, Ti und Zr, umfasst oder ist,

(ii) Kalzinieren des in Schritt (i) erhaltenen Produktes bei einer Temperatur aus einem Bereich von 400°C bis 1000°C unter Erhalt des rotfarbenen Effektpigments.

**12.** Verfahren gemäß einem der Ansprüche 10 oder 11, wobei die in Schicht B enthaltenen Metallionen wenigstens teilweise, in Schicht A und/oder in Schicht C und/oder in die Schicht, welche in Richtung Substrat unmittelbar an die Schicht B angrenzt unter Ausbildung der wenigstens einen Abstandslage im kalzinierten Effektpigment diffundieren.

**13.** Verfahren gemäß einem der Ansprüche 10 bis 12, wobei die zwei oder drei sequenziell aufgebrachten Metalloxide, Metallhydroxide und/oder Metalloxidhydrate zur Erzeugung der Schichten B und C oder der Schichten A, B und C, kein Metallion, ausgewählt aus der Gruppe der Metalle, bestehend aus Si, Mg und Al, umfasst.

**14.** Verwendung des rotfarbenen Effektpigments gemäß einem der Ansprüche 1 bis 9 in kosmetischen Formulierungen, Kunststoffen, Folien, Textilien, keramischen Materialien, Gläsern, Farben, Druckfarben, Tinten, Lacken, Pulverlacken und/oder als Lasermarkierungsadditiv.

**15.** Gegenstand umfassend wenigstens ein rotfarbenes Effektpigment gemäß einem der Ansprüche 1 bis 9.

**Claims**

**1.** A red-coloured effect pigment comprising a non-metallic, platelet-like substrate and a coating applied on the substrate, wherein the coating comprises at least one metal oxide, metal hydroxide and/or metal oxide hydrate, the metal ions of the metal oxide, metal hydroxide and/or metal oxide hydrate comprise at least two different metal ions selected from the group of metals consisting of Fe, Sn, Ti and Zr, the proportion of iron ions, determined by means of XRF and calculated as elemental iron, is in total less than 17 wt%, with respect to the total weight of the red-coloured effect pigment, and wherein the hue angle $h^*_{15}$ in the CIELCh colour space is in a range of from 320° to 360° and 0° to 60°.

**2.** The red-coloured effect pigment according to claim 1, wherein the proportion of tin, titanium and zirconium ions is in total ≤ 20 wt%, each determined by means of XRF, each calculated as elementary metal and each with respect to the total weight of the red-coloured effect pigment.

**3.** The red-coloured effect pigment according to one of the preceding claims, wherein the proportion of metal ions, selected from the group of metals consisting of Sn, Ti and Zr,

   i. is in total ≤ 17 wt% in the case of tin ions, or
   ii. is in total ≤ 15 wt% in the case of titanium ions, or
   iii. is in total ≤ 18 wt% in the case of zirconium ions, or
   iv. is in total ≤ 19 wt% in the case of tin and titanium ions,

each determined by means of XRF, each calculated as elemental metal and each with respect to the total weight of the red-coloured effect pigment.

**4.** The red-coloured effect pigment according to one of the preceding claims, wherein the coating comprises

   a) optionally a layer 1 which has a mean layer thickness of less than 10 nm and comprises or consists of tin oxide, tin hydroxide and/or tin oxide hydrate,
   b) a layer 2 which comprises at least one metal oxide, metal hydroxide and/or metal oxide hydrate,
   c) a layer 3 which comprises at least one metal oxide, metal hydroxide and/or metal oxide hydrate,

at least one of the layers 2 or 3 comprises at least two different metal ions selected from the group of metals consisting of Fe, Sn, Ti and Zr, wherein at least one of the layers 2 or 3 comprises iron ions and the layers 2 and 3 are disconnected by a spacer layer.

**5.** The red-coloured effect pigment according to one of the preceding claims, wherein the non-metallic substrate in platelet form is selected from the group consisting of natural mica platelets, synthetic mica platelets, iron mica platelets, glass platelets, $SiO_2$ platelets, $Al_2O_3$ platelets, kaolin platelets, talc platelets, bismuth oxychloride platelets and mixtures thereof, and the non-metallic platelet-like substrate is optionally coated with at least one metal oxide, metal hydroxide and/or metal oxide hydrate.

**6.** The red-coloured effect pigment according to any of the preceding claims 4 or 5, wherein the at least one spacer layer has a mean height $h_a$ in a range of from 3 nm to 120 nm.

**7.** The red-coloured effect pigment according to one of the preceding claims 4 to 6, wherein the at least one spacer layer includes connections and cavities.

**8.** The red-coloured effect pigment according to one of the preceding claims 4 to 7, wherein the at least one spacer layer has a network density of < 85%.

**9.** The red-coloured effect pigment according to one of the preceding claims 4 to 8, wherein the effect pigment comprises further high-refractive and/or low-refractive layers and optionally at least one further spacer layer.

**10.** A method for the production of the red-coloured effect pigment according to one of claims 1 to 9, wherein the method comprises the following steps:

   (i) optionally applying an uncalcined layer comprising or consisting of tin oxide, tin hydroxide and/or tin oxide hydrate to the non-metallic platelet-like substrate,
   (ii) sequentially applying three uncalcined layers A, B and C, each consisting of or comprising at least one metal oxide, metal hydroxide and/or metal oxide hydrate, wherein the metal ion of the layers A and C each comprises or is at least one metal ion selected from the group of metals consisting of Fe, Ti and Zr, the metal ion of layer B comprises or is at least one metal ion selected from the group of metals consisting of Sn, Ti and Zr, at least one metal ion of the layers A and/or C comprises or is an iron ion, the layers A, B and C are arranged directly on top of one another and wherein the at least one metal oxide, metal hydroxide and/or metal oxide hydrate applied in layer B is different in terms of the metal ion from the metal ion or ions of the metal oxide, metal hydroxide and/or metal oxide hydrate of the layer A and layer C,
   (iii) calcining the product obtained in step (ii) at a temperature in a range of from 400°C to 1000°C to obtain the red-coloured effect pigment.

11. The method for the production of the red-coloured effect pigment according to one of claims 1 to 9, wherein the method comprises the following steps:

(i) sequentially applying two uncalcined layers B and C, each consisting of or comprising at least one metal oxide, metal hydroxide and/or metal oxide hydrate to a calcinated singly or multiply coated non-metallic substrate, wherein the metal ion of the layer B comprises or is at least one metal ion selected from the group of metals consisting of Ti, Sn and Zr, the metal ion of layer C comprises or is at least one metal ion selected from the group of metals consisting of Fe, Ti and Zr, at least one metal ion of the layer which is directly adjacent to the layer B in the direction of the substrate and/or at least one metal ion of the layer C comprises or is an iron ion, the layers B and C are arranged directly on top of one another and wherein the at least one metal oxide, metal hydroxide and/or metal oxide hydrate applied in layer B is different in terms of the metal ion from the metal ion or ions of the metal oxide, metal hydroxide and/or metal oxide hydrate of the layer C and of the layer which is directly adjacent to the layer B in the direction of the substrate, wherein the metal ion or ions of the metal oxide, metal hydroxide and/or metal oxide hydrate of the layer which is directly adjacent to the layer B in the direction of the substrate comprises or is at least one metal ion selected from the group of metals consisting of Fe, Ti and Zr,
(ii) calcining the product obtained in step (i) at a temperature in a range of from 400°C to 1000°C to obtain the red-coloured effect pigment.

12. The method according to any of claims 10 or 11, wherein the metal ions contained in layer B diffuse at least partly into layer A and/or into layer C and/or into the layer directly adjacent to layer B in the direction of the substrate to form the at least one spacer layer in the calcined effect pigment.

13. The method according to any of claims 10 to 12, wherein the two or three sequentially applied metal oxides, metal hydroxides and/or metal oxide hydrates for producing the layers B and C or the layers A, B and C do not comprise any metal ion selected from the group of metals consisting of Si, Mg and Al.

14. Use of the red-coloured effect pigment according to one of claims 1 to 9 in cosmetic formulations, plastics, films, textiles, ceramic materials, glasses, paints, printing inks, writing inks, varnishes, powder coatings and/or laser marking additives.

15. An article comprising at least one red-coloured effect pigment according to one of claims 1 to 9.

**Revendications**

1. Pigment à effet de couleur rouge comprenant un substrat lamellaire non métallique et un revêtement appliqué sur le substrat, le revêtement comprenant au moins un oxyde métallique, un hydroxyde métallique et/ou un oxyde métallique hydraté, les ions métalliques de l'oxyde métallique, de l'hydroxyde métallique et/ou de l'oxyde métallique hydraté comprenant au moins deux ions métalliques différents, sélectionnés dans le groupe des métaux constitués de Fe, Sn, Ti et Zr, la proportion d'ions fer, déterminée par FXA et calculée en fer élémentaire, étant au total d'au moins 17 % en poids, rapporté au poids total du pigment à effet de couleur rouge, et l'angle de tonalité chromatique $h^*_{15}$ dans l'espace colorimétrique CIE-LCh étant compris dans une plage entre 320° et 360° et entre 0° et 60°.

2. Pigment à effet couleur rouge selon la revendication 1, la proportion d'ions étain, titane et zirconium étant au total ≤ 20 % en poids, chacun déterminé par FXA, chacun calculé en tant que métal élémentaire et chacun rapporté au poids total du pigment à effet de couleur rouge.

3. Pigment à effet de couleur rouge selon l'une des revendications précédentes, la proportion d'ions métalliques sélectionnés dans le groupe des métaux constitués de Sn, Ti et Zr,

i. en présence d'ions étain, étant au total ≤ 17 % en poids ou
ii. en présence d'ions titane, étant au total ≤ 15 % en poids ou
iii. en présence d'ions zirconium, étant au total ≤ 18 % en poids ou
iv. en présence d'ions étain et titane, étant au total ≤ 19 % en poids,

chacun déterminé par FXA, chacun calculé en tant que métal élémentaire et chacun rapporté au poids total du pigment à effet de couleur rouge.

4. Pigment à effet de couleur rouge selon l'une des revendications précédentes, le revêtement

a) présentant éventuellement une couche 1 ayant une épaisseur moyenne inférieure à 10 nm, qui comprend de l'oxyde d'étain, de l'hydroxyde d'étain et/ou de l'oxyde d'étain hydraté, ou en est constituée
b) une couche 2 comprenant au moins un oxyde métallique, un hydroxyde métallique et/ou un oxyde métallique hydraté,
c) une couche 3 comprenant au moins un oxyde métallique, un hydroxyde métallique et/ou un oxyde métallique hydraté,

au moins l'une des couches 2 ou 3 comprenant deux ions métalliques différents, sélectionnés dans le groupe des métaux constitués de Fe, Sn, Ti et Zr, au moins une des couches 2 ou 3 comprenant des ions fer et les couches 2 ou 3 étant interrompues par une strate d'espacement.

5. Pigment à effet de couleur rouge selon l'une des revendications précédentes, le substrat lamellaire non métallique étant sélectionné dans le groupe constitué des paillettes de mica naturel, des paillettes de mica synthétique, des paillettes d'oxyde de fer micacé, des paillettes de verre, des paillettes de $SiO_2$, des paillettes d'$Al_2O_3$, des paillettes de kaolin, des paillettes de talc, des paillettes d'oxychlorure de bismuth et leurs mélanges, et le substrat lamellaire non métallique étant éventuellement revêtu d'au moins un oxyde métallique, un hydroxyde métallique et/ou un oxyde métallique hydraté.

6. Pigment à effet de couleur rouge selon l'une quelconque des revendications précédentes 4 ou 5, ladite strate d'espacement présentant une hauteur moyenne $h_a$ comprise dans une plage entre 3 nm et 120 nm.

7. Pigment à effet de couleur rouge selon l'une des revendications précédentes 4 à 6, ladite strate d'espacement présentant des liaisons et des cavités.

8. Pigment à effet de couleur rouge selon l'une des revendications précédentes 4 à 7, ladite strate d'espacement présentant un rapport entre le nombre d'éléments d'écartement et le nombre de lignes < 85 %.

9. Pigment à effet de couleur rouge selon l'une des revendications précédentes 4 à 8, le pigment à effet comprenant d'autres couches faiblement et/ou hautement réfringentes ainsi que, éventuellement, au moins une autre strate d'espacement.

10. Procédé de production du pigment à effet de couleur rouge selon l'une des revendications 1 à 9, le procédé comprenant les étapes suivantes :

(i) appliquer éventuellement une couche non calcinée, qui comprend de l'oxyde d'étain, de l'hydroxyde d'étain et/ou de l'oxyde d'étain hydraté, sur le substrat lamellaire non métallique, ou en est constituée
(ii) appliquer séquentiellement trois couches non calcinées A, B et C constituées chacune d'au moins un oxyde métallique, un hydroxyde métallique et/ou un oxyde métallique hydraté ou le comportant, l'ion métallique e chacune des couches A et C comprenant ou étant un ion métallique sélectionné dans le groupe des métaux constitués de Fe, Ti et Zr, l'ion métallique de la couche B comprenant ou étant un ion métallique sélectionné dans le groupe de métaux constitués de Sn, Ti et Zr, au moins un ion métallique des couches A et/ou C comprenant ou étant un ion fer, les couches A, B et C étant disposées directement les unes sur les autres et l'au moins un oxyde métallique et/ou hydroxyde métallique et/ou oxyde métallique hydraté appliqué dans la couche B étant, pour ce qui est de l'ion métallique, différent du ou des ions métalliques des oxydes métalliques, des hydroxydes métalliques et/ou des oxydes métalliques hydratés de la couche A et de la couche C,
(iii) calciner le produit obtenu à l'étape (ii) à une température dans une plage comprise entre 400°C et 1 000°C pour obtenir le pigment à effet de couleur rouge.

11. Procédé de production du pigment à effet de couleur rouge selon l'une des revendications 1 à 9, le procédé comprenant les étapes suivantes :

(i) appliquer séquentiellement deux couches non calcinées B et C constituées chacune d'au moins un oxyde métallique, un hydroxyde métallique et/ou un oxyde métallique hydraté, ou le comportant, sur un substrat non métallique monocouche ou multicouche, l'ion métallique de la couche B comprenant ou étant au moins un ion métallique sélectionné dans le groupe des métaux constitués de Ti, Sn et Zr, l'ion métallique de la couche C comprenant ou étant au moins un ion métallique sélectionné dans le groupe des métaux constitué de Fe, Ti ou Zr, au moins un ion métallique de la couche, qui dans la direction du substrat est directement adjacente à la couche B, et/ou de la couche C comprenant ou étant un ion fer, les couches B et C étant disposées directement les unes sur les autres et l'au moins un oxyde métallique et/ou hydroxyde métallique et/ou oxyde métallique hydraté appliqué dans la couche B étant, par rapport à l'ion métallique, différent du ou des ions métalliques de l'oxyde métallique, de l'hydroxyde métallique et/ou de l'oxyde métallique hydraté de la couche C et de la couche qui dans la direction du substrat est directement adjacente à la couche B, le ou les ions métalliques de l'oxyde métallique, de l'hydroxyde métallique et/ou de l'oxyde métallique hydraté de la couche qui dans la direction du substrat est directement adjacente à la couche B comprenant ou étant au moins un ion métallique sélectionné dans le groupe des métaux constitués de Fe, Ti et Zr,

(ii) calciner le produit obtenu à l'étape (i) à une température dans une plage comprise entre 400°C et 1 000°C pour obtenir le pigment à effet de couleur rouge.

12. Procédé selon l'une des revendications 10 ou 11, les ions métalliques contenus dans la couche B se diffusant au moins partiellement dans la couche A et/ou dans la couche C et/ou dans la couche qui dans la direction du substrat est directement adjacente à la couche B pour former ladite strate d'espacement dans le pigment à effet calciné.

13. Procédé selon l'une des revendications 10 ou 12, les deux ou trois oxydes métalliques, hydroxydes métalliques et/ou oxydes métalliques hydratés appliqués séquentiellement pour produire les couches B et C ou les couches A, B et C ne comprenant aucun ion métallique sélectionné dans le groupe des métaux constitués de Si, Mg et Al.

14. Utilisation du pigment à effet de couleur rouge selon l'une des revendications 1 à 9 dans des formulations cosmétiques, des matières plastiques, des feuilles, des textiles, des matériaux céramiques, des verres, des peintures, des encres d'impression, des encres, des vernis, des peintures en poudre et/ou comme additif pour marquage laser.

15. Objet comprenant au moins un pigment à effet de couleur rouge selon l'une des revendications 1 à 9.

Abbildung 1:

Abbildung 2:

Abbildung 3:

Abbildung 4:

Abbildung 5:

Abbildung 6:

→ Höhe $h_a$ der Abstandslage

Plättchenförmiges Substrat

Abbildung 7:

→ 6/6 keine Abstandslage erlaubt

→ 2/6 bis 5/6 Abstandslage erlaubt

→ 1/6 keine Abstandslage erlaubt

**Plättchenförmiges Substrat**

Abbildung 8:

**90° +b* gelb**

**180° -a* grün**

**360° und 0° +a* rot**

**270° -b* blau**

Abbildung 9:

beanspruchter Farbraum

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1572812 A1
- EP 1213330 A1
- EP 1025168 B2
- EP 1621585 A2
- EP 0948572 A1
- EP 0950693 A1
- EP 1306412 A1
- EP 1587881 A2
- EP 2632988 A1
- EP 1474486 A2
- EP 1375601 A1
- EP 1281732 A1
- EP 0753545 A2
- US 20040003758 A1
- EP 1422268 A2
- US 20150344677 A1
- US 8585818 B1
- WO 2014094993 A1
- US 3711308 A
- EP 1980594 B1
- EP 1829833 B1

- EP 2042474 B1
- EP 289240 B1
- CN 102718229 A
- US 20140251184 A1
- EP 0723997 A1
- EP 2371908 A2
- EP 1546063 A1
- EP 1121334 A1
- EP 2217664 A1
- EP 2346950 A1
- EP 2356181 A1
- EP 2346949 A1
- EP 2367889 A1
- WO 2006021386 A1
- WO 2012130897 A1
- WO 2014053454 A1
- EP 2698403 A1
- EP 2576702 A1
- WO 2006136435 A2
- WO 2007115675 A2

**In der Beschreibung aufgeführte Nicht-Patentliteratur**

- **BYK-GARDNER.** *Qualitätskontrolle für Lacke und Kunststoffe,* 2011, 97, , 98